# EUROPÄISCHE PATENTSCHRIFT

(11) **EP 3 619 201 B1**
(45) Veröffentlichungstag und Bekanntmachung des Hinweises auf die Patenterteilung: **10.08.2022**
(21) Anmeldenummer: 18719892.4
(22) Anmeldetag: 26.04.2018
(51) Int. Cl.: C07D 317/14, C07D 255/02, C07D 257/06, C07D 271/04, C07D 271/06, A01N 43/26, A01N 43/824

(54) **HERBIZID WIRKSAME 4-DIFLUORMETHYLBENZOYLAMIDE**
HERBICIDALLY ACTIVE 4-DIFLUORMETHYLBENZOYLAMIDE
4-DIFLUORO-MÉTHYL BENZOYLAMIDE À EFFICACITÉ HERBICIDE

(30) Priorität: 04.05.2017 EP 17169505
(43) Veröffentlichungstag der Anmeldung: 11.03.2020
(73) Patentinhaber: Bayer CropScience Aktiengesellschaft, 40789 Monheim am Rhein (DE)
(72) Erfinder: AHRENS, Hartmut, 63225 Langen (DE); TIEBES, Jörg, 60431 Frankfurt (DE); WALDRAFF, Christian, 61118 Bad Vilbel (DE); DIETRICH, Hansjörg, 65835 Liederbach am Taunus (DE); GATZWEILER, Elmar, 61231 Bad Nauheim (DE); ROSINGER, Christopher, Hugh, 65719 Hofheim (DE); MACHETTIRA, Anu, Bheemaiah, 60326 Frankfurt am Main (DE)
(74) Vertreter: BIP Patents
(86) Internationale Anmeldenummer: PCT/EP2018/060709
(87) Internationale Veröffentlichungsnummer: WO 2018/202535

(56) Entgegenhaltungen:
- WO-A1-2011/035874
- WO-A1-2012/028579
- WO-A1-2012/126932
- WO-A1-2016/146561

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

WO 2011/035874 A1, WO 2012/126932 A1, WO 2012/028579 A1 und WO 2016/146561 Albeschreiben herbizid wirksame Benzoylamide, die sich im Wesentlichen durch die Art des heterocyclischen Substituenten voneinander unterscheiden. Diese Benzoylamide können in den 2-, 3-und 4-Positionen des Phenylrings durch eine Vielzahl von unterschiedlichen Resten substituiert sein. WO 2016/146561 A1 offenbart unter den Tabellenbeispielen 1-38 und 1-41 jeweils die Natriumsalze der beiden Verbindungen 4-Difluormethyl-3-ethylsulfinyl-2-methyl-N-(5-methyl-1,3,4-oxadiazol-2-yl)benzamid und 4-Difluormethyl-3-ethylsulfonyl-2-methyl-N-(5-methyl-1,3,4-oxadiazol-2-yl)benzamid. Jedoch weisen die aus den oben genannten Schriften bekannten Benzoylamide nicht immer eine ausreichende herbizide Wirkung und/oder Verträglichkeit gegenüber Kulturpflanzen auf. Aufgabe der vorliegenden Erfindung ist es, alternative herbizid wirksame Wirkstoffe bereitzustellen. Diese Aufgabe wird durch die nachfolgend beschriebenen erfindungsgemäßen Benzoylamide gelöst, die in 2-Position des Phenylrings eine Alkyl-, Cycloalkyl- oder Halogen-Gruppe, in 3-Position einen schwefel-haltigen Rest und in 4-Position eine CHF₂-Gruppe tragen.

Ein Gegenstand der vorliegenden Erfindung sind somit Benzoylamide der Formel (I) und deren Salze 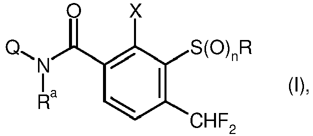 worin die Symbole und Indizes folgende Bedeutungen haben:
Q bedeutet einen Rest Q1, Q2, Q3 oder Q4, 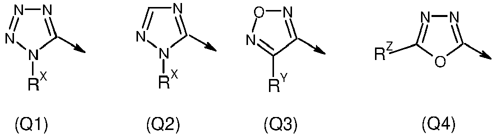
X bedeutet (C₁-C₆)-Alkyl, (C₃-C₆)-Cycloalkyl oder Halogen,
R bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
R^{a} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C, R¹O(O)C, (R¹)₂N(O)C, R¹O, (R¹)₂N, R²O(O)C(R¹)N, (R¹)₂N(O)C(R¹)N, R²(O)₂S,
oder jeweils durch s Reste aus der Gruppe bestehend aus Methyl, Ethyl, Methoxy, Nitro, Trifluormethyl und Halogen substituiertes Benzyl,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus Nitro, Cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl-S(O)ₙ, (C₁-C₆)-Alkoxy, Halogen-(Ci-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, (C₁-C₆)-Alkoxy, Halogen-(Ci-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxyund Halogen substituiertes Heteroaryl, Heterocyclyl oder Phenyl, und wobei Heterocyclyl n Oxogruppen trägt,
R^{Z} bedeutet Wasserstoff, (C₁-C₆)-Alkyl, R¹O-(C₁-C₆)-Alkyl, R*´*CH₂, (C₃-C₇)-Cycloalkyl, Halogen-(C₁-C₆)-alkyl,(C₂-C₆)-Alkenyl, Halogen-(C₂-C₆)-alkenyl, (C₂-C₆)-Alkinyl, Halogen-(C₃-C₆)-alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxyund (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(Ci-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl,Heterocyclyl, Heterocyclyl-(C₁-C₆)-alkyl, Phenyl-O-(C₁-C₆)-alkyl, Heteroaryl-O-(C₁-C₆)-alkyl,Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl,
R*'* bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
n bedeutet 0, 1 oder 2,
s bedeutet 0, 1, 2 oder 3,
mit der Maßgabe, dass die Verbindungen 4-Difluormethyl-3-ethylsulfinyl-2-methyl-N-(5-methyl-1,3,4-oxadiazol-2-yl)benzamid und 4-Difluormethyl-3-ethylsulfonyl-2-methyl-N-(5-methyl-1,3,4-oxadiazol-2-yl)benzamid sowie deren Natriumsalze ausgenommen sind.

In den Resten Q1, Q2, Q3 und Q4 bedeutet der Pfeil die Verknüpfung zum Amid-Stickstoffatom der Verbindungen der Formel (I).

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl.

Halogen steht für Fluor, Chlor, Brom oder Iod.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Die Verbindungen der Formel (I) können Salze bilden. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogen-carbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR*'*R*"*R‴]⁺, worin R bis R‴ jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie p-Toluolsulfonsäure, an eine basische Gruppe, wie Amino, Alkylamino, Dialkylamino, Piperidino, Morpholino oder Pyridino, Salze bilden. Diese Salze enthalten dann die konjugierte Base der Säure als Anion.

Bevorzugt sind Verbindungen der allgemeinen Formel (I), wobei die Symbole und Indices folgende Bedeutungen haben:
Q bedeutet einen Rest Q1, Q2, Q3 oder Q4, 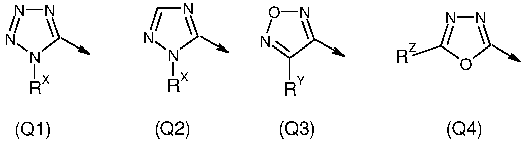
X bedeutet (C₁-C₆)-Alkyl oder (C₃-C₆)-Cycloalkyl,
R bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
R^{a} bedeutet Wasserstoff,
RX bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste durch s Reste aus der Gruppe bestehend aus (C₁-C₆)-Alkyl, Halogen-(C₁-C₆)-alkyl, (C₁-C₆)-Alkoxy und Halogen substituiert sind, und wobei Heterocyclyl n Oxogruppen trägt,
oder R^{X} bedeutet (C₃-C₇)-Cycloalkyl, wobei dieser Rest durch s Reste aus der Gruppe bestehend aus Halogen, (C₁-C₆)-Alkyl und Halogen-(C₁-C₆)-alkyl substituiert ist,
RY bedeutet Wasserstoff, (C₁-C₆)-Alkyl, Halogen-(C₁-C₆)-alkyl, (C₃-C₇)-Cycloalkyl, (C₁-C₆)-Alkoxy, Methoxycarbonyl, Methoxycarbonylmethyl, Halogen, Amino, Aminocarbonyl oder Methoxymethyl,
RZ bedeutet Wasserstoff, (C₁-C₆)-Alkyl, R¹O-(C₁-C₆)-Alkyl, R´CH₂, (C₃-C₇)-Cycloalkyl, Halogen-(C₁-C₆)-alkyl,R¹O, R¹(H)N, Methoxycarbonyl, Acetylamino oder Methylsulfonyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl,Heterocyclyl, Heterocyclyl-(C₁-C₆)-alkyl, Phenyl-O-(C₁-C₆)-alkyl, Heteroaryl-O-(C₁-C₆)-alkyl,Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl,Heterocyclyl, Heterocyclyl-(C₁-C₆)-alkyl,Phenyl-O-(C₁-C₆)-alkyl, Heteroaryl-O-(C₁-C₆)-alkyl,Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R*'* bedeutet Acetoxy, Acetamido, Methoxycarbonyl oder (C₃-C₆)-Cycloalkyl,
n bedeutet 0, 1 oder 2,
s bedeutet 0, 1, 2 oder 3.

Bevorzugt sind auch Verbindungen der allgemeinen Formel (I), wobei die Symbole und Indices folgende Bedeutungen haben:
Q bedeutet einen Rest Q1, Q2, Q3 oder Q4, 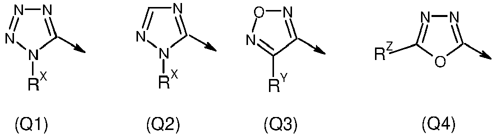
X bedeutet Halogen,
R bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
R^{a} bedeutet Wasserstoff,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste durch s Reste aus der Gruppe bestehend aus (C₁-C₆)-Alkyl, Halogen-(C₁-C₆)-alkyl, (C₁-C₆)-Alkoxy und Halogen substituiert sind, und wobei Heterocyclyl n Oxogruppen trägt,
oder R^{X} bedeutet (C₃-C₇)-Cycloalkyl, wobei dieser Rest durch s Reste aus der Gruppe bestehend aus Halogen, (C₁-C₆)-Alkyl und Halogen-(C₁-C₆)-alkyl substituiert ist,
R^{Y} bedeutet Wasserstoff, (C₁-C₆)-Alkyl, Halogen-(C₁-C₆)-alkyl, (C₃-C₇)-Cycloalkyl, (C₁-C₆)-Alkoxy, Methoxycarbonyl, Methoxycarbonylmethyl, Halogen, Amino, Aminocarbonyl oder Methoxymethyl,
R^{Z} bedeutet Wasserstoff, (C₁-C₆)-Alkyl, R¹O-(C₁-C₆)-Alkyl, R´CH₂, (C₃-C₇)-Cycloalkyl, Halogen-(C₁-C₆)-alkyl,R¹O, R¹(H)N, Methoxycarbonyl, Acetylamino oder Methylsulfonyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl,Heterocyclyl, Heterocyclyl-(C₁-C₆)-alkyl, Phenyl-O-(C₁-C₆)-alkyl, Heteroaryl-O-(C₁-C₆)-alkyl,Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl,Heterocyclyl, Heterocyclyl-(C₁-C₆)-alkyl,Phenyl-O-(C₁-C₆)-alkyl, Heteroaryl-O-(C₁-C₆)-alkyl,Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R´ bedeutet Acetoxy, Acetamido, Methoxycarbonyl oder (C₃-C₆)-Cycloalkyl,
n bedeutet 0, 1 oder 2,
s bedeutet 0, 1, 2 oder 3.

Ganz bevorzugt sind Verbindungen der allgemeinen Formel (I), wobei die Symbole und Indices folgende Bedeutungen haben:
- Q: bedeutet einen Rest Q1, Q2, Q3 oder Q4,

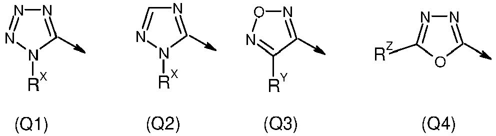

- X: bedeutet Methyl, Ethyl oder Cyclopropyl,
- R: bedeutet Methyl, Ethyl, Cyclopropylmethyl oder Methoxyethyl,
- R^{a}: bedeutet Wasserstoff,
- R^{X}: bedeutet Methyl, Ethyl oder n-Propyl,
- R^{Y}: bedeutet Methyl oder Chlor,
- R^{Z}: bedeutet Methyl,
- n: bedeutet 0, 1 oder 2.

Ganz bevorzugt sind auch Verbindungen der allgemeinen Formel (I), wobei die Symbole und Indices folgende Bedeutungen haben:
- Q: bedeutet einen Rest Q1, Q2, Q3 oder Q4,

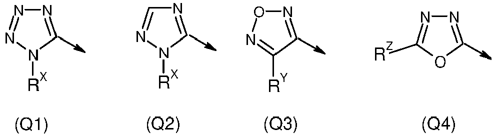

- X: bedeutet Fluor, Chlor, Brom oder Iod,
- R: bedeutet Methyl, Ethyl, Cyclopropylmethyl oder Methoxyethyl,
- R^{a}: bedeutet Wasserstoff,
- R^{X}: bedeutet Methyl, Ethyl oder n-Propyl,
- R^{Y}: bedeutet Methyl oder Chlor,
- R^{Z}: bedeutet Methyl,
- n: bedeutet 0, 1 oder 2.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen Q für Q1 oder Q2 steht, sowie die diesen Amiden zugrunde liegenden Aminotetrazole und Aminotriazole können beispielsweise nach den in WO 2012/028579 A1 angegebenen Methoden hergestellt werden.

Erfindungsgemäße Verbindungen, in denen Q für Q3 steht, sowie die diesen Amiden zugrunde liegenden Aminofurazane können beispielsweise nach den in WO 2011/035874A1 angegebenen Methoden hergestellt werden.

Erfindungsgemäße Verbindungen, in denen Q für Q4 steht, können beispielsweise nach den in WO 2012/126932 A1 angegebenen Methoden hergestellt werden. Die diesen Amiden zugrunde liegenden 2-Amino-1,3,4-oxadiazole sind kommerziell verfügbar bzw. nach gängigen und aus der Literatur bekannten Methoden synthetisch zugänglich.

Die den erfindungsgemäßen Verbindungen (I) zugrunde liegenden Benzoesäurechloride beziehungsweise die entsprechenden Benzoesäuren können beispielsweise gemäß der in Schema 1 angegebenen Methode hergestellt werden. Die hierfür benötigten 2-Hydroxybenzoesäureester sind nach dem in WO 2014/090766 A1 (siehe insbesondere dortiges Synthesebeispiel 2 auf S. 6) angegebenen Verfahren zugänglich. Die Hydroxygruppe wird methyliert, gefolgt von einer Esterverseifung. Nach dem Aufbau der Oxazolingruppe kann die Methoxygruppe gegen Alkyl-, Cycloalkyl- oder Aminogruppen nucleophil ausgetauscht werden (A. I. Meyers et al., J. Org. Chem., 1978, 43 (7), 1372-1379; A. I. Meyers et al., J. Org. Chem., 1977, 42 (15), 2653-2654; A. I. Meyers et al., Tetrahedron, 1994, 50 (8), 2297-2360; T. W. Greene, P. G. M. Wuts, Protective Groups in Organic Synthesis, 2nd Edition, John Wiley & Sons, Inc. 1991, S. 265 ff.; Z. Hell et al., Tetrahedron Letters, 2002, 43, 3985-3987.). Die anschließende Oxazolinspaltung liefert die substituierte 4-Difluormethylbenzoesäure, welche je nach gewünschtem Substitutionsmuster weiter modifiziert werden kann. Beispielsweise können 2-Aminobenzoesäuren über eine Sandmeyerreaktion in ihre 2-Halogenbenzoesäuren überführt werden.

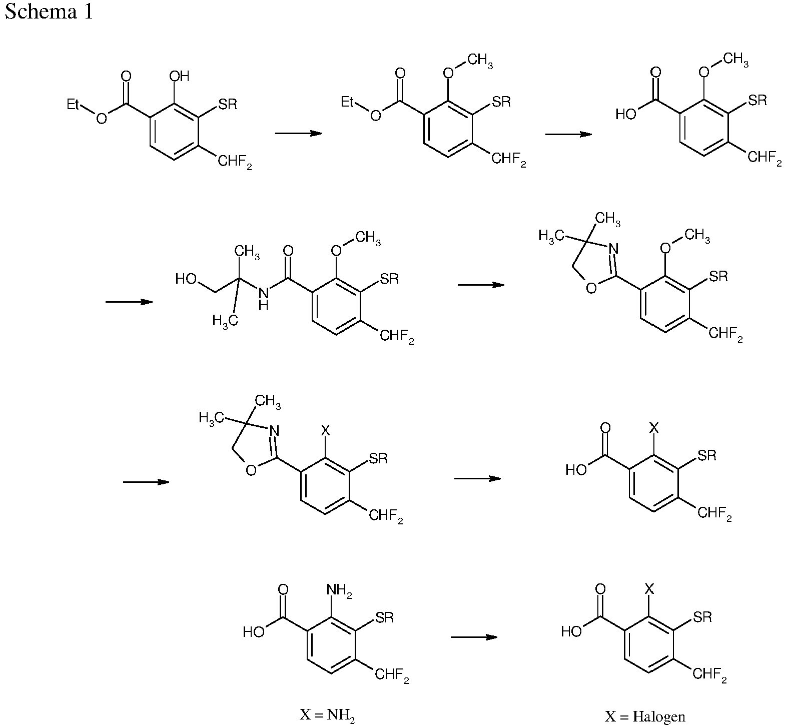

Aus dem Stand der Technik sind eine Reihe weiterer Methoden zur Einführung einer Difluormethylgruppe bekannt, so zum Beispiel aus: Y. Lu, C. Liu, Q.-Y. Chen, Curr. Org. Chem., 2015, 19, 1638-1650.

Der Thioether kann beispielsweise gemäß Schema 2 weiter zu dem entsprechenden Sulfoxid oder Sulfon oxidiert werden. Oxidationsmethoden, die gezielt zum Sulfoxid oder Sulfon führen, sind in der Literatur bekannt. Es bietet sich eine Anzahl an Oxidationssystemen an, beispielsweise Persäuren wie meta-Chlorperbenzoesäure, die gegebenenfalls in situ erzeugt werden (zum Beispiel Peressigsäure im System Essigsäure/Wasserstoffperoxid/Natriumwolframat(VI)) (Houben-Weyl, Methoden der Organischen Chemie, Georg Thieme Verlag Stuttgart, Bd. E 11, Erweiterungs- und Folgebände zur vierten Auflage 1985, S. 702 ff., S. 718 ff. sowie S. 1194 ff.).

Unter anderem hängt es vom Substitutionsmuster und vom Oxidationsmittel ab, an welcher Stelle der Synthesekaskade die Oxidation des Thioethers zweckmäßig ist. Eine Oxidation kann beispielsweise, wie in Schema 2 gezeigt, auf der Stufe der freien Benzoesäure oder auf der Stufe des Amids der Formel (I) mit R^{a} = H und n = 0 sinnvoll sein.

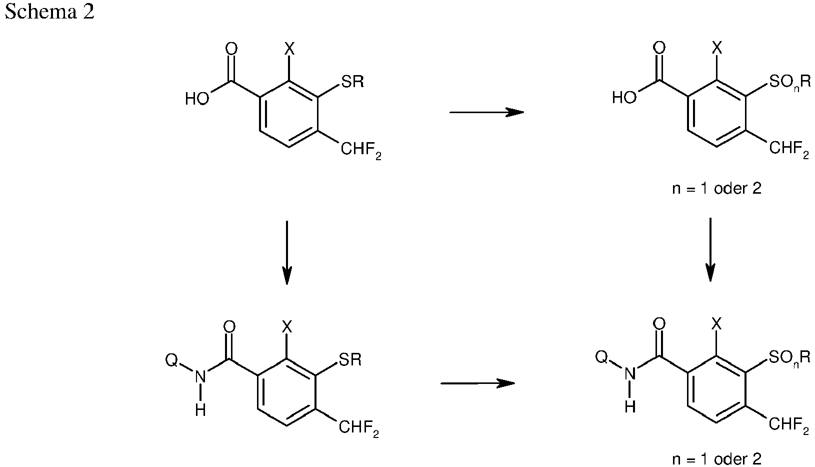

Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid der Formel (I) mit R^{a} = H und n = 0 herzustellen und danach den Thioether zum Sulfoxid zu oxidieren.

Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Die Herstellung der erfindungsgemäßen Verbindungen (I) kann, wie oben beschrieben, über substituierte Benzoesäuren der Formel (II) oder der entsprechenden Benzoylchloride der Formel (III) verlaufen.

Verbindungen der Formel (II) eignen sich sehr gut als Intermediate zur Herstellung der erfindungsgemäßen Verbindungen der Formel (I).

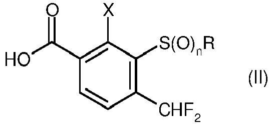

In Formel (II) haben die Symbole und Indizes folgende Bedeutungen:
- X: bedeutet (C₃-C₆)-Cycloalkyl oder Halogen,
- R: bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
- n: bedeutet 0, 1 oder 2.

Verbindungen der Formel (III) eignen sich sehr gut als Intermediate zur Herstellung der erfindungsgemäßen Verbindungen der Formel (I).

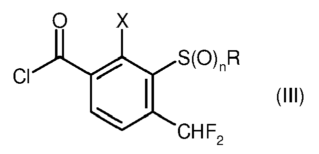

In Formel (III) haben die Symbole und Indizes folgende Bedeutungen :
- X: bedeutet (C₁-C₆)-Alkyl, (C₃-C₆)-Cycloalkyl oder Halogen,
- R: bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
- n: bedeutet 0, 1 oder 2.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl in fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria und Sorghum.
Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola und Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen, abhängig von ihrer jeweiligen chemischen Struktur und der ausgebrachten Aufwandmenge, hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Maniok, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking").

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykol-ethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### Chemische Beispiele

### Synthese von 4-(Difluormethyl)-2-methyl-3-(methylsulfanyl)-N-(1-methyl-1H-1,2,4-triazol-5-yl)benzamid (Beispiel-Nr. 4-1)

### Schritt 1: Synthese von 4-Difluormethyl-2-methoxy-3-methylthiobenzoesäureethylester

104.4 g (398.1 mmol) 4-Difluormethyl-2-hydroxy-3-methylthiobenzoesäureethylester wurden in 310 ml Aceton mit 66.02 g (477.7 mmol) Kaliumcarbonat versetzt. Hierzu wurde eine Mischung aus 6.04 g (59.7 mmol) Triethylamin und 7.53 g (59.7 mmol) Dimethylsulfat zugegeben. Danach wurden 57.74 g (457.8 mmol) Dimethylsulfat zugetropft. Das Reaktionsgemisch wurde anschließend 16 h bei Raumtemperatur (RT) gerührt. Zur Aufarbeitung wurde das Reaktionsgemisch vom Lösungsmittel befreit und der Rückstand wurde mit 1000 ml IM Natronlauge 2 h gerührt. Das Gemisch wurde mit CH₂Cl₂ versetzt und nach der Phasentrennung wurde die organische Phase getrocknet. Das Filtrat wurde vom Lösungsmittel befreit. Als Rückstand wurden 104.7 g des gewünschten Produkts gewonnen.

### Schritt 2: Synthese von 4-Difluormethyl-2-methoxy-3-methylthiobenzoesäure

104.7 g (378.9 mmol) 4-Difluormethyl-2-methoxy-3-methylthiobenzoesäureethylester wurden mit einer Mischung aus 420 ml 1M Natronlauge und 715 ml Methanol 16 h bei RT gerührt. Zur Aufarbeitung wurde das Methanol entfernt. Der Rückstand wurde mit Essigsäureethylester extrahiert und die wässrige Phase wurde anschließend mit Salzsäure angesäuert. Das Gemisch wurde danach zweimal mit Essigsäureethylester extrahiert. Die vereinigten organischen Phasen wurden getrocknet und das Filtrat wurde vom Lösungsmittel befreit. Als Rückstand wurden 90.0 g des gewünschten Produkts gewonnen.

### Schritt 3: Synthese von 2-[4-(Difluormethyl)-2-methoxy-3-(methylsulfanyl)phenyl]-4,4-dimethyl-4,5-dihydro-1,3-oxazol

40.0 g (161.1 mmol) 4-Difluormethyl-2-methoxy-3-methylthiobenzoesäure wurden in 600 ml Dichlormethan mit 1.24 ml (16.1 mmol) N,N-Dimethylformamid versetzt. Anschließend wurden 23.2 ml (265.9 mmol) Oxalsäuredichlorid tropfenweise zugegeben. Das Reaktionsgemnisch wurde 16 h bei RT gerührt. Zur Vervollständigung der Reaktion wurden anschließend 2.81 ml (32.2 mmol) Oxalsäuredichlorid zugegeben und das Gemisch weitere 24 h bei RT gerührt. Danach wurde der Inhalt eingeengt und der Rückstand wurde erneut in 300 ml CH₂Cl₂ gelöst. Anschließend wurde eine Lösung aus 15.08 g (169.2 mmol) 2-Amino-2-methyl-1-propanol in 10%-iger Natronlauge tropfenweise unter leichter Eisbadkühlung zugegeben. Das Gemisch wurde 24 h bei RT gerührt. Zur Aufarbeitung wurde das Gemisch zunächst mit CH₂Cl₂ und etwas Wasser verdünnt. Nach der Phasentrennung wurde die wässrige Phase mit CH₂Cl₂ extrahiert. Die vereinigten organischen Phasen wurden getrocknet und das Filtrat wurde eingeengt. Zur Ausführung des zweiten Reaktionsschritts wurde der Rückstand in 600 ml CH₂Cl₂ gelöst und mit 32.9 ml (451.2 mmol) Thionylchlorid versetzt. Das Reaktionsgemisch wurde anschließend 16 h bei RT gerührt. Zur Aufarbeitung wurden unter Eisbadkühlung 500 ml 10%-ige Natronlauge innerhalb von 2 h zugegeben, gefolgt von der Zugabe von weiteren 100 ml 10%-iger Natronlauge. Nach der Phasentrennung wurde die wässrige Phase mit CH₂Cl₂ extrahiert. Die vereinigten organischen Phasen wurden getrocknet und das Filtrat wurde eingeengt. Der Rückstand wurde mit 200 ml 6M Salzsäure versetzt und das Gemisch wurde dreimal mit je 60 ml CH₂Cl₂ extrahiert. Die vereinigten organischen Phasen wurden zweimal mit je 25 ml 6M Salzsäure, anschließend zweimal mit je 20 ml 6M Salzsäure extrahiert. Die Salzsäure-Phasen wurden im Eisbad gekühlt und mit festem NaOH portionsweise alkalisiert. Dann wurde das Gemisch zweimal mit je 200 ml CH₂Cl₂, danach noch einmal mit 100 ml CH₂Cl₂ extrahiert. Die organischen Phasen wurden getrocknet und das Filtrat wurde vom Lösungsmittel befreit. Als Rückstand wurden 32.6 g des gewünschten Produkts gewonnen.

### Schritt 4: Synthese von 2-[4-(Difluormethyl)-2-methyl-3-(methylsulfanyl)phenyl]-4,4-dimethyl-4,5-dihydro-1,3-oxazol

Eine Lösung von 17.0 g (56.4 mmol) 2-[4-(Difluormethyl)-2-methoxy-3-(methylsulfanyl)phenyl]-4,4-dimethyl-4,5-dihydro-1,3-oxazol in 280 ml Diethylether wurde bei RT tropfenweise mit 84.6 ml einer 1M Lösung (84.6 mmol) von Methylmagnesiumbromid in THF versetzt. Nach 2 h wurden weitere 56 ml der 1M Lösung (56 mmol) von Methylmagnesiumbromid in THF innerhalb von 3 h tropfenweise zugegeben. Das Gemisch wurde 72 h bei RT gerührt. Zur Aufarbeitung wurde der Inhalt vorsichtig auf eine Mischung aus Eis und verdünnter Salzsäure gegossen. Das Gemisch wurde mit NaOH neutralisiert und zweimal mit Diethylether extrahiert. Die organischen Phasen wurden getrocknet und das Filtrat wurde vom Lösungsmittel befreit. Als Rückstand wurden 15.9 g des gewünschten Produkts gewonnen.

### Schritt 5: Synthese von 4-Difluormethyl-2-methyl-3-methylthiobenzoesäure

15.47 g (54.2 mmol) 2-[4-(Difluormethyl)-2-methyl-3-(methylsulfanyl)phenyl]-4,4-dimethyl-4,5-dihydro-1,3-oxazol wurden in 250 ml Aceton mit 30.9 ml (497 mmol) Iodmethan versetzt und anschließend 2 h bei 40°C gerührt. Anschließend wurden weitere 25 ml (402 mmol) Iodmethan zugegeben und das Gemisch wurde danach 20 h bei einer Temperatur von 40°C gerührt. Zur Vervollständigung der Reaktion wurden danach nochmals 10 ml (161 mmol) Iodmethan zugegeben und das Gemisch wurde anschließend 15 h bei einer Temperatur von 40°C gerührt. Zur Aufarbeitung wurde das Reaktionsgemisch auf RT abgekühlt und eingeengt. Zur Umsetzung des zweiten Reaktionsschritts wurde der Rückstand mit 150 ml Methanol und 150 ml 20%-iger Natronlauge versetzt und 2 h unter Rückfluss erhitzt. Abschließend wurde das Reaktionsgemisch 72 h bei RT gerührt. Zur Aufarbeitung wurde der Inhalt eingeengt und der Rückstand wurde in wenig Wasser aufgenommen. Das Gemisch wurde mit CH₂Cl₂ gewaschen und die wässrige Phase wurde mit konzentrierter Salzsäure angesäuert. Anschließend wurde das Gemisch mit CH₂Cl₂ extrahiert. Die organische Phase wurde vom Lösungsmittel befreit. Als Rückstand wurden 11.8 g des gewünschten Produkts gewonnen.

### Schritt 6: Synthese von 4-(Difluormethyl)-2-methyl-3-(methylsulfanyl)-N-(1-methyl-1H-1,2,4-triazol-5-yl)benzamid (Nr. 4-1)

232.2 mg (1.0 mmol) 4-Difluormethyl-2-methyl-3-methylthiobenzoesäure wurden bei RT in 5 ml Pyridin mit 137.4 mg (1.4 mmol) 1-Methyl-1H-1,2,4-triazol-5-amin versetzt. Unter Kühlung wurden 177.7 mg (1.4 mmol) Oxalsäuredichlorid zugegeben und anschließend 16 h bei RT gerührt. Zur Aufarbeitung wurde das Gemisch eingeengt und der Rückstand wurde in CH₂Cl₂ aufgenommen. Das Gemisch wurde einmal mit einer wässrigen NaHCO₃-Lösung und danach einmal mit Wasser extrahiert. Die organische Phase wurde getrocknet und das Filtrat wurde eingeengt. Der Rückstand wurde chromatographisch gereinigt, wobei 62.6 mg des gewünschten Produkts isoliert wurden.

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:

| | | | |
|---|---|---|---|
| Ph = Phenyl | Me = Methyl | Et = Ethyl | c-Pr = cyclo-Propyl |

**Tabelle 1: Erfindungsgemäße Verbindungen der allgemeinen Formel (I), worin Q für Q1 und R^{x} für eine Methylgruppe stehen, sowie R^{a} Wasserstoff bedeutet**

| 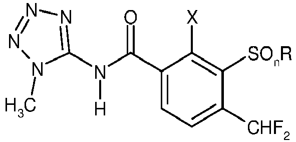 | | | |
|---|---|---|---|
| **Nr.** | **X** | **n** | **R** |
| 1-1 | Me | 0 | Me |
| 1-2 | Me | 1 | Me |
| 1-3 | Me | 2 | Me |
| 1-4 | Me | 0 | Et |
| 1-5 | Me | 1 | Et |
| 1-6 | Me | 2 | Et |
| 1-7 | Me | 0 | CH₂c-Pr |
| 1-8 | Me | 1 | CH₂c-Pr |
| 1-9 | Me | 2 | CH₂c-Pr |
| 1-10 | Me | 0 | CH₂CH₂OMe |
| 1-11 | Me | 1 | CH₂CH₂OMe |
| 1-12 | Me | 2 | CH₂CH₂OMe |
| 1-13 | Et | 0 | Me |
| 1-14 | Et | 1 | Me |
| 1-15 | Et | 2 | Me |
| 1-16 | Et | 0 | Et |
| 1-17 | Et | 1 | Et |
| 1-18 | Et | 2 | Et |
| 1-19 | Et | 0 | CH₂c-Pr |
| 1-20 | Et | 1 | CH₂c-Pr |
| 1-21 | Et | 2 | CH₂c-Pr |
| 1-22 | Et | 0 | CH₂CH₂OMe |
| 1-23 | Et | 1 | CH₂CH₂OMe |
| 1-24 | Et | 2 | CH₂CH₂OMe |
| 1-25 | c-Pr | 0 | Me |
| 1-26 | c-Pr | 1 | Me |
| 1-27 | c-Pr | 2 | Me |
| 1-28 | c-Pr | 0 | Et |
| 1-29 | c-Pr | 1 | Et |
| 1-30 | c-Pr | 2 | Et |
| 1-31 | c-Pr | 0 | CH₂c-Pr |
| 1-32 | c-Pr | 1 | CH₂c-Pr |
| 1-33 | c-Pr | 2 | CH₂c-Pr |
| 1-34 | c-Pr | 0 | CH₂CH₂OMe |
| 1-35 | c-Pr | 1 | CH₂CH₂OMe |
| 1-36 | c-Pr | 2 | CH₂CH₂OMe |
| 1-37 | F | 0 | Me |
| 1-38 | F | 1 | Me |
| 1-39 | F | 2 | Me |
| 1-40 | F | 0 | Et |
| 1-41 | F | 1 | Et |
| 1-42 | F | 2 | Et |
| 1-43 | F | 0 | CH₂c-Pr |
| 1-44 | F | 1 | CH₂c-Pr |
| 1-45 | F | 2 | CH₂c-Pr |
| 1-46 | F | 0 | CH₂CH₂OMe |
| 1-47 | F | 1 | CH₂CH₂OMe |
| 1-48 | F | 2 | CH₂CH₂OMe |
| 1-49 | Cl | 0 | Me |
| 1-50 | Cl | 1 | Me |
| 1-51 | Cl | 2 | Me |
| 1-52 | Cl | 0 | Et |
| 1-53 | Cl | 1 | Et |
| 1-54 | Cl | 2 | Et |
| 1-55 | Cl | 0 | CH₂c-Pr |
| 1-56 | Cl | 1 | CH₂c-Pr |
| 1-57 | Cl | 2 | CH₂c-Pr |
| 1-58 | Cl | 0 | CH₂CH₂OMe |
| 1-59 | Cl | 1 | CH₂CH₂OMe |
| 1-60 | Cl | 2 | CH₂CH₂OMe |
| 1-61 | Br | 0 | Me |
| 1-62 | Br | 1 | Me |
| 1-63 | Br | 2 | Me |
| 1-64 | Br | 0 | Et |
| 1-65 | Br | 1 | Et |
| 1-66 | Br | 2 | Et |
| 1-67 | Br | 0 | CH₂c-Pr |
| 1-68 | Br | 1 | CH₂c-Pr |
| 1-69 | Br | 2 | CH₂c-Pr |
| 1-70 | Br | 0 | CH₂CH₂OMe |
| 1-71 | Br | 1 | CH₂CH₂OMe |
| 1-72 | Br | 2 | CH₂CH₂OMe |
| 1-73 | I | 0 | Me |
| 1-74 | I | 1 | Me |
| 1-75 | I | 2 | Me |
| 1-76 | I | 0 | Et |
| 1-77 | I | 1 | Et |
| 1-78 | I | 2 | Et |
| 1-79 | I | 0 | CH₂c-Pr |
| 1-80 | I | 1 | CH₂c-Pr |
| 1-81 | I | 2 | CH₂c-Pr |
| 1-82 | I | 0 | CH₂CH₂OMe |
| 1-83 | I | 1 | CH₂CH₂OMe |
| 1-84 | I | 2 | CH₂CH₂OMe |

**Tabelle 2: Erfindungsgemäße Verbindungen der allgemeinen Formel (I), worin Q für Q1 und R^{x} für eine Ethylgruppe stehen, sowie R^{a} Wasserstoff bedeutet.**

| 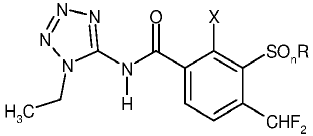 | | | |
|---|---|---|---|
| **Nr.** | **X** | **n** | **R** |
| 2-1 | Me | 0 | Me |
| 2-2 | Me | 1 | Me |
| 2-3 | Me | 2 | Me |
| 2-4 | Me | 0 | Et |
| 2-5 | Me | 1 | Et |
| 2-6 | Me | 2 | Et |
| 2-7 | Me | 0 | CH₂c-Pr |
| 2-8 | Me | 1 | CH₂c-Pr |
| 2-9 | Me | 2 | CH₂c-Pr |
| 2-10 | Me | 0 | CH₂CH₂OMe |
| 2-11 | Me | 1 | CH₂CH₂OMe |
| 2-12 | Me | 2 | CH₂CH₂OMe |
| 2-13 | Et | 0 | Me |
| 2-14 | Et | 1 | Me |
| 2-15 | Et | 2 | Me |
| 2-16 | Et | 0 | Et |
| 2-17 | Et | 1 | Et |
| 2-18 | Et | 2 | Et |
| 2-19 | Et | 0 | CH₂c-Pr |
| 2-20 | Et | 1 | CH₂c-Pr |
| 2-21 | Et | 2 | CH₂c-Pr |
| 2-22 | Et | 0 | CH₂CH₂OMe |
| 2-23 | Et | 1 | CH₂CH₂OMe |
| 2-24 | Et | 2 | CH₂CH₂OMe |
| 2-25 | c-Pr | 0 | Me |
| 2-26 | c-Pr | 1 | Me |
| 2-27 | c-Pr | 2 | Me |
| 2-28 | c-Pr | 0 | Et |
| 2-29 | c-Pr | 1 | Et |
| 2-30 | c-Pr | 2 | Et |
| 2-31 | c-Pr | 0 | CH₂c-Pr |
| 2-32 | c-Pr | 1 | CH₂c-Pr |
| 2-33 | c-Pr | 2 | CH₂c-Pr |
| 2-34 | c-Pr | 0 | CH₂CH₂OMe |
| 2-35 | c-Pr | 1 | CH₂CH₂OMe |
| 2-36 | c-Pr | 2 | CH₂CH₂OMe |
| 2-37 | F | 0 | Me |
| 2-38 | F | 1 | Me |
| 2-39 | F | 2 | Me |
| 2-40 | F | 0 | Et |
| 2-41 | F | 1 | Et |
| 2-42 | F | 2 | Et |
| 2-43 | F | 0 | CH₂c-Pr |
| 2-44 | F | 1 | CH₂c-Pr |
| 2-45 | F | 2 | CH₂c-Pr |
| 2-46 | F | 0 | CH₂CH₂OMe |
| 2-47 | F | 1 | CH₂CH₂OMe |
| 2-48 | F | 2 | CH₂CH₂OMe |
| 2-49 | Cl | 0 | Me |
| 2-50 | Cl | 1 | Me |
| 2-51 | Cl | 2 | Me |
| 2-52 | Cl | 0 | Et |
| 2-53 | Cl | 1 | Et |
| 2-54 | Cl | 2 | Et |
| 2-55 | Cl | 0 | CH₂c-Pr |
| 2-56 | Cl | 1 | CH₂c-Pr |
| 2-57 | Cl | 2 | CH₂c-Pr |
| 2-58 | Cl | 0 | CH₂CH₂OMe |
| 2-59 | Cl | 1 | CH₂CH₂OMe |
| 2-60 | Cl | 2 | CH₂CH₂OMe |
| 2-61 | Br | 0 | Me |
| 2-62 | Br | 1 | Me |
| 2-63 | Br | 2 | Me |
| 2-64 | Br | 0 | Et |
| 2-65 | Br | 1 | Et |
| 2-66 | Br | 2 | Et |
| 2-67 | Br | 0 | CH₂c-Pr |
| 2-68 | Br | 1 | CH₂c-Pr |
| 2-69 | Br | 2 | CH₂c-Pr |
| 2-70 | Br | 0 | CH₂CH₂OMe |
| 2-71 | Br | 1 | CH₂CH₂OMe |
| 2-72 | Br | 2 | CH₂CH₂OMe |
| 2-73 | I | 0 | Me |
| 2-74 | I | 1 | Me |
| 2-75 | I | 2 | Me |
| 2-76 | I | 0 | Et |
| 2-77 | I | 1 | Et |
| 2-78 | I | 2 | Et |
| 2-79 | I | 0 | CH₂c-Pr |
| 2-80 | I | 1 | CH₂c-Pr |
| 2-81 | I | 2 | CH₂c-Pr |
| 2-82 | I | 0 | CH₂CH₂OMe |
| 2-83 | I | 1 | CH₂CH₂OMe |
| 2-84 | I | 2 | CH₂CH₂OMe |

**Tabelle 3: Erfindungsgemäße Verbindungen der allgemeinen Formel (I), worin Q für Q1 und R^{x} für eine n-Propylgruppe stehen, sowie R^{a} Wasserstoff bedeutet,**

| 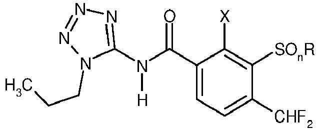 | | | |
|---|---|---|---|
| **Nr.** | **X** | **n** | **R** |
| 3-1 | Me | 0 | Me |
| 3-2 | Me | 1 | Me |
| 3-3 | Me | 2 | Me |
| 3-4 | Me | 0 | Et |
| 3-5 | Me | 1 | Et |
| 3-6 | Me | 2 | Et |
| 3-7 | Me | 0 | CH₂c-Pr |
| 3-8 | Me | 1 | CH₂c-Pr |
| 3-9 | Me | 2 | CH₂c-Pr |
| 3-10 | Me | 0 | CH₂CH₂OMe |
| 3-11 | Me | 1 | CH₂CH₂OMe |
| 3-12 | Me | 2 | CH₂CH₂OMe |
| 3-13 | Et | 0 | Me |
| 3-14 | Et | 1 | Me |
| 3-15 | Et | 2 | Me |
| 3-16 | Et | 0 | Et |
| 3-17 | Et | 1 | Et |
| 3-18 | Et | 2 | Et |
| 3-19 | Et | 0 | CH₂c-Pr |
| 3-20 | Et | 1 | CH₂c-Pr |
| 3-21 | Et | 2 | CH₂c-Pr |
| 3-22 | Et | 0 | CH₂CH₂OMe |
| 3-23 | Et | 1 | CH₂CH₂OMe |
| 3-24 | Et | 2 | CH₂CH₂OMe |
| 3-25 | c-Pr | 0 | Me |
| 3-26 | c-Pr | 1 | Me |
| 3-27 | c-Pr | 2 | Me |
| 3-28 | c-Pr | 0 | Et |
| 3-29 | c-Pr | 1 | Et |
| 3-30 | c-Pr | 2 | Et |
| 3-31 | c-Pr | 0 | CH₂c-Pr |
| 3-32 | c-Pr | 1 | CH₂c-Pr |
| 3-33 | c-Pr | 2 | CH₂c-Pr |
| 3-34 | c-Pr | 0 | CH₂CH₂OMe |
| 3-35 | c-Pr | 1 | CH₂CH₂OMe |
| 3-36 | c-Pr | 2 | CH₂CH₂OMe |
| 3-37 | F | 0 | Me |
| 3-38 | F | 1 | Me |
| 3-39 | F | 2 | Me |
| 3-40 | F | 0 | Et |
| 3-41 | F | 1 | Et |
| 3-42 | F | 2 | Et |
| 3-43 | F | 0 | CH₂c-Pr |
| 3-44 | F | 1 | CH₂c-Pr |
| 3-45 | F | 2 | CH₂c-Pr |
| 3-46 | F | 0 | CH₂CH₂OMe |
| 3-47 | F | 1 | CH₂CH₂OMe |
| 3-48 | F | 2 | CH₂CH₂OMe |
| 3-49 | Cl | 0 | Me |
| 3-50 | Cl | 1 | Me |
| 3-51 | Cl | 2 | Me |
| 3-52 | Cl | 0 | Et |
| 3-53 | Cl | 1 | Et |
| 3-54 | Cl | 2 | Et |
| 3-55 | Cl | 0 | CH₂c-Pr |
| 3-56 | Cl | 1 | CH₂c-Pr |
| 3-57 | Cl | 2 | CH₂c-Pr |
| 3-58 | Cl | 0 | CH₂CH₂OMe |
| 3-59 | Cl | 1 | CH₂CH₂OMe |
| 3-60 | Cl | 2 | CH₂CH₂OMe |
| 3-61 | Br | 0 | Me |
| 3-62 | Br | 1 | Me |
| 3-63 | Br | 2 | Me |
| 3-64 | Br | 0 | Et |
| 3-65 | Br | 1 | Et |
| 3-66 | Br | 2 | Et |
| 3-67 | Br | 0 | CH₂c-Pr |
| 3-68 | Br | 1 | CH₂c-Pr |
| 3-69 | Br | 2 | CH₂c-Pr |
| 3-70 | Br | 0 | CH₂CH₂OMe |
| 3-71 | Br | 1 | CH₂CH₂OMe |
| 3-72 | Br | 2 | CH₂CH₂OMe |
| 3-73 | I | 0 | Me |
| 3-74 | I | 1 | Me |
| 3-75 | I | 2 | Me |
| 3-76 | I | 0 | Et |
| 3-77 | I | 1 | Et |
| 3-78 | I | 2 | Et |
| 3-79 | I | 0 | CH₂c-Pr |
| 3-80 | I | 1 | CH₂c-Pr |
| 3-81 | I | 2 | CH₂c-Pr |
| 3-82 | I | 0 | CH₂CH₂OMe |
| 3-83 | I | 1 | CH₂CH₂OMe |
| 3-84 | I | 2 | CH₂CH₂OMe |

**Tabelle 4: Erfindungsgemäße Verbindungen der allgemeinen Formel (I), worin Q für Q2 und R^{x} für eine Methylgruppe stehen, sowie R^{a} Wasserstoff bedeutet**

| 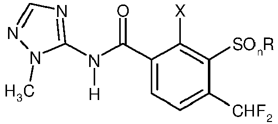 | | | |
|---|---|---|---|
| **Nr.** | **X** | **n** | **R** |
| 4-1 | Me | 0 | Me |
| 4-2 | Me | 1 | Me |
| 4-3 | Me | 2 | Me |
| 4-4 | Me | 0 | Et |
| 4-5 | Me | 1 | Et |
| 4-6 | Me | 2 | Et |
| 4-7 | Me | 0 | CH₂c-Pr |
| 4-8 | Me | 1 | CH₂c-Pr |
| 4-9 | Me | 2 | CH₂c-Pr |
| 4-10 | Me | 0 | CH₂CH₂OMe |
| 4-11 | Me | 1 | CH₂CH₂OMe |
| 4-12 | Me | 2 | CH₂CH₂OMe |
| 4-13 | Et | 0 | Me |
| 4-14 | Et | 1 | Me |
| 4-15 | Et | 2 | Me |
| 4-16 | Et | 0 | Et |
| 4-17 | Et | 1 | Et |
| 4-18 | Et | 2 | Et |
| 4-19 | Et | 0 | CH₂c-Pr |
| 4-20 | Et | 1 | CH₂c-Pr |
| 4-21 | Et | 2 | CH₂c-Pr |
| 4-22 | Et | 0 | CH₂CH₂OMe |
| 4-23 | Et | 1 | CH₂CH₂OMe |
| 4-24 | Et | 2 | CH₂CH₂OMe |
| 4-25 | c-Pr | 0 | Me |
| 4-26 | c-Pr | 1 | Me |
| 4-27 | c-Pr | 2 | Me |
| 4-28 | c-Pr | 0 | Et |
| 4-29 | c-Pr | 1 | Et |
| 4-30 | c-Pr | 2 | Et |
| 4-31 | c-Pr | 0 | CH₂c-Pr |
| 4-32 | c-Pr | 1 | CH₂c-Pr |
| 4-33 | c-Pr | 2 | CH₂c-Pr |
| 4-34 | c-Pr | 0 | CH₂CH₂OMe |
| 4-35 | c-Pr | 1 | CH₂CH₂OMe |
| 4-36 | c-Pr | 2 | CH₂CH₂OMe |
| 4-37 | F | 0 | Me |
| 4-38 | F | 1 | Me |
| 4-39 | F | 2 | Me |
| 4-40 | F | 0 | Et |
| 4-41 | F | 1 | Et |
| 4-42 | F | 2 | Et |
| 4-43 | F | 0 | CH₂c-Pr |
| 4-44 | F | 1 | CH₂c-Pr |
| 4-45 | F | 2 | CH₂c-Pr |
| 4-46 | F | 0 | CH₂CH₂OMe |
| 4-47 | F | 1 | CH₂CH₂OMe |
| 4-48 | F | 2 | CH₂CH₂OMe |
| 4-49 | Cl | 0 | Me |
| 4-50 | Cl | 1 | Me |
| 4-51 | Cl | 2 | Me |
| 4-52 | Cl | 0 | Et |
| 4-53 | Cl | 1 | Et |
| 4-54 | Cl | 2 | Et |
| 4-55 | Cl | 0 | CH₂c-Pr |
| 4-56 | Cl | 1 | CH₂c-Pr |
| 4-57 | Cl | 2 | CH₂c-Pr |
| 4-58 | Cl | 0 | CH₂CH₂OMe |
| 4-59 | Cl | 1 | CH₂CH₂OMe |
| 4-60 | Cl | 2 | CH₂CH₂OMe |
| 4-61 | Br | 0 | Me |
| 4-62 | Br | 1 | Me |
| 4-63 | Br | 2 | Me |
| 4-64 | Br | 0 | Et |
| 4-65 | Br | 1 | Et |
| 4-66 | Br | 2 | Et |
| 4-67 | Br | 0 | CH₂c-Pr |
| 4-68 | Br | 1 | CH₂c-Pr |
| 4-69 | Br | 2 | CH₂c-Pr |
| 4-70 | Br | 0 | CH₂CH₂OMe |
| 4-71 | Br | 1 | CH₂CH₂OMe |
| 4-72 | Br | 2 | CH₂CH₂OMe |
| 4-73 | I | 0 | Me |
| 4-74 | I | 1 | Me |
| 4-75 | I | 2 | Me |
| 4-76 | I | 0 | Et |
| 4-77 | I | 1 | Et |
| 4-78 | I | 2 | Et |
| 4-79 | I | 0 | CH₂c-Pr |
| 4-80 | I | 1 | CH₂c-Pr |
| 4-81 | I | 2 | CH₂c-Pr |
| 4-82 | I | 0 | CH₂CH₂OMe |
| 4-83 | I | 1 | CH₂CH₂OMe |
| 4-84 | I | 2 | CH₂CH₂OMe |

**Tabelle 5: Erfindungsgemäße Verbindungen der allgemeinen Formel (I), worin Q für Q3 und R^{y} für eine Methylgruppe stehen, sowie R^{a} Wasserstoff bedeutet**

| 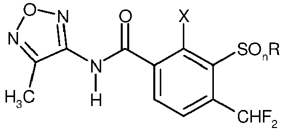 | | | |
|---|---|---|---|
| **Nr.** | **X** | **n** | **R** |
| 5-1 | Me | 0 | Me |
| 5-2 | Me | 1 | Me |
| 5-3 | Me | 2 | Me |
| 5-4 | Me | 0 | Et |
| 5-5 | Me | 1 | Et |
| 5-6 | Me | 2 | Et |
| 5-7 | Me | 0 | CH₂c-Pr |
| 5-8 | Me | 1 | CH₂c-Pr |
| 5-9 | Me | 2 | CH₂c-Pr |
| 5-10 | Me | 0 | CH₂CH₂OMe |
| 5-11 | Me | 1 | CH₂CH₂OMe |
| 5-12 | Me | 2 | CH₂CH₂OMe |
| 5-13 | Et | 0 | Me |
| 5-14 | Et | 1 | Me |
| 5-15 | Et | 2 | Me |
| 5-16 | Et | 0 | Et |
| 5-17 | Et | 1 | Et |
| 5-18 | Et | 2 | Et |
| 5-19 | Et | 0 | CH₂c-Pr |
| 5-20 | Et | 1 | CH₂c-Pr |
| 5-21 | Et | 2 | CH₂c-Pr |
| 5-22 | Et | 0 | CH₂CH₂OMe |
| 5-23 | Et | 1 | CH₂CH₂OMe |
| 5-24 | Et | 2 | CH₂CH₂OMe |
| 5-25 | c-Pr | 0 | Me |
| 5-26 | c-Pr | 1 | Me |
| 5-27 | c-Pr | 2 | Me |
| 5-28 | c-Pr | 0 | Et |
| 5-29 | c-Pr | 1 | Et |
| 5-30 | c-Pr | 2 | Et |
| 5-31 | c-Pr | 0 | CH₂c-Pr |
| 5-32 | c-Pr | 1 | CH₂c-Pr |
| 5-33 | c-Pr | 2 | CH₂c-Pr |
| 5-34 | c-Pr | 0 | CH₂CH₂OMe |
| 5-35 | c-Pr | 1 | CH₂CH₂OMe |
| 5-36 | c-Pr | 2 | CH₂CH₂OMe |
| 5-37 | F | 0 | Me |
| 5-38 | F | 1 | Me |
| 5-39 | F | 2 | Me |
| 5-40 | F | 0 | Et |
| 5-41 | F | 1 | Et |
| 5-42 | F | 2 | Et |
| 5-43 | F | 0 | CH₂c-Pr |
| 5-44 | F | 1 | CH₂c-Pr |
| 5-45 | F | 2 | CH₂c-Pr |
| 5-46 | F | 0 | CH₂CH₂OMe |
| 5-47 | F | 1 | CH₂CH₂OMe |
| 5-48 | F | 2 | CH₂CH₂OMe |
| 5-49 | Cl | 0 | Me |
| 5-50 | Cl | 1 | Me |
| 5-51 | Cl | 2 | Me |
| 5-52 | Cl | 0 | Et |
| 5-53 | Cl | 1 | Et |
| 5-54 | Cl | 2 | Et |
| 5-55 | Cl | 0 | CH₂c-Pr |
| 5-56 | Cl | 1 | CH₂c-Pr |
| 5-57 | Cl | 2 | CH₂c-Pr |
| 5-58 | Cl | 0 | CH₂CH₂OMe |
| 5-59 | Cl | 1 | CH₂CH₂OMe |
| 5-60 | Cl | 2 | CH₂CH₂OMe |
| 5-61 | Br | 0 | Me |
| 5-62 | Br | 1 | Me |
| 5-63 | Br | 2 | Me |
| 5-64 | Br | 0 | Et |
| 5-65 | Br | 1 | Et |
| 5-66 | Br | 2 | Et |
| 5-67 | Br | 0 | CH₂c-Pr |
| 5-68 | Br | 1 | CH₂c-Pr |
| 5-69 | Br | 2 | CH₂c-Pr |
| 5-70 | Br | 0 | CH₂CH₂OMe |
| 5-71 | Br | 1 | CH₂CH₂OMe |
| 5-72 | Br | 2 | CH₂CH₂OMe |
| 5-73 | I | 0 | Me |
| 5-74 | I | 1 | Me |
| 5-75 | I | 2 | Me |
| 5-76 | I | 0 | Et |
| 5-77 | I | 1 | Et |
| 5-78 | I | 2 | Et |
| 5-79 | I | 0 | CH₂c-Pr |
| 5-80 | I | 1 | CH₂c-Pr |
| 5-81 | I | 2 | CH₂c-Pr |
| 5-82 | I | 0 | CH₂CH₂OMe |
| 5-83 | I | 1 | CH₂CH₂OMe |
| 5-84 | I | 2 | CH₂CH₂OMe |

**Tabelle 6: Erfindungsgemäße Verbindungen der allgemeinen Formel (I), worin Q für Q3 und R^{y} für Chlor stehen, sowie R^{a} Wasserstoff bedeutet**

| 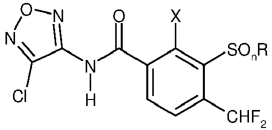 | | | |
|---|---|---|---|
| **Nr.** | **X** | **n** | **R** |
| 6-1 | Me | 0 | Me |
| 6-2 | Me | 1 | Me |
| 6-3 | Me | 2 | Me |
| 6-4 | Me | 0 | Et |
| 6-5 | Me | 1 | Et |
| 6-6 | Me | 2 | Et |
| 6-7 | Me | 0 | CH₂c-Pr |
| 6-8 | Me | 1 | CH₂c-Pr |
| 6-9 | Me | 2 | CH₂c-Pr |
| 6-10 | Me | 0 | CH₂CH₂OMe |
| 6-11 | Me | 1 | CH₂CH₂OMe |
| 6-12 | Me | 2 | CH₂CH₂OMe |
| 6-13 | Et | 0 | Me |
| 6-14 | Et | 1 | Me |
| 6-15 | Et | 2 | Me |
| 6-16 | Et | 0 | Et |
| 6-17 | Et | 1 | Et |
| 6-18 | Et | 2 | Et |
| 6-19 | Et | 0 | CH₂c-Pr |
| 6-20 | Et | 1 | CH₂c-Pr |
| 6-21 | Et | 2 | CH₂c-Pr |
| 6-22 | Et | 0 | CH₂CH₂OMe |
| 6-23 | Et | 1 | CH₂CH₂OMe |
| 6-24 | Et | 2 | CH₂CH₂OMe |
| 6-25 | c-Pr | 0 | Me |
| 6-26 | c-Pr | 1 | Me |
| 6-27 | c-Pr | 2 | Me |
| 6-28 | c-Pr | 0 | Et |
| 6-29 | c-Pr | 1 | Et |
| 6-30 | c-Pr | 2 | Et |
| 6-31 | c-Pr | 0 | CH₂c-Pr |
| 6-32 | c-Pr | 1 | CH₂c-Pr |
| 6-33 | c-Pr | 2 | CH₂c-Pr |
| 6-34 | c-Pr | 0 | CH₂CH₂OMe |
| 6-35 | c-Pr | 1 | CH₂CH₂OMe |
| 6-36 | c-Pr | 2 | CH₂CH₂OMe |
| 6-37 | F | 0 | Me |
| 6-38 | F | 1 | Me |
| 6-39 | F | 2 | Me |
| 6-40 | F | 0 | Et |
| 6-41 | F | 1 | Et |
| 6-42 | F | 2 | Et |
| 6-43 | F | 0 | CH₂c-Pr |
| 6-44 | F | 1 | CH₂c-Pr |
| 6-45 | F | 2 | CH₂c-Pr |
| 6-46 | F | 0 | CH₂CH₂OMe |
| 6-47 | F | 1 | CH₂CH₂OMe |
| 6-48 | F | 2 | CH₂CH₂OMe |
| 6-49 | Cl | 0 | Me |
| 6-50 | Cl | 1 | Me |
| 6-51 | Cl | 2 | Me |
| 6-52 | Cl | 0 | Et |
| 6-53 | Cl | 1 | Et |
| 6-54 | Cl | 2 | Et |
| 6-55 | Cl | 0 | CH₂c-Pr |
| 6-56 | Cl | 1 | CH₂c-Pr |
| 6-57 | Cl | 2 | CH₂c-Pr |
| 6-58 | Cl | 0 | CH₂CH₂OMe |
| 6-59 | Cl | 1 | CH₂CH₂OMe |
| 6-60 | Cl | 2 | CH₂CH₂OMe |
| 6-61 | Br | 0 | Me |
| 6-62 | Br | 1 | Me |
| 6-63 | Br | 2 | Me |
| 6-64 | Br | 0 | Et |
| 6-65 | Br | 1 | Et |
| 6-66 | Br | 2 | Et |
| 6-67 | Br | 0 | CH₂c-Pr |
| 6-68 | Br | 1 | CH₂c-Pr |
| 6-69 | Br | 2 | CH₂c-Pr |
| 6-70 | Br | 0 | CH₂CH₂OMe |
| 6-71 | Br | 1 | CH₂CH₂OMe |
| 6-72 | Br | 2 | CH₂CH₂OMe |
| 6-73 | I | 0 | Me |
| 6-74 | I | 1 | Me |
| 6-75 | I | 2 | Me |
| 6-76 | I | 0 | Et |
| 6-77 | I | 1 | Et |
| 6-78 | I | 2 | Et |
| 6-79 | I | 0 | CH₂c-Pr |
| 6-80 | I | 1 | CH₂c-Pr |
| 6-81 | I | 2 | CH₂c-Pr |
| 6-82 | I | 0 | CH₂CH₂OMe |
| 6-83 | I | 1 | CH₂CH₂OMe |
| 6-84 | I | 2 | CH₂CH₂OMe |

**Tabelle 7: Erfindungsgemäße Verbindungen der allgemeinen Formel (I), worin Q für Q4 und R^{z} für eine Methylgruppe stehen sowie R^{a} Wasserstoff bedeutet**

| 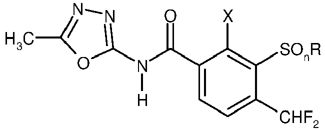 | | | |
|---|---|---|---|
| **Nr.** | **X** | **n** | **R** |
| 7-1 | Me | 0 | Me |
| 7-2 | Me | 1 | Me |
| 7-3 | Me | 2 | Me |
| 7-4 | Me | 0 | Et |
| 7-5 | Me | 0 | CH₂c-Pr |
| 7-6 | Me | 1 | CH₂c-Pr |
| 7-7 | Me | 2 | CH₂c-Pr |
| 7-8 | Me | 0 | CH₂CH₂OMe |
| 7-9 | Me | 1 | CH₂CH₂OMe |
| 7-10 | Me | 2 | CH₂CH₂OMe |
| 7-11 | Et | 0 | Me |
| 7-12 | Et | 1 | Me |
| 7-13 | Et | 2 | Me |
| 7-14 | Et | 0 | Et |
| 7-15 | Et | 1 | Et |
| 7-16 | Et | 2 | Et |
| 7-17 | Et | 0 | CH₂c-Pr |
| 7-18 | Et | 1 | CH₂c-Pr |
| 7-19 | Et | 2 | CH₂c-Pr |
| 7-20 | Et | 0 | CH₂CH₂OMe |
| 7-21 | Et | 1 | CH₂CH₂OMe |
| 7-22 | Et | 2 | CH₂CH₂OMe |
| 7-23 | c-Pr | 0 | Me |
| 7-24 | c-Pr | 1 | Me |
| 7-25 | c-Pr | 2 | Me |
| 7-26 | c-Pr | 0 | Et |
| 7-27 | c-Pr | 1 | Et |
| 7-28 | c-Pr | 2 | Et |
| 7-29 | c-Pr | 0 | CH₂c-Pr |
| 7-30 | c-Pr | 1 | CH₂c-Pr |
| 7-31 | c-Pr | 2 | CH₂c-Pr |
| 7-32 | c-Pr | 0 | CH₂CH₂OMe |
| 7-33 | c-Pr | 1 | CH₂CH₂OMe |
| 7-34 | c-Pr | 2 | CH₂CH₂OMe |
| 7-35 | F | 0 | Me |
| 7-36 | F | 1 | Me |
| 7-37 | F | 2 | Me |
| 7-38 | F | 0 | Et |
| 7-39 | F | 1 | Et |
| 7-40 | F | 2 | Et |
| 7-41 | F | 0 | CH₂c-Pr |
| 7-42 | F | 1 | CH₂c-Pr |
| 7-43 | F | 2 | CH₂c-Pr |
| 7-44 | F | 0 | CH₂CH₂OMe |
| 7-45 | F | 1 | CH₂CH₂OMe |
| 7-46 | F | 2 | CH₂CH₂OMe |
| 7-47 | Cl | 0 | Me |
| 7-48 | Cl | 1 | Me |
| 7-49 | Cl | 2 | Me |
| 7-50 | Cl | 0 | Et |
| 7-51 | Cl | 1 | Et |
| 7-52 | Cl | 2 | Et |
| 7-53 | Cl | 0 | CH₂c-Pr |
| 7-54 | Cl | 1 | CH₂c-Pr |
| 7-55 | Cl | 2 | CH₂c-Pr |
| 7-56 | Cl | 0 | CH₂CH₂OMe |
| 7-57 | Cl | 1 | CH₂CH₂OMe |
| 7-58 | Cl | 2 | CH₂CH₂OMe |
| 7-59 | Br | 0 | Me |
| 7-60 | Br | 1 | Me |
| 7-61 | Br | 2 | Me |
| 7-62 | Br | 0 | Et |
| 7-63 | Br | 1 | Et |
| 7-64 | Br | 2 | Et |
| 7-65 | Br | 0 | CH₂c-Pr |
| 7-66 | Br | 1 | CH₂c-Pr |
| 7-67 | Br | 2 | CH₂c-Pr |
| 7-68 | Br | 0 | CH₂CH₂OMe |
| 7-69 | Br | 1 | CH₂CH₂OMe |
| 7-70 | Br | 2 | CH₂CH₂OMe |
| 7-71 | I | 0 | Me |
| 7-72 | I | 1 | Me |
| 7-73 | I | 2 | Me |
| 7-74 | I | 0 | Et |
| 7-75 | I | 1 | Et |
| 7-76 | I | 2 | Et |
| 7-77 | I | 0 | CH₂c-Pr |
| 7-78 | I | 1 | CH₂c-Pr |
| 7-79 | I | 2 | CH₂c-Pr |
| 7-80 | I | 0 | CH₂CH₂OMe |
| 7-81 | I | 1 | CH₂CH₂OMe |
| 7-82 | I | 2 | CH₂CH₂OMe |

**Tabelle 8: Erfindungsgemäße Verbindungen der allgemeinen Formel (I) in Form der Natriumsalze, worin Q für Q1 und R^{x} für eine Methylgruppe stehen**

| 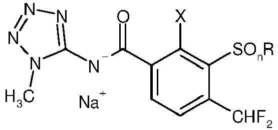 | | | |
|---|---|---|---|
| **Nr.** | **X** | **n** | **R** |
| 8-1 | Me | 0 | Me |
| 8-2 | Me | 1 | Me |
| 8-3 | Me | 2 | Me |
| 8-4 | Me | 0 | Et |
| 8-5 | Me | 1 | Et |
| 8-6 | Me | 2 | Et |
| 8-7 | Me | 0 | CH₂c-Pr |
| 8-8 | Me | 1 | CH₂c-Pr |
| 8-9 | Me | 2 | CH₂c-Pr |
| 8-10 | Me | 0 | CH₂CH₂OMe |
| 8-11 | Me | 1 | CH₂CH₂OMe |
| 8-12 | Me | 2 | CH₂CH₂OMe |
| 8-13 | Et | 0 | Me |
| 8-14 | Et | 1 | Me |
| 8-15 | Et | 2 | Me |
| 8-16 | Et | 0 | Et |
| 8-17 | Et | 1 | Et |
| 8-18 | Et | 2 | Et |
| 8-19 | Et | 0 | CH₂c-Pr |
| 8-20 | Et | 1 | CH₂c-Pr |
| 8-21 | Et | 2 | CH₂c-Pr |
| 8-22 | Et | 0 | CH₂CH₂OMe |
| 8-23 | Et | 1 | CH₂CH₂OMe |
| 8-24 | Et | 2 | CH₂CH₂OMe |
| 8-25 | c-Pr | 0 | Me |
| 8-26 | c-Pr | 1 | Me |
| 8-27 | c-Pr | 2 | Me |
| 8-28 | c-Pr | 0 | Et |
| 8-29 | c-Pr | 1 | Et |
| 8-30 | c-Pr | 2 | Et |
| 8-31 | c-Pr | 0 | CH₂c-Pr |
| 8-32 | c-Pr | 1 | CH₂c-Pr |
| 8-33 | c-Pr | 2 | CH₂c-Pr |
| 8-34 | c-Pr | 0 | CH₂CH₂OMe |
| 8-35 | c-Pr | 1 | CH₂CH₂OMe |
| 8-36 | c-Pr | 2 | CH₂CH₂OMe |
| 8-37 | F | 0 | Me |
| 8-38 | F | 1 | Me |
| 8-39 | F | 2 | Me |
| 8-40 | F | 0 | Et |
| 8-41 | F | 1 | Et |
| 8-42 | F | 2 | Et |
| 8-43 | F | 0 | CH₂c-Pr |
| 8-44 | F | 1 | CH₂c-Pr |
| 8-45 | F | 2 | CH₂c-Pr |
| 8-46 | F | 0 | CH₂CH₂OMe |
| 8-47 | F | 1 | CH₂CH₂OMe |
| 8-48 | F | 2 | CH₂CH₂OMe |
| 8-49 | Cl | 0 | Me |
| 8-50 | Cl | 1 | Me |
| 8-51 | Cl | 2 | Me |
| 8-52 | Cl | 0 | Et |
| 8-53 | Cl | 1 | Et |
| 8-54 | Cl | 2 | Et |
| 8-55 | Cl | 0 | CH₂c-Pr |
| 8-56 | Cl | 1 | CH₂c-Pr |
| 8-57 | Cl | 2 | CH₂c-Pr |
| 8-58 | Cl | 0 | CH₂CH₂OMe |
| 8-59 | Cl | 1 | CH₂CH₂OMe |
| 8-60 | Cl | 2 | CH₂CH₂OMe |
| 8-61 | Br | 0 | Me |
| 8-62 | Br | 1 | Me |
| 8-63 | Br | 2 | Me |
| 8-64 | Br | 0 | Et |
| 8-65 | Br | 1 | Et |
| 8-66 | Br | 2 | Et |
| 8-67 | Br | 0 | CH₂c-Pr |
| 8-68 | Br | 1 | CH₂c-Pr |
| 8-69 | Br | 2 | CH₂c-Pr |
| 8-70 | Br | 0 | CH₂CH₂OMe |
| 8-71 | Br | 1 | CH₂CH₂OMe |
| 8-72 | Br | 2 | CH₂CH₂OMe |
| 8-73 | I | 0 | Me |
| 8-74 | I | 1 | Me |
| 8-75 | I | 2 | Me |
| 8-76 | I | 0 | Et |
| 8-77 | I | 1 | Et |
| 8-78 | I | 2 | Et |
| 8-79 | I | 0 | CH₂c-Pr |
| 8-80 | I | 1 | CH₂c-Pr |
| 8-81 | I | 2 | CH₂c-Pr |
| 8-82 | I | 0 | CH₂CH₂OMe |
| 8-83 | I | 1 | CH₂CH₂OMe |
| 8-84 | I | 2 | CH₂CH₂OMe |

Zu zahlreichen in obigen Tabellen genannten erfindungsgemäßen Verbindungen der Formel (I) werden nachfolgend NMR-Daten im sogenannten NMR-Peak-Listenverfahren offenbart. Dabei werden die ¹H-NMR-Daten ausgewählter Beispiele in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet. Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden. Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der erfindungsgemäßen Verbindungen und/oder Peaks von Verunreinigungen haben gewöhnlich im Durchschnitt eine geringere Intensität als die Peaks der erfindungsgemäßen Verbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der erfindungsgemäßen Verbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

| |
|---|
| Beispiel 1-1: ¹H-NMR(400.0 MHz, CDC13): |
| δ= 7.79(0.78);7.77(1.41);7.723(1.33);7.703(0.72);7.518(0.54);7.506(1.01);7.368(2.17);7.26(92.56);7.229(1.07);6.996(0.51);4.12 7(14.78);2.797(9.61);2.308(16);2.252(0.56);1.553(1.29);0.008(1.14);0(32.43);-0.008(0.97) |
| Beispiel 1-2: ¹H-NMR(400.0 MHz, d₆-DMSO): |
| δ= 11.755(0.94);7.995(0.74);7.923(0.57);7.902(0.93);7.842(1.16);7.821(0.68);4.007(16);3.311(53.71);3.002(12.55);2.674(0.69); 2.669(0.92);2.665(0.7);2.59(7.06);2.523(2.83);2.518(4.26);2.51(55.71);2.505(120.68);2.5(167.23);2.496(116.4);2.491(51.7);2.45 (0.53);2.332(0.71);2.327(0.96);2.323(0.69);0.008(0.95);0(31.89);-0.008(0.89) |
| Beispiel 1-3: ¹H-NMR(400.0 MHz, d₆-DMSO): |
| δ= 11.829(0.84);8.047(0.55);7.937(0.88);7.919(0.94);7.783(1.21);7.646(0.6);4.021(16);3.446(10.93);3.309(31.28);2.75(7.59);2.6 69(0.55);2.523(1.58);2.518(2.4);2.509(30.08);2.505(65.21);2.5(91.14);2.496(63.77);2.491(28.46);2.327(0.53);0.008(0.54);0(17.3 6) |
| Beispiel 1-4: ¹H-NMR(400.0 MHz, CDC13): |
| δ= 7.778(0.71);7.761(1.15);7.715(1.06);7.523(1.62);7.519(1.13);7.385(3.47);7.27(0.65);7.27(0.73);7.269(0.81);7.268(0.84);7.26 7(1.06);7.266(1.35);7.26(155.87);7.247(2);6.996(0.85);4.128(1.1);4.117(16);2.784(2.73);2.773(8.76);2.766(7.1);2.747(5.05);2.72 8(1.75);2.042(1.82);1.57(2.2);1.284(0.63);1.275(1.24);1.265(1.7);1.257(2.02);1.239(1.19);1.234(7.07);1.216(14.14);1.197(6.63); 0.899(0.91);0.882(3.32);0.864(1.23);0.008(1.65);0.006(0.61);0.006(0.65);0.005(0.77);0(55.26);-0.006(0.8);-0.007(0.64);-0.008(1.72) |
| Beispiel 1-5: ¹H-NMR(400.0 MHz, d₆-DMSO): |
| δ= 11.757(1);7.932(0.55);7.912(0.92);7.856(1.09);7.836(0.68);5.753(0.57);4.008(16);3.31(44.61);3.227(0.59);3.208(0.58);3.126( 0.66);3.107(0.69);2.568(5.11);2.523(1.11);2.518(1.62);2.509(21.94);2.505(46.65);2.5(64.18);2.496(44.74);2.491(20.18);1.3(2.55 );1.282(5.31);1.263(2.39);0(4.67) |
| Beispiel 1-6: ¹H-NMR(400.0 MHz, d₆-DMSO): |
| δ= 11.826(0.98);8.065(0.55);7.964(0.9);7.944(0.68);7.914(0.63);7.778(1.36);7.641(0.65);4.022(16);3.542(0.71);3.524(2.42);3.50 6(2.48);3.488(0.75);3.309(87.5);2.742(8.48);2.669(0.56);2.523(1.92);2.509(34.37);2.505(72.77);2.5(99.75);2.496(69.83);2.491(3 1.46);2.327(0.57);1.267(2.62);1.248(5.94);1.23(2.62);0(3.28) |
| Beispiel 1-13: ¹H-NMR(400.0 MHz, CDC13): |
| δ= 7.763(0.8);7.743(1.83);7.713(2.2);7.693(0.97);7.524(0.88);7.386(1.93);7.267(13.77);7.248(0.97);4.104(15.81);3.235(0.68);3. 216(2.31);3.198(2.35);3.179(0.72);2.331(16);1.27(2.84);1.252(6.76);1.233(2.81);0(5.47) |
| Beispiel 1-14: ¹H-NMR(400,0 MHz, d₆-DMSO): |
| δ= 11,796(1,2);8,121(0,6);7,962(0,5);7,942(0,9);7,892(1,2);7,871(0,7);4,010(16,0);3,310(34,6);3,044(11,9);3,032(0,5);2,523(1,2) ;2,518(1,8);2,509(22,8);2,505(49,0);2,500(68,3);2,496(47,6);2,491(21,1);1,242(1,8);1,223(4,3);1,205(1,7);0,008(0,7);0,000(23,5) ;-0,009(0,7) |
| Beispiel 1-15: ¹H-NMR(400,0 MHz, d₆-DMSO): |
| δ= 11,890(0,7);7,958(0,8);7,938(0,7);7,777(1,2);7,641(0,6);4,026(16,0);3,440(10,7);3,310(22,0);3,226(1,1);3,208(1,1);2,518(0,8) ;2,510(12,2);2,505(27,2);2,500(38,4);2,496(26,7);2,491(11,7);1,281(1,5);1,263(4,0);1,244(1,6);0,000(17,4) |
| Beispiel 1-16: ¹H-NMR(400.0 MHz, CDC13): |
| δ= 7.792(0.77);7.772(1.59);7.735(2.01);7.715(0.98);7.537(0.8);7.399(1.81);7.261(16.53);5.298(1.09);4.119(16);3.253(0.59);3.23 4(2.01);3.216(2.06);3.197(0.64);2.797(1.14);2.778(3.65);2.76(3.69);2.741(1.19);1.258(4.01);1.239(8.35);1.228(2.65);1.221(4.02) ;1.21(6.13);1.191(2.52);0(6.53) |
| Beispiel 1-17: ¹H-NMR(400.0 MHz, d₆-DMSO): |
| δ= 11.805(1.75);7.967(0.92);7.947(1.25);7.895(1.45);7.875(0.9);4.01(16);3.31(32.77);3.286(0.72);3.268(0.74);3.253(0.9);3.235( 0.82);3.112(0.68);3.093(0.81);3.078(0.61);3.06(0.59);2.889(0.52);2.669(0.51);2.523(0.75);2.518(1.23);2.509(21.71);2.505(54.6); 2.5(86.23);2.496(78.63);2.491(50.39);2.45(0.77);2.327(0.57);1.333(2.67);1.314(5.76);1.295(2.73);1.236(2.18);1.218(5.11);1.199 (2.25);0.008(0.55);0(20.1) |
| Beispiel 1-18: ¹H-NMR(400.0 MHz, d₆-DMSO): |
| δ= 11.893(0.71);7.984(0.74);7.963(0.58);7.907(0.53);7.771(1.23);7.634(0.63);4.026(16);3.512(0.62);3.494(2.16);3.475(2.18);3.4 57(0.67);3.31(42.94);3.208(1);3.19(0.97);2.523(0.75);2.518(1.2);2.51(21.1);2.505(47.16);2.5(66.67);2.496(46.4);2.491(20.66);2. 45(0.51);1.276(2.41);1.267(1.83);1.257(5.73);1.249(4.46);1.239(2.59);1.231(1.78);0.008(0.64);0(25.34);-0.009(0.75) |
| Beispiel 1-25: ¹H-NMR(400.0 MHz, d₆-DMSO): |
| δ= 11.6077 (1.3); 7.6627 (2.3); 7.6435 (0.5); 7.5981 (0.9); 7.4606 (2.0); 7.3232 (0.9); 4.0382 (16.0); 3.3110 (39.5); 2.5224 (1.2); 2.5048 (39.0); 2.5004 (50.9); 2.4959 (37.0); 2.4915 (18.2); 2.4274 (8.2); 1.1329 (0.5); 1.1211 (1.7); 1.1177 (1.7); 1.0998 (1.7); 1.0850 (0.6); 0.6649 (0.6); 0.6508 (2.0); 0.6390 (2.0); 0.6247 (0.5); -0.0002 (8.8) |
| Beispiel 1-26: ¹H-NMR(400.0 MHz, d₆-DMSO): |
| δ= 8.4191 (0.9); 8.2814 (2.0); 8.1437 (1.0); 7.9077 (1.1); 7.8874 (1.8); 7.8205 (2.0); 7.8004 (1.3); 4.0383 (1.3); 4.0204 (1.6); 4.0088 (14.3); 3.3113 (10.0); 3.0436 (16.0); 2.5229 (1.6); 2.5182 (2.2); 2.5095 (22.6); 2.5050 (46.2); 2.5005 (62.8); 2.4959 (44.0); 2.4914 (20.3); 2.0763 (0.7); 1.9878 (5.4); 1.2587 (0.6); 1.2366 (2.5); 1.1923 (1.5); 1.1745 (3.0); 1.1567 (1.7); 1.1286 (0.8); 1.1188 (0.7); 1.0038 (0.6); 0.9920 (0.7); 0.8539 (0.5); 0.7331 (0.6); 0.7162 (1.5); 0.7078 (1.3); 0.7015 (1.4); 0.6834 (0.6); - 0.0002 (12.8) |
| Beispiel 1-27: ¹H-NMR(400.0 MHz, d₆-DMSO): |
| δ= 11.7346 (0.8); 7.9246 (0.7); 7.8507 (0.5); 7.7145 (1.3); 7.5784 (0.6); 4.0346 (16.0); 3.5507 (11.0); 3.3125 (58.4); 2.5229 (1.1); 2.5095 (18.7); 2.5050 (39.5); 2.5004 (54.9); 2.4958 (38.2); 2.4913 (17.4); 1.9877 (1.1); 1.9079 (0.9); 1.1745 (0.6); 1.0897 (1.0); 1.0679 (1.0); 0.7579 (1.0); 0.7454 (0.9); -0.0002 (2.7) |
| Beispiel 1-28: ¹H-NMR(400.0 MHz, d₆-DMSO): |
| δ= 11.6295 (0.9); 7.6749 (2.0); 7.6085 (0.8); 7.4711 (1.7); 7.3336 (0.8); 4.0397 (16.0); 3.3095 (63.0); 2.9118 (1.4); 2.8935 (1.4); 2.8749 (0.5); 2.5229 (0.8); 2.5181 (1.2); 2.5094 (19.5); 2.5049 (42.9); 2.5003 (60.3); 2.4957 (43.1); 2.4912 (20.1); 1.1708 (1.7); 1.1525 (3.4); 1.1340 (1.7); 1.1148 (1.4); 1.1109 (1.5); 1.0992 (0.8); 1.0934 (1.4); 1.0896 (1.4); 0.6400 (1.7); 0.6288 (1.6); - 0.0002 (13.9) |
| Beispiel 1-29: ¹H-NMR(400.0 MHz, d₆-DMSO): |
| δ= 11.6711 (0.7); 8.4143 (0.8); 8.2767 (1.6); 8.1391 (0.9); 7.9189 (1.1); 7.8985 (1.7); 7.8399 (1.4); 7.8198 (0.9); 4.0561 (0.5); 4.0382 (2.1); 4.0306 (16.0); 4.0206 (1.8); 4.0028 (0.5); 3.3099 (19.0); 3.1895 (1.3); 3.1721 (1.8); 3.1543 (1.5); 3.1366 (0.5); 2.5226 (1.5); 2.5092 (20.9); 2.5049 (40.9); 2.5004 (54.3); 2.4960 (39.0); 2.4917 (19.0); 1.9878 (5.8); 1.3407 (2.8); 1.3222 (5.9); 1.3036 (2.7); 1.1923 (1.8); 1.1746 (3.4); 1.1655 (1.0); 1.1568 (2.0); 1.0269 (0.6); 1.0145 (0.8); 0.7196 (1.9); 0.7058 (2.0); 0.6916 (0.5); -0.0002 (10.8) |
| Beispiel 1-30: ¹H-NMR(400.0 MHz, d₆-DMSO): |
| δ= 11.7409 (1.0); 7.9943 (0.8); 7.9747 (1.5); 7.9399 (2.1); 7.9199 (1.0); 7.8511 (0.9); 7.7150 (2.0); 7.5793 (1.0); 4.0265 (16.0); 3.6682 (1.0); 3.6498 (3.1); 3.6313 (3.2); 3.6128 (1.0); 3.3090 (28.7); 2.6694 (0.5); 2.6176 (0.7); 2.5046 (55.9); 2.5003 (70.6); 2.4960 (51.8); 1.9877 (1.9); 1.3642 (3.3); 1.3458 (7.0); 1.3273 (3.2); 1.1923 (0.5); 1.1745 (1.0); 1.1567 (0.5); 1.0801 (2.0); 1.0586 (1.9); 0.7547 (2.3); 0.7425 (2.2); -0.0002 (13.0) |
| Beispiel 1-37: ¹H-NMR(400.0 MHz, d₆-DMSO): |
| δ= 11.7857 (1.1); 7.9334 (0.6); 7.9140 (0.8); 7.8969 (0.6); 7.6578 (1.1); 7.6371 (1.0); 7.5220 (0.6); 7.3856 (1.4); 7.2492 (0.6); 3.9824 (16.0); 3.3115 (48.1); 2.5230 (0.8); 2.5184 (1.1); 2.5097 (17.9); 2.5051 (39.4); 2.5005 (55.5); 2.4959 (39.4); 2.4914 (17.8); 2.4735 (7.9); -0.0002 (14.1) |
| Beispiel 1-38: ¹H-NMR(400.0 MHz, d₆-DMSO): |
| δ= 8.1061 (0.8); 8.0870 (1.3); 8.0692 (1.0); 7.8970 (1.0); 7.7721 (1.8); 7.7607 (2.1); 7.7520 (1.7); 7.6243 (1.1); 3.9297 (16.0); 3.3095 (12.4); 3.0901 (13.2); 2.6694 (0.5); 2.5228 (1.8); 2.5181 (2.5); 2.5094 (28.2); 2.5049 (58.4); 2.5004 (80.0); 2.4958 (56.2); 2.4913 (26.2); 0.0079 (0.8); -0.0002 (22.5); -0.0085 (0.8) |
| Beispiel 1-39: ¹H-NMR(400.0 MHz, d₆-DMSO): |
| δ= 8.2804 (0.9); 8.2607 (1.3); 8.2433 (1.0); 7.9003 (1.7); 7.8796 (1.6); 7.8690 (1.0); 7.7327 (2.1); 7.5967 (1.0); 3.9669 (16.0); 3.5139 (10.5); 3.3104 (80.6); 3.1748 (1.6); 3.1619 (1.6); 2.6740 (0.7); 2.6694 (0.9); 2.6648 (0.7); 2.5228 (3.8); 2.5181 (5.2); 2.5094 (52.7); 2.5049 (109.3); 2.5003 (150.0); 2.4957 (102.8); 2.4911 (46.4); 2.3317 (0.7); 2.3271 (0.9); 2.3224 (0.6); 1.9876 (0.6); 1.9077 (5.9); 1.2361 (0.8); 0.0081 (0.6); -0.0002 (20.8); -0.0085 (0.6) |
| Beispiel 1-40: ¹H-NMR(400.0 MHz, d₆-DMSO): |
| δ= 7.9143 (2.3); 7.6397 (2.7); 7.6175 (2.2); 7.3770 (2.8); 3.9227 (16.0); 3.3088 (54.4); 2.9380 (4.1); 2.9203 (3.9); 2.9018 (1.9); 2.6694 (1.7); 2.5002 (303.4); 2.4959 (221.2); 2.3263 (1.7); 1.1770 (4.2); 1.1590 (8.6); 1.1410 (4.3); -0.0002 (77.2) |
| Beispiel 1-41: ¹H-NMR(400.0 MHz, d₆-DMSO): |
| δ= 11.8887 (1.1); 8.0993 (1.5); 7.9430 (1.0); 7.8078 (2.7); 7.7950 (1.7); 7.6704 (1.4); 4.0555 (1.1); 4.0381 (3.4); 4.0203 (3.6); 4.0027 (1.1); 3.9461 (9.2); 3.4292 (1.2); 3.4110 (1.4); 3.3611 (2.9); 3.3468 (3.1); 3.3426 (5.5); 3.3096 (2040.4); 3.2729 (2.1); 3.2595 (9.3); 2.6785 (3.9); 2.6740 (8.5); 2.6693 (11.7); 2.6647 (8.4); 2.6600 (3.9); 2.6195 (1.2); 2.5612 (4.2); 2.5471 (2.8); 2.5228 (44.4); 2.5181 (62.7); 2.5094 (668.3); 2.5048 (1388.2); 2.5002 (1912.9); 2.4957 (1320.7); 2.4911 (600.8); 2.4649 (3.8); 2.4600 (5.1); 2.4553 (6.7); 2.4501 (7.0); 2.4056 (1.2); 2.3361 (4.1); 2.3316 (8.3); 2.3270 (11.7); 2.3224 (8.5); 2.0720 (2.4); 1.9876 (16.0); 1.9077 (13.6); 1.2530 (3.9); 1.2345 (8.9); 1.2165 (3.8); 1.1923 (4.6); 1.1745 (9.3); 1.1567 (4.6); 0.1458 (1.8); 0.0080 (17.5); -0.0002 (568.1); -0.0085 (16.8); -0.0505 (1.1); -0.1497 (1.7) |
| Beispiel 1-42: ¹H-NMR(400.0 MHz, d₆-DMSO): |
| δ= 8.2076 (1.1); 7.8342 (2.7); 7.8051 (2.4); 7.7863 (1.8); 7.6979 (5.2); 7.5615 (2.4); 4.0559 (1.0); 4.0382 (2.8); 4.0203 (2.8); 4.0026 (0.9); 3.7961 (8.4); 3.5613 (2.0); 3.5428 (5.2); 3.5243 (5.3); 3.5076 (2.1); 3.4229 (0.7); 3.3778 (1.0); 3.3614 (1.5); 3.3443 (2.7); 3.3099 (734.5); 3.2789 (1.7); 3.2583 (1.0); 2.6740 (4.1); 2.6693 (5.6); 2.6646 (4.1); 2.6601 (2.1); 2.6302 (0.7); 2.5693 (1.2); 2.5227 (24.1); 2.5181 (34.2); 2.5094 (325.9); 2.5048 (662.1); 2.5003 (900.8); 2.4957 (622.0); 2.4912 (282.5); 2.4410 (1.2); 2.3316 (3.7); 2.3270 (5.3); 2.3224 (3.7); 2.1781 (0.7); 2.0719 (0.8); 1.9876 (12.3); 1.9073 (7.0); 1.2362 (13.8); 1.2240 (16.0); 1.2056 (7.5); 1.1923 (4.2); 1.1745 (7.6); 1.1567 (3.7); 1.1133 (0.7); 0.8539 (1.6); 0.8365 (0.6); 0.1459 (0.7); 0.0080 (6.8); - 0.0002 (209.6); -0.0085 (6.7); -0.1497 (0.6) |
| Beispiel 1-49: ¹H-NMR(400.0 MHz, d₆-DMSO): |
| δ= 7.904(0.66);7.885(0.88);7.795(1.36);7.775(0.93);7.611(0.58);7.474(1.35);7.338(0.69);4.014(16);3.309(38.07);2.669(0.64);2.5 23(1.94);2.518(2.95);2.51(36.77);2.505(78.98);2.5(109.62);2.496(76.69);2.491(34.21);2.426(8.5);2.327(0.66);0(15.6) |
| Beispiel 1-50: ¹H-NMR(400.0 MHz, d₆-DMSO): |
| δ= 11.958(0.9);8.2(0.6);8.063(1.32);8.045(0.8);8.01(1.29);7.926(0.68);4.01(16);3.305(22.19);3.097(8.07);2.669(0.94);2.522(2.77 );2.518(4.06);2.509(59.83);2.505(130.08);2.5(181.46);2.495(127.32);2.491(58.04);2.327(1.11);0.008(3.08);0(89.77);-0.008(2.67) |
| Beispiel 1-51: ¹H-NMR(400.0 MHz, d₆-DMSO): |
| δ= 12.036(0.97);8.194(0.61);8.074(1.13);8.053(0.84);7.94(0.6);7.803(1.34);7.666(0.7);4.028(16);3.571(8.8);3.301(23.52);2.674( 0.75);2.669(1.08);2.664(0.74);2.55(0.55);2.522(3.09);2.518(4.34);2.509(58.23);2.504(126.06);2.5(175.99);2.495(123.31);2.49(5 5.64);2.456(0.51);2.331(0.76);2.326(1.05);2.322(0.76);0.008(0.69);0(26.33);-0.008(0.84) |
| Beispiel 1-52: ¹H-NMR(400.0 MHz, CDC13): |
| δ= 7.775(3.97);7.772(3.32);7.47 (0.93);7.332(2.04);7.26(29.31);7.195(0.99);4.144(16);2.963(0.81);2.944(2.5);2.926(2.59);2.907 ( 0.99);1.257(3.42);1.239(6.98);1.22(3.25);1.216(0.8);0(12.32) |
| Beispiel 1-53: ¹H-NMR(400.0 MHz, d₆-DMSO): |
| δ= 11.966(0.87);8.098(0.94);8.028(0.88);4.038(0.72);4.02(0.93);4.012(16);3.314(11.93);3.29(1.28);3.271(1.27);2.523(1.31);2.51 8(1.82);2.51(25.42);2.505(55.43);2.5(77.74);2.496(54.39);2.491(24.2);2.455(0.57);2.45(0.7);2.446(0.51);1.988(3.4);1.332(1.47); 1.314(3.02);1.295(1.43);1.192(0.96);1.174(1.9);1.157(0.94);0.008(1.45);0(49.32);-0.008(1.5) |
| Beispiel 1-54: ¹H-NMR(400.0 MHz, d₆-DMSO): |
| δ= 12.042(0.98);8.104(0.76);8.083(0.6);7.941(0.58);7.805(1.33);7.668(0.67);4.031(16);3.695(1.14);3.676(1.17);3.313(4.96);2.51 8(0.64);2.51(8.92);2.505(19.32);2.5(26.8);2.496(18.67);2.491(8.35);1.269(1.53);1.251(3.17);1.232(1.53);0(8.17) |
| Beispiel 1-61: ¹H-NMR(400.0 MHz, d₆-DMSO): |
| δ= 11.891(0.6);7.845(0.74);7.834(1.19);7.623(0.51);7.486(1.18);7.349(0.58);4.04(16);3.309(17.41);2.523(0.93);2.518(1.43);2.50 9(20.81);2.505(45.5);2.5(63.89);2.496(44.28);2.491(19.4);2.45(0.57);2.422(4.44);0.008(1.2);0(45.19);-0.003(1.91);-0.004(0.69);-0.005(0.51);-0.008(1.3) |
| Beispiel 1-62: ¹H-NMR(400.0 MHz, d₆-DMSO): |
| δ= 11.95(0.97);8.119(0.85);8.042(0.88);8.009(0.55);7.982(0.73);4.032(16);3.313(14.24);3.091(5.78);2.523(0.75);2.518(1.05);2.5 1(14.9);2.505(32.81);2.5(45.95);2.496(32.3);2.491(14.95);2.451(1.03);0.008(0.52);0(17.37);-0.008(0.58) |
| Beispiel 1-63: ¹H-NMR(400.0 MHz, d₆-DMSO): |
| δ= 12.024(1.45);8.156(0.57);8.136(1.19);8.109(1.47);8.088(0.65);7.945(0.64);7.809(1.41);7.672(0.71);4.052(16);4.033(0.52);3.5 78(7.24);3.315(24.01);2.523(1.12);2.518(1.71);2.509(22.57);2.505(48.42);2.5(66.89);2.496(46.84);2.491(21.11);2.45(0.51);1.90 8(0.63);0(14.66) |
| Beispiel 1-64: ¹H-NMR(400.0 MHz, d₆-DMSO): |
| δ= 11.895(0.69):7.847(1.98):7.623(0.63):7.487(1.38):7.35(0.67):4.039(16):3.31(24.78):2.947(1.27):2.929(1.29):2.523(1.32):2.51 8(1.97);2.51(25.95);2.505(55.92);2.5(77.48);2.496(53.77);2.491(23.63);2.45(0.51);1.18(1.52);1.162(3.05);1.143(1.55);0.008(1.6 5);0.005(0.58);0(57.33);-0.006(0.78);-0.007(0.67);-0.008(1.71) |
| Beispiel 1-65: ¹H-NMR(400.0 MHz, d₆-DMSO): |
| δ= 11.952(1.03);8.144(0.84);8.072(0.51);8.051(0.95);8.008(0.74);5.754(6.01);4.03(16);3.312(32.93);3.29(0.97);3.273(1.14);3.25 5(1.07);3.238(0.51);2.523(0.84);2.518(1.19);2.51(23.97);2.505(54.05);2.5(76.85);2.496(55.23);2.491(26.33);2.448(1.56);2.327(0 .52);1.358(1.55);1.34(3.27);1.321(1.6);0.008(0.63);0(29.26);-0.008(1.13) |
| Beispiel 1-66: ¹H-NMR(400.0 MHz, d₆-DMSO): |
| δ= 12.024(2);8.178(0.76);8.158(1.87);8.135(2.05);8.115(0.84);7.945(0.94);7.809(2.09);7.673(1.03);4.052(16);4.032(0.78);3.736( 0.8);3.718(2.2);3.699(2.26);3.681(0.89);3.408(8.24);2.523(1.56);2.518(2.34);2.51(28.84);2.505(61.43);2.501(84.52);2.496(58.95 );2.492(26.44);2.451(0.53);2.327(0.52);1.908(2.96);1.285(2.36);1.266(4.74);1.248(2.44);0(13.92) |
| Beispiel 1-73: ¹H-NMR(400.0 MHz, d₆-DMSO): |
| δ= 11.838(1.13);7.83(0.9);7.81(1.38);7.747(0.84);7.728(0.56);7.616(0.75);7.479(1.67);7.342(0.82);4.072(16);3.308(32.82);2.669 (0.51);2.523(1.56);2.518(2.23);2.509(28.29);2.505(60.63);2.5(84.02);2.496(59.23);2.491(26.7);2.389(7.95);0.008(1.28);0(41.59); -0.008(1.17) |
| Beispiel 1-74: ¹H-NMR(400.0 MHz, d₆-DMSO): |
| δ= 11.8934 (2.0); 8.3097 (0.8); 8.1727 (1.5); 8.0599 (1.1); 8.0377 (1.5); 7.8836 (0.9); 7.8618 (0.7); 4.0630 (16.0); 3.3137 (92.6); 3.0611 (10.2); 2.6739 (0.8); 2.6693 (1.1); 2.6649 (0.8); 2.5093 (67.9); 2.5048 (133.1); 2.5003 (178.2); 2.4958 (128.5); 2.4914 (61.6); 2.4668 (0.9); 2.3316 (0.8); 2.3271 (1.0); 2.3226 (0.8); 1.9078 (1.0); 0.0079 (0.7); -0.0002 (15.6) |
| Beispiel 1-75: ¹H-NMR(400.0 MHz, d₆-DMSO): |
| δ= 11.9573 (0.6); 8.0873 (0.6); 8.0668 (0.8); 8.0024 (0.8); 7.7909 (0.6); 4.6702 (16.0); 4.0858 (3.6); 3.7444 (0.6); 3.7272 (0.6); 3.5478 (3.4); 2.5073 (63.7); 1.1028 (1.1); 1.0860 (0.6); -0.0002 (7.0) |
| Beispiel 1-76: ¹H-NMR(400.0 MHz, d₆-DMSO): |
| δ= 11.845(1.1);7.845(1.01);7.824(1.49);7.752(0.93);7.732(0.73);7.621(0.84);7.484(1.79);7.347(0.91);4.072(16);3.309(22.23);2.9 34(0.71);2.916(1.87);2.898(1.91);2.879(0.72);2.523(1.04);2.51(23.62);2.505(50.6);2.5(69.98);2.496(49.39);2.492(22.64);1.201(2 .17);1.182(4.37);1.164(2.23);0.008(0.79);0(25.78);0(26.27);-0.008(0.89) |
| Beispiel 1-77: ¹H-NMR(400.6 MHz, d₆-DMSO): |
| δ= 11.907(1.26);8.316(0.51);8.179(1.11);8.066(1);8.045(1.52);7.889(0.71);7.87(0.59);4.062(16);3.322(71.9);3.271(0.71);3.253(0 .65);3.22(0.94);3.201(1);3.186(0.58);2.524(1.42);2.519(1.95);2.51(18.46);2.506(38.47);2.501(53.07);2.497(37.86);2.492(17.47); 1.908(0.81);1.392(2.57);1.374(5.24);1.355(2.46);0(2.75) |
| Beispiel 1-78: ¹H-NMR(400.6 MHz, d₆-DMSO): |
| δ= 11.9547 (3.0); 8.1145 (1.7); 8.0944 (2.4); 8.0249 (2.0); 8.0044 (1.4); 7.9278 (1.3); 7.7916 (2.8); 7.6554 (1.4); 4.0838 (16.0); 4.0628 (1.2); 3.7147 (1.3); 3.6968 (3.4); 3.6786 (3.4); 3.6603 (1.3); 3.3498 (1.4); 3.3246 (83.1); 2.5054 (50.1); 2.5012 (64.4); 2.4972 (49.3); 1.3078 (3.6); 1.2898 (7.0); 1.2718 (3.6); -0.0002 (1.0) |
| Beispiel 2-1: ¹H-NMR(400.0 MHz, CDC13): |
| δ= 7.782(0.64);7.762(1.18);7.717(1.18);7.697 (0.67);7.518(0.55);7.504(0.93);7.366(2.07);7.26(99.7);7.228(1.08);6.996(0.53);4.5 05(1.09);4.487(3.51);4.469(3.55);4.451(1.15);2.795(8.78);2.306(16);1.655(4);1.637(8.77);1.619(3.98);0.008(1.1);0(41.08);-0.008(1.35) |
| Beispiel 2-2: ¹H-NMR(400.0 MHz, d₆-DMSO): |
| δ= 11.653(1.38);7.996(0.99);7.913(0.74);7.893(1.22);7.858(0.61);7.843(1.5);7.823(0.84);4.381(1.22);4.362(3.94);4.344(4.01);4. 326(1.24);3.308(63.88);3.004(16);2.674(0.83);2.669(1.17);2.665(0.79);2.588(9.37);2.523(4.05);2.518(5.99);2.509(67.99);2.505( 144.81);2.5(198.98);2.496(137.97);2.491(61.37);2.332(0.8);2.327(1.2);2.322(0.85);1.495(4.44);1.476(10.14);1.458(4.38);0.008( 1.03);0(33.01);-0.008(1) |
| Beispiel 2-3: ¹H-NMR(400.0 MHz, d₆-DMSO): |
| δ= 11.725(2.33);8.062(0.79);8.04(0.99);7.94(1.78);7.92(2.29);7.784(2.61);7.648(1.23);4.396(1.93);4.378(6.18);4.36(6.2);4.342(1 .93);3.953(0.62);3.448(22.11);3.307(82.43);2.749(16);2.678(0.56);2.674(1.21);2.669(1.7);2.665(1.21);2.66(0.6);2.554(0.74);2.55 (1.08);2.545(1.09);2.54(1.19);2.523(5.51);2.518(7.94);2.509(97.61);2.505(210.76);2.5(294.34);2.496(205.42);2.491(91.2);2.449( 0.56);2.444(0.58);2.336(0.6);2.332(1.32);2.327(1.73);2.322(1.25);2.318(0.64);1.499(6.55);1.488(0.97);1.481(15.03);1.463(6.47); 0.008(1.61);0(55.05);-0.008(1.58) |
| Beispiel 2-4: ¹H-NMR(400.0 MHz, CDC13): |
| δ= 7.734(1.82);7.713(1.8);7.523(1.88);7.518(1.48);7.385(4.08);7.259(231.31);7.247(2.6);6.995(1.28);5.298(2.5);4.501(1.83);4.4 83(5.75);4.464(5.86);4.446(2);2.781(3.06);2.771(12.83);2.763(7.54);2.744(6.31);2.726(2.13);2.042(1.22);1.657(6.23);1.639(13.1 7);1.62(6.2);1.549(2.26);1.331(0.56);1.284(0.96);1.275(0.87);1.258(2.45);1.234(7.93);1.215(16);1.197(7.45);0.882(1.4);0.864(0. 65);0.008(3.22);0(94.1);-0.008(2.86) |
| Beispiel 2-5: ¹H-NMR(400.0 MHz, d₆-DMSO): |
| δ= 11.658(2.62);8.018(0.79);7.923(1.2);7.902(2.04);7.857(2.63);7.837(1.41);5.753(0.64);4.381(2.1);4.363(6.57);4.345(6.61);4.3 26(2.09);3.36(1.48);3.31(147.53);3.242(1.06);3.227(1.35);3.208(1.25);3.127(1.46);3.108(1.59);3.094(1.02);3.075(0.93);2.674(1. 24);2.669(1.68);2.665(1.25);2.566(12.77);2.55(3.76);2.523(9.33);2.518(11.63);2.509(91.56);2.505(188.98);2.5(258.72);2.496(18 2.09);2.491(83.39);2.439(0.56);2.336(0.58);2.332(1.13);2.327(1.54);2.322(1.05);1.494(7.27);1.476(16);1.458(7.09);1.301(5.62); 1.283(11.89);1.264(5.31);0(11.34) |
| Beispiel 2-6: ¹H-NMR(400.0 MHz, CDC13): |
| δ= 7.971(1);7.952(3.22);7.922(1.79);7.902(0.79);7.814(2.84);7.676(1.43);7.518(1.29);7.259(209.94);6.995(1.08);4.515(1.03);4.4 96(2.82);4.478(2.89);4.46(1.07);3.345(1.6);3.326(4.87);3.307(5);3.288(1.56);2.869(16);1.66(4.56);1.642(9.29);1.624(4.63);1.565 (0.98);1.446(5.4);1.428(11.03);1.409(5.05);1.33(1);1.284(1.24);1.255(1.35);0.008(2.95);0(83.48);-0.008(3.21) |
| Beispiel 2-13: ¹H-NMR(400.0 MHz, CDC13): |
| δ= 7.776(0.71);7.756(1.67);7.728(2.13);7.708(0.9);7.522(0.87);7.384(1.93);7.26(26.96);7.246(0.99);4.513(1.12);4.494(3.67);4.4 76(3.7);4.458(1.16);3.234(0.66);3.215(2.22);3.196(2.25);3.178(0.69);2.331(16);2.279(1.29);1.653(3.97);1.634(8.64);1.616(3.91); 1.259(2.77);1.24(6.6);1.222(2.76);0(11.06) |
| Beispiel 2-14: ¹H-NMR(400.0 MHz, d₆-DMSO): |
| δ= 11.692(1.42);8.122(0.85);7.95(0.61);7.93(1.17);7.892(1.62);7.872(0.8);4.382(1.13);4.364(3.67);4.345(3.75);4.327(1.17);3.36( 0.71);3.31(123.1);3.045(16);3.028(0.59);2.884(0.58);2.867(0.51);2.674(0.59);2.669(0.84);2.665(0.59);2.555(0.52);2.551(0.65);2. 523(2.39);2.518(3.56);2.51(49.94);2.505(108.7);2.5(152.09);2.496(105.57);2.491(46.48);2.45(0.52);2.332(0.71);2.327(0.99);2.3 23(0.68);1.496(4.41);1.478(10.45);1.46(4.34);1.243(2.28);1.224(5.68);1.206(2.3);0.008(1.4);0(53);-0.008(1.59) |
| Beispiel 2-15: ¹H-NMR(400.0 MHz, d₆-DMSO): |
| δ= 11.786(2.88);8.099(0.76);8.079(0.95);7.961(1.95);7.941(1.51);7.914(1.19);7.778(2.66);7.641(1.33);4.398(1.87);4.38(6.21);4. 362(6.32);4.344(1.99);3.442(23.78);3.31(71.85);3.26(0.53);3.243(0.81);3.224(2.56);3.206(2.61);3.188(0.82);2.674(0.55);2.669(0 .78);2.665(0.57);2.523(2.17);2.518(3.27);2.509(46.2);2.505(100.26);2.5(140.15);2.496(97.51);2.491(43.24);2.455(0.96);2.45(1.1 9);2.446(0.82);2.332(0.66);2.327(0.9);2.322(0.66);1.508(6.92);1.49(16);1.471(6.85);1.283 (3.68);1.265(9.36);1.246(3.68);0.008(1 .44);0(52.54);-0.009(1.58) |
| Beispiel 2-16: ¹H-NMR(400.0 MHz, CDC13): |
| δ= 7.697(13.47);7.536(1.83);7.519(0.89);7.398(4.27);7.26(151.86);7.21(1.21);6.996(0.85);5.299(2.93);4.495(1.8);4.476(5.74);4. 458(5.82);4.44(1.87);3.234(1.21);3.215(4.19);3.196(4.29);3.178(1.36);2.791(2.11);2.773(6.77);2.754(6.92);2.736(2.25);1.649(6. 3);1.63(13.48);1.612(6.48);1.562(1.18);1.258(7.71);1.239(16);1.23(4.76);1.221(7.78);1.211(10.55);1.192(4.55);0.008(1.64);0(62. 7);-0.008(2.04);-0.05(0.51) |
| Beispiel 2-17: ¹H-NMR(400.0 MHz, d₆-DMSO): |
| δ= 11.702(3.4);8.105(0.58);7.957(1.15);7.938(1.79);7.898(2.55);7.878(1.29);4.384(1.92);4.366(6.16);4.348(6.2);4.329(1.96);3.4 08(4.77);3.287(1.05);3.269(1.21);3.254(1.57);3.236(1.45);3.115(1.21);3.096(1.34);3.081(1.01);3.063(0.95);3.003(0.58);2.91(0.6) ;2.892(0.74);2.873(0.59);2.67(0.57);2.523(2.07);2.518(2.96);2.51(34.26);2.505(73.09);2.5(100.87);2.496(71.3);2.491(32.71);2.4 55(0.85);2.45(0.95);2.446(0.67);2.327(0.65);1.498(6.96);1.48(16);1.462(6.95);1.334(5.11);1.316(11.02);1.297(4.99);1.238(4.02); 1.22(9.38);1.201(3.94);1.097(0.51);0.008(1.4);0(46.28);-0.008(1.61) |
| Beispiel 2-25: ¹H-NMR(400.0 MHz, d₆-DMSO): |
| δ= 11.4970 (5.3); 7.6651 (5.7); 7.5989 (1.8); 7.4617 (4.1); 7.3241 (2.0); 4.4055 (2.5); 4.3875 (7.8); 4.3694 (7.9); 4.3512 (2.6); 3.3109 (92.1); 2.6700 (0.7); 2.5048 (90.3); 2.5004 (124.7); 2.4960 (95.3); 2.4277 (17.1); 2.3269 (0.8); 2.2239 (1.1); 1.5140 (7.4); 1.4960 (16.0); 1.4778 (7.4); 1.1276 (1.2); 1.1124 (3.9); 1.0906 (3.8); 0.6604 (4.2); 0.6483 (4.2); -0.0002 (20.4) |
| Beispiel 2-26: ¹H-NMR(400.0 MHz, d₆-DMSO): |
| δ= 11.5506 (0.6); 8.4204 (0.9); 8.2828 (2.0); 8.1452 (1.0); 7.9244 (1.2); 7.9043 (1.8); 7.8322 (1.3); 7.8122 (0.9); 4.3953 (1.4); 4.3771 (4.5); 4.3590 (4.6); 4.3408 (1.5); 3.3111 (15.5); 3.0501 (16.0); 2.5228 (1.4); 2.5180 (2.0); 2.5094 (19.3); 2.5049 (39.1); 2.5004 (52.9); 2.4959 (37.0); 2.4914 (17.0); 2.0912 (0.6); 1.5076 (5.0); 1.4895 (10.9); 1.4714 (4.8); 1.1359 (0.9); 1.1262 (0.7); 1.0041 (0.7); 0.9916 (0.8); 0.7379 (0.6); 0.7226 (1.7); 0.7149 (1.4); 0.7073 (1.6); 0.6910 (0.6); -0.0002 (9.7) |
| Beispiel 2-27: ¹H-NMR(400.0 MHz, d₆-DMSO): |
| δ= 11.6185 (0.9); 7.9585 (0.6); 7.9220 (1.3); 7.9027 (0.7); 7.8503 (0.8); 7.7148 (1.8); 7.5785 (0.9); 4.3976 (1.2); 4.3794 (4.0); 4.3612 (4.0); 4.3430 (1.3); 3.5516 (16.0); 3.3127 (55.9); 2.6691 (0.6); 2.5230 (1.4); 2.5182 (2.1); 2.5095 (24.1); 2.5050 (50.8); 2.5004 (70.5); 2.4959 (49.0); 2.4913 (22.3); 1.9877 (0.8); 1.5026 (4.5); 1.4845 (10.3); 1.4663 (4.4); 1.0834 (1.6); 1.0612 (1.5); 0.7677 (1.4); 0.7550 (1.4); -0.0002 (3.4) |
| Beispiel 2-28: ¹H-NMR(400.0 MHz, d₆-DMSO): |
| δ= 11.5133 (0.9); 7.6696 (7.3); 7.6086 (1.6); 7.4711 (3.7); 7.3337 (1.8); 4.4020 (2.1); 4.3838 (6.9); 4.3657 (7.0); 4.3475 (2.2); 4.1051 (0.8); 4.0870 (0.8); 3.3107 (89.4); 2.9283 (1.4); 2.9099 (4.2); 2.8915 (4.3); 2.8731 (1.5); 2.6696 (0.7); 2.6651 (0.5); 2.5502 (0.6); 2.5231 (1.9); 2.5184 (2.7); 2.5097 (38.7); 2.5051 (85.2); 2.5005 (119.6); 2.4959 (85.2); 2.4913 (39.3); 2.3319 (0.5); 2.3273 (0.7); 2.3227 (0.5); 2.1684 (0.6); 1.9878 (1.2); 1.5132 (7.1); 1.4950 (16.0); 1.4769 (7.1); 1.3132 (1.0); 1.2951 (2.0); 1.2770 (0.9); 1.2364 (1.2); 1.1745 (1.2); 1.1681 (5.3); 1.1497 (11.0); 1.1313 (5.2); 1.1173 (1.1); 1.1054 (2.8); 1.1016 (2.9); 1.0963 (1.6); 1.0900 (1.5); 1.0843 (2.8); 1.0803 (2.9); 1.0689 (1.0); 0.6665 (1.0); 0.6517 (3.6); 0.6406 (3.3); 0.6373 (2.9); 0.6256 (0.9); -0.0002 (8.6) |
| Beispiel 2-29: ¹H-NMR(400.0 MHz, d₆-DMSO): |
| δ= 11.5628 (0.8); 8.4128 (1.7); 8.2753 (3.1); 8.1370 (1.8); 7.9186 (2.3); 7.8975 (3.2); 7.8340 (2.4); 7.8130 (1.5); 4.3916 (2.3); 4.3734 (6.9); 4.3552 (6.6); 4.3371 (2.1); 4.1055 (0.8); 4.0382 (1.1); 4.0207 (1.0); 3.3124 (98.0); 3.2069 (1.2); 3.1884 (2.6); 3.1736 (3.0); 3.1554 (2.7); 2.6694 (1.0); 2.5095 (67.8); 2.5051 (133.9); 2.5006 (178.2); 2.4961 (126.0); 2.4917 (59.2); 2.3274 (1.1); 2.0454 (1.2); 1.9879 (4.6); 1.9077 (1.2); 1.5063 (7.3); 1.4883 (16.0); 1.4701 (7.4); 1.3408 (5.8); 1.3223 (12.3); 1.3038 (6.0); 1.2363 (2.7); 1.1924 (1.4); 1.1746 (3.0); 1.1569 (2.7); 0.9953 (1.7); 0.7284 (3.6); 0.7134 (3.5); -0.0002 (8.2) |
| Beispiel 2-30: ¹H-NMR(400.0 MHz, d₆-DMSO): |
| δ= 11.6232 (1.1); 7.9885 (0.7); 7.9676 (1.4); 7.9403 (2.8); 7.9201 (1.2); 7.8509 (1.3); 7.7150 (3.1); 7.5792 (1.5); 4.3930 (1.9); 4.3749 (6.1); 4.3567 (6.2); 4.3386 (1.9); 4.0382 (1.0); 4.0205 (1.0); 3.6699 (1.4); 3.6515 (4.9); 3.6330 (5.0); 3.6145 (1.5); 3.3090 (61.8); 2.6739 (0.6); 2.6693 (0.8); 2.6647 (0.6); 2.6166 (0.7); 2.5228 (3.1); 2.5181 (4.2); 2.5094 (43.8); 2.5048 (90.9); 2.5003 (124.8); 2.4957 (86.5); 2.4911 (39.4); 2.3316 (0.5); 2.3270 (0.7); 2.3224 (0.5); 1.9877 (4.5); 1.4994 (7.1); 1.4813 (16.0); 1.4631 (6.9); 1.3647 (5.2); 1.3463 (11.9); 1.3277 (5.1); 1.2477 (0.9); 1.1923 (1.3); 1.1745 (2.5); 1.1568 (1.2); 1.0902 (0.7); 1.0789 (2.4); 1.0755 (2.4); 1.0572 (2.3); 1.0538 (2.4); 1.0432 (0.8); 0.8584 (1.6); 0.8408 (0.5); 0.7623 (2.6); 0.7504 (2.6); 0.0080 (1.1); - 0.0002 (34.1); -0.0085 (1.0) |
| Beispiel 2-37: ¹H-NMR(400.0 MHz, d₆-DMSO): |
| δ= 11.6992 (0.6); 7.9256 (1.4); 7.9063 (2.0); 7.8890 (1.6); 7.6505 (2.7); 7.6303 (2.4); 7.5208 (1.3); 7.3840 (3.2); 7.2476 (1.4); 4.3607 (2.0); 4.3424 (6.3); 4.3242 (6.3); 4.3061 (2.1); 3.3121 (54.1); 2.6698 (0.6); 2.5231 (2.1); 2.5184 (3.1); 2.5098 (40.3); 2.5052 (87.1); 2.5006 (121.2); 2.4961 (85.1); 2.4915 (38.2); 2.4692 (18.3); 2.3273 (0.6); 1.4797 (7.2); 1.4615 (16.0); 1.4434 (7.1); 0.0081 (0.7); -0.0002 (24.4) |
| Beispiel 2-38: ¹H-NMR(400.0 MHz, d₆-DMSO): |
| δ= 8.1029 (1.1); 8.0835 (1.7); 8.0657 (1.1); 7.8972 (1.3); 7.7619 (3.8); 7.7439 (1.9); 7.6243 (1.4); 4.3200 (1.4); 4.3019 (4.2); 4.2837 (4.3); 4.2656 (1.5); 4.0383 (1.5); 4.0205 (1.5); 3.3104 (14.2); 3.0864 (16.0); 2.6696 (0.6); 2.5353 (0.6); 2.5229 (2.5); 2.5183 (3.4); 2.5096 (38.0); 2.5050 (79.6); 2.5004 (109.8); 2.4959 (77.0); 2.4913 (35.5); 2.3273 (0.6); 1.9877 (7.0); 1.9077 (4.3); 1.4529 (4.7); 1.4348 (10.1); 1.4166 (4.7); 1.2363 (1.3); 1.1923 (1.9); 1.1745 (3.9); 1.1567 (1.9); 0.0080 (0.9); -0.0002 (30.6); - 0.0085 (1.0) |
| Beispiel 2-39: ¹H-NMR(400.0 MHz, d₆-DMSO): |
| δ= 8.2399 (2.3); 8.2215 (3.8); 8.2023 (2.3); 7.8493 (2.4); 7.8165 (4.9); 7.7959 (4.4); 7.7127 (4.9); 7.5765 (2.4); 4.2684 (3.2); 4.2503 (9.0); 4.2322 (9.1); 4.2142 (3.5); 3.4864 (25.8); 3.3109 (31.8); 3.1687 (2.0); 2.6693 (1.3); 2.5044 (158.8); 2.5004 (191.6); 2.4964 (145.5); 2.3270 (2.5); 1.9075 (2.4); 1.4240 (8.0); 1.4060 (16.0); 1.3879 (8.9); 1.2988 (1.5); 1.2589 (1.9); 1.2365 (5.0); 0.8541 (0.8); -0.0002 (32.4) |
| Beispiel 2-40: ¹H-NMR(400.0 MHz, d₆-DMSO): |
| δ= 11.7204 (1.7); 7.9359 (2.4); 7.9183 (3.8); 7.9009 (2.4); 7.6571 (4.5); 7.6372 (3.7); 7.5194 (2.3); 7.3838 (4.3); 7.2484 (2.0); 4.3409 (2.6); 4.3242 (7.4); 4.3051 (7.4); 4.2869 (2.8); 3.3090 (49.0); 2.9597 (2.2); 2.9413 (6.4); 2.9230 (6.8); 2.9038 (2.8); 2.6687 (1.9); 2.5002 (293.9); 2.3271 (1.8); 1.4679 (7.5); 1.4498 (16.0); 1.4313 (8.1); 1.2334 (1.6); 1.1775 (7.4); 1.1591 (14.7); 1.1410 (8.1); -0.0002 (78.4) |
| Beispiel 2-41: ¹H-NMR(400.0 MHz, d₆-DMSO): |
| δ= 8.1068 (2.0); 8.0885 (3.3); 8.0698 (2.0); 7.9403 (2.4); 7.8377 (0.6); 7.8038 (5.2); 7.7669 (4.1); 7.7464 (3.5); 7.7057 (0.6); 7.6674 (2.6); 4.2904 (2.6); 4.2722 (7.4); 4.2540 (7.7); 4.2360 (2.8); 4.0383 (1.4); 4.0206 (1.4); 3.5519 (0.6); 3.5341 (0.6); 3.3104 (49.0); 3.2792 (3.9); 3.2512 (2.9); 3.2323 (2.8); 3.2173 (1.8); 3.1990 (1.3); 3.1815 (0.6); 2.6740 (1.2); 2.6695 (1.5); 2.5092 (104.5); 2.5050 (194.7); 2.5005 (250.3); 2.4961 (176.6); 2.4918 (84.2); 2.3314 (1.1); 2.3272 (1.4); 2.3227 (1.1); 1.9877 (6.1); 1.4341 (7.8); 1.4160 (16.0); 1.3978 (8.2); 1.2987 (0.9); 1.2586 (2.0); 1.2455 (8.4); 1.2362 (6.9); 1.2272 (15.5); 1.2087 (7.9); 1.1924 (2.6); 1.1746 (3.6); 1.1568 (1.8); 0.8540 (0.8); 0.0078 (2.7); -0.0002 (52.5); -0.0084 (2.4) |
| Beispiel 2-42: ¹H-NMR(400.0 MHz, d₆-DMSO): |
| δ= 8.2767 (1.8); 8.2576 (2.9); 8.2395 (1.9); 7.8731 (3.6); 7.8514 (4.7); 7.7140 (4.1); 7.5779 (2.0); 4.3036 (2.2); 4.2854 (6.8); 4.2673 (7.0); 4.2491 (2.6); 4.0383 (1.1); 4.0205 (1.1); 3.5835 (1.8); 3.5649 (5.2); 3.5465 (5.4); 3.5283 (2.1); 3.3105 (19.4); 2.6741 (0.8); 2.6695 (1.2); 2.6649 (0.9); 2.5391 (0.6); 2.5230 (4.3); 2.5183 (6.0); 2.5096 (65.2); 2.5051 (136.3); 2.5005 (187.8); 2.4959 (130.6); 2.4913 (60.0); 2.4746 (0.6); 2.4695 (0.6); 2.4647 (0.6); 2.3319 (0.8); 2.3273 (1.1); 2.3226 (0.8); 1.9877 (5.3); 1.9077 (5.4); 1.4445 (6.8); 1.4264 (14.1); 1.4083 (7.1); 1.3757 (0.8); 1.3568 (0.6); 1.2986 (0.8); 1.2523 (7.0); 1.2342 (16.0); 1.2155 (7.0); 1.1923 (1.9); 1.1791 (0.6); 1.1745 (3.3); 1.1567 (1.6); 0.8539 (0.8); 0.0079 (2.0); 0.0063 (0.9); 0.0054 (0.9); - 0.0002 (60.8); -0.0068 (0.8); -0.0085 (1.9) |
| Beispiel 2-49: ¹H-NMR(400.0 MHz, CDC13): |
| δ= 7,764(6,0); 7,519(0,6); 7,449(1,5); 7,312(3,2); 7,288(0,6); 7,281(0,7); 7,274(1,0); 7,270(1,1); 7,261(99,8); 7,253(0,8); 7,238(1,0); 7,184(0,8); 7,175(1,8); 6,997(0,5); 4,533(1,4); 4,515(4,3); 4,497(4,4); 4,479(1,5); 2,434(16,0); 2,356(2,5); 1,658(5,4); 1,639(11,0); 1,621(5,6); 1,599(1,1); 0,008(1,1); 0,000(31,9); -0,008(0,9) |
| Beispiel 2-50: ¹H-NMR(400.0 MHz, CDC13): |
| δ= 8.132(0.89);8(1.75);7.992(1.3);7.983(1.51);7.902(1.89);7.882(1.32);7.858(1.04);7.519(1.68);7.312(0.71);7.31(0.84);7.26(298. 81);7.25(2.51);7.246(1.94);7.245(1.75);7.238(1.35);7.235(1.24);7.23(1.29);7.226(1.19);7.212(1.82);7.21(2.71);6.996(1.68);4.516 (0.77);4.498(2.34);4.479(2.42);4.462(0.94);3.491(3.07);3.113(16);1.65(4.71);1.632(9.95);1.613(5.13);1.581(1.19);1.284(0.54);1. 264(0.51);0.008(3.84);0.006(1.8);0(131.12);-0.006(2.66);-0.007(2.35);-0.008(4.88);-0.012(1.21);-0.014(1.02);-0.015(1);-0.016(0.93);-0.016(0.92);-0.023(0.72);-0.031(0.65);-0.034(0.59);-0.049(0.89);-0.05(1.13) |
| Beispiel 2-51: ¹H-NMR(400.0 MHz, d₆-DMSO): |
| δ= 11.958(4.87);8.222(1.31);8.204(1.63);8.08(2.62);8.06(2.11);7.941(1.46);7.805(3.15);7.668(1.59);4.415(2.72);4.397(8.24);4.3 78(8.39);4.36(2.91);3.578(16);3.322(26.13);2.6(0.51);2.51(30.41);2.505(58.09);2.501(76.3);2.496(56.56);2.492(29.5);2.376(0.86 );2.328(0.51);1.908(0.61);1.503(7.09);1.485(14.54);1.467(7.11);0(5.05) |
| Beispiel 2-52: ¹H-NMR(400.0 MHz, d₆-DMSO): |
| δ= 11.815(4.01);7.914(0.97);7.894(1.25);7.818(2.55);7.798(1.69);7.613(1.3);7.477(2.89);7.341(1.5);4.406(2.74);4.388(8.88);4.3 69(9);4.351(2.81);3.312(50.97);2.968(1.01);2.949(2.73);2.931(2.85);2.913(1.15);2.674(0.97);2.67(1.36);2.665(1.01);2.523(3.6);2 .518(5.5);2.51(74.19);2.505(159.44);2.5(220.37);2.496(153.51);2.491(68.91);2.455(1.13);2.45(1.5);2.446(1.22);2.332(1);2.327(1 .36);2.323(0.97);1.501(7.28);1.483(16);1.464(7.26);1.172(3.24);1.153(6.44);1.135(3.27);1.124(0.96);0.008(1.66);0(55.84);-0.008(1.75) |
| Beispiel 2-53: ¹H-NMR(400.0 MHz, d₆-DMSO): |
| δ= 11.8750 (1.0); 8.2361 (1.6); 8.0993 (3.4); 8.0449 (1.3); 8.0259 (3.8); 8.0088 (3.9); 7.9895 (1.2); 7.9615 (1.8); 4.3757 (1.5); 4.3582 (4.2); 4.3400 (4.2); 4.3230 (1.5); 4.0382 (1.0); 4.0205 (1.2); 3.3098 (43.4); 3.2858 (6.5); 3.2671 (6.4); 3.2486 (2.4); 2.6696 (1.3); 2.5229 (4.3); 2.5183 (6.1); 2.5095 (73.4); 2.5050 (154.6); 2.5004 (215.2); 2.4958 (148.1); 2.4912 (66.6); 2.3272 (1.3); 1.9877 (4.9); 1.4810 (6.8); 1.4630 (14.4); 1.4448 (6.4); 1.3330 (7.3); 1.3143 (16.0); 1.2958 (7.0); 1.1924 (1.4); 1.1745 (2.6); 1.1567 (1.4); 0.0080 (2.8); -0.0002 (81.0); -0.0086 (2.2) |
| Beispiel 2-54: ¹H-NMR(400.0 MHz, d₆-DMSO): |
| δ= 11.942(2);8.228(1.01);8.207(1.26);8.097(2.84);8.076(2.1);7.94(1.56);7.804(3.52);7.668(1.78);4.409(2.5);4.391(8.06);4.373(8. 18);4.355(2.57);4.019(0.52);3.713(1.22);3.695(3.77);3.676(3.8);3.658(1.26);3.359(0.55);3.309(140.03);2.679(0.52);2.674(1.09); 2.669(1.55);2.665(1.11);2.66(0.51);2.55(0.99);2.546(0.9);2.523(4.38);2.518(6.36);2.509(88.23);2.505(190);2.5(264.68);2.496(18 7.05);2.491(85.07);2.336(0.55);2.332(1.15);2.327(1.58);2.322(1.12);2.318(0.52);1.908(1.85);1.5(7.27);1.482(16);1.464(7.18);1.2 69(4.93);1.251(10.54);1.232(4.85);0.008(0.63);0(21.63);-0.008(0.63) |
| Beispiel 2-61: ¹H-NMR(400.0 MHz, d₆-DMSO): |
| δ= 11.789(0.78);7.832(4.59);7.624(1.36);7.488(3.17);7.351(1.57);4.425(2.21);4.407(7.28);4.389(7.44);4.371(2.36);3.313(44.01); 2.67(0.6);2.523(1.36);2.518(1.97);2.51(31.73);2.505(70.06);2.501(98.7);2.496(68.26);2.491(29.74);2.455(0.71);2.451(0.9);2.446 (0.73);2.423(13.86);2.327(0.66);2.323(0.54);1.506(7.04);1.488(16);1.477(0.88);1.47(7.03);1.295(0.67);0.008(1.25);0(53.01);-0.009(1.52) |
| Beispiel 2-62: ¹H-NMR(400.0 MHz, d₆-DMSO): |
| δ= 11.848(1.55);8.258(0.92);8.12(1.77);8.058(1.16);8.038(2.12);7.993(1.36);7.983(1.5);7.974(0.79);4.411(1.93);4.393(6.15);4.3 75(6.27);4.357(1.93);3.311(34.49);3.092(16);2.674(0.61);2.67(0.82);2.665(0.6);2.523(2.33);2.518(3.43);2.51(46.02);2.505(100.0 5);2.5(138.84);2.496(96.41);2.491(42.71);2.455(0.73);2.45(0.99);2.446(0.72);2.332(0.63);2.327(0.89);2.323(0.63);1.498(6.39);1. 488(0.97);1.48(14.61);1.462(6.34);0.008(1.18);0(40.28);-0.009(1.17) |
| Beispiel 2-63: ¹H-NMR(400.0 MHz, d₆-DMSO): |
| δ= 11.914(5.85);8.15(1.2);8.13(2.85);8.108(3.49);8.087(1.51);7.945(1.55);7.81(3.19);7.673(1.6);4.434(3.14);4.416(10.37);4.398( 10.6);4.379(3.5);3.581(16);3.313(71.82);3.263(0.56);3.094(0.55);2.674(1.08);2.669(1.51);2.665(1.14);2.544(0.83);2.533(0.85);2. 523(4.25);2.518(6.48);2.509(87.05);2.505(187.69);2.5(260.68);2.496(183.67);2.491(83.21);2.455(1.32);2.45(1.82);2.446(1.48);2 .332(1.2);2.327(1.64);2.322(1.21);2.072(0.73);1.908(1.31);1.509(7.03);1.491(14.66);1.473(6.98);0.008(1.03);0(32.97);-0.009(1.01) |
| Beispiel 2-64: ¹H-NMR(400.0 MHz, d₆-DMSO): |
| δ= 11.796(3.41);7.845(5.27);7.625(1.35);7.488(3.09);7.352(1.52);4.425(2.64);4.407(8.62);4.388(8.82);4.37(2.87);3.313(85.07);3 .281(0.78);3.262(1.54);2.967(1);2.949(2.87);2.931(2.97);2.913(1.17);2.674(0.67);2.67(0.96);2.665(0.65);2.523(2.38);2.518(3.4); 2.51(52.5);2.505(116.35);2.501(163.67);2.496(116.08);2.491(54.4);2.451(3.76);2.332(0.79);2.327(1.09);2.323(0.81);1.506(7.17) ;1.488(16);1.47(7.33);1.182(3.23);1.164(6.44);1.145(3.41);0.008(1.54);0(58.7);-0.008(2.17);-0.05(0.55) |
| Beispiel 2-65: ¹H-NMR(400.0 MHz, d₆-DMSO): |
| δ= 11.851(3.1);8.281(0.9);8.144(2.02);8.07(1.29);8.05(2.28);8.007(2.18);7.987(0.74);5.753(3.48);4.412(2.63);4.394(8.03);4.376( 8.01);4.358(2.49);3.308(51.28);3.292(1.78);3.274(2.42);3.256(2.06);3.238(0.73);2.674(0.84);2.669(1.13);2.665(0.81);2.523(3.61 );2.518(5.24);2.509(62.91);2.505(134.1);2.5(185.44);2.496(128.96);2.491(57.39);2.332(0.81);2.327(1.13);2.322(0.77);1.498(7.2) ;1.48(16);1.462(7.08);1.36(3.86);1.342(7.87);1.323(3.7);0.008(1.31);0(44.58);-0.009(1.27) |
| Beispiel 2-66: ¹H-NMR(400.0 MHz, d₆-DMSO): |
| δ= 11.907(6.58);8.148(3 .05);8.132(4.16);8.111 (1.56);7.946(2.21);7.809(4.95);7.673(2.56);5.753(3.58);4.431(4.08);4.413(12.83); 4.395(13.14);4.377(4.33);3.736(1.64);3.718(4.27);3.7(4.27);3.682(1.67);3.308(154.84);2.674(2.34);2.669(3.29);2.664(2.34);2.60 5(0.68);2.6(0.69);2.596(0.52);2.523(11.08);2.518(16.52);2.509(186.01);2.505(391.83);2.5(538.2);2.496(378.66);2.491(170.21);2 .405(0.58);2.401(0.55);2.336(1.18);2.332(2.34);2.327(3.22);2.322(2.24);2.072(0.74);1.507(7.73);1.489(16);1.471(7.64);1.285(4. 92);1.268(9.62);1.249(5.24);0.008(3.72);0(118.16);-0.008(3.62) |
| Beispiel 2-73: ¹H-NMR(400.0 MHz, d₆-DMSO): |
| δ= 11.7312 (2.7); 7.8291 (1.6); 7.8091 (2.3); 7.7295 (1.2); 7.7094 (0.9); 7.6171 (1.2); 7.4801 (2.7); 7.3432 (1.4); 4.4651 (1.8); 4.4469 (5.8); 4.4287 (5.8); 4.4106 (1.9); 4.1047 (1.2); 4.0866 (1.2); 3.3096 (124.1); 2.6740 (0.8); 2.6694 (1.2); 2.6648 (0.8); 2.5396 (1.0); 2.5229 (4.2); 2.5182 (5.7); 2.5095 (67.9); 2.5049 (143.6); 2.5003 (198.8); 2.4958 (137.4); 2.4912 (62.6); 2.4282 (0.5); 2.3895 (14.3); 2.3318 (0.9); 2.3271 (1.2); 2.3225 (0.9); 2.0724 (1.2); 1.5146 (7.0); 1.4965 (16.0); 1.4784 (7.0); 1.3127 (1.3); 1.2947 (2.7); 1.2765 (1.2); -0.0002 (1.4) |
| Beispiel 2-75: ¹H-NMR(400.0 MHz, d₆-DMSO): |
| δ= 11.8398 (0.9); 8.0607 (1.6); 8.0404 (2.3); 7.9492 (1.5); 7.9288 (1.3); 7.7868 (2.0); 7.6505 (1.0); 4.4498 (1.0); 4.4316 (3.1); 4.4135 (3.2); 4.3954 (1.1); 3.5447 (16.0); 3.3082 (73.5); 2.6738 (0.7); 2.6693 (1.0); 2.6645 (0.7); 2.5225 (4.7); 2.5178 (6.5); 2.5091 (57.0); 2.5047 (114.6); 2.5001 (153.9); 2.4956 (107.8); 2.4911 (49.8); 2.3315 (0.6); 2.3269 (0.9); 1.9076 (0.6); 1.5032 (4.2); 1.4851 (9.3); 1.4670 (4.2); 0.0079 (1.0); -0.0002 (22.9); -0.0085 (0.8) |
| Beispiel 2-76: ¹H-NMR(400.0 MHz, d₆-DMSO): |
| δ= 11.734(3.5);7.845(1.51);7.825(2.18);7.736(1.27);7.719(1);7.622(1.21);7.485(2.66);7.348(1.31);4.465(1.85);4.447(5.86);4.429 (5.95);4.411(1.97);3.31(15.7);2.935(1.05);2.917(3.01);2.898(3.1);2.88(1.08);2.523(1.13);2.518(1.54);2.51(27.58);2.505(60.79);2. 5(85.15);2.496(59.71);2.491(26.86);1.516(7.27);1.497(16);1.479(7.16);1.202(3.41);1.184(6.77);1.165(3.3);0.008(1.06);0(37.76); -0.009(1) |
| Beispiel 2-77: ¹H-NMR(400.6 MHz, d₆-DMSO): |
| δ= 11.804(3.11);8.317(0.87);8.18(1.85);8.065(1.77);8.044(2.97);7.875(1.2);7.856(1);5.756(2.22);4.45(2.21);4.432(6.58);4.414(6. 53);4.396(2.09);3.371(0.61);3.348(0.8);3.344(0.92);3.321(147.23);3.3(1.36);3.295(0.98);3.274(1.21);3.256(1.09);3.238(0.85);3.2 2(1.62);3.202(1.7);3.187(0.99);3.168(0.77);2.674(0.56);2.67(0.77);2.665(0.53);2.524(3.93);2.519(5.79);2.51(47.64);2.506(96.69) ;2.501(130.94);2.496(91.41);2.492(40.65);2.48(1.21);2.476(0.93);2.333(0.56);2.328(0.78);2.324(0.53);1.908(1.8);1.508(7.33);1. 49(16);1.472(7.12);1.394(4.4);1.375(8.88);1.357(4.11);0.008(0.79);0(21.3);-0.009(0.63) |
| Beispiel 2-78: ¹H-NMR(400.6 MHz, d₆-DMSO): |
| δ= 11.8469 (6.1); 8.1145 (2.7); 8.0943 (3.8); 8.0140 (3.3); 7.9939 (2.3); 7.9290 (2.0); 7.7926 (4.4); 7.6569 (2.2); 4.4720 (2.6); 4.4539 (7.6); 4.4358 (7.6); 4.4177 (2.6); 3.7188 (1.9); 3.7008 (5.1); 3.6822 (5.2); 3.6641 (1.9); 3.3760 (1.3); 3.3530 (2.2); 3.3273 (264.9); 3.2765 (0.5); 2.6701 (0.7); 2.5237 (3.8); 2.5105 (42.7); 2.5060 (87.1); 2.5014 (118.0); 2.4969 (83.3); 2.4924 (37.8); 2.3332 (0.5); 2.3286 (0.7); 1.5170 (7.8); 1.4989 (16.0); 1.4808 (7.7); 1.3113 (5.4); 1.2930 (10.6); 1.2746 (5.3); -0.0002 (2.2) |
| Beispiel 3-1: ¹H-NMR(400.0 MHz, CDC13): |
| δ= 7.765(0.6);7.745(1.24);7.713(1.31);7.693(0.61);7.518(0.61);7.504(1.1);7.365(2.37);7.26(110.75);7.227(1.16);6.996(0.61);4.4 32(1.91);4.414(2.54);4.395(1.99);2.79(8.62);2.305(16);2.065(1.18);2.047(2.14);2.028(2.13);2.01(1.26);1.018(4.29);0.999(8.94);0 .98(4.15);0.008(1.31);0(45.01);-0.008(1.28) |
| Beispiel 3-2: ¹H-NMR(400.0 MHz, d₆-DMSO): |
| δ= 11.635(1.2);7.994(0.94);7.9(0.62);7.88(1.28);7.857(0.62);7.847(1.64);7.827(0.76);4.323(2.06);4.305(3.02);4.287(2.13);3.31(3 2.73);3.006(16);2.669(0.58);2.587(9.22);2.523(1.72);2.518(2.56);2.51(32.26);2.505(70.05);2.5(97.77);2.496(68.17);2.491(30.37) ;2.327(0.54);1.918(1.19);1.9(2.12);1.882(2.12);1.864(1.21);0.908(4.27);0.89(9.45);0.871 (4.02);0.008(0.59);0(20.02);-0.009(0.56) |
| Beispiel 3-3: ¹H-NMR(400.0 MHz, d₆-DMSO): |
| δ= 11.709(2.02);8.042(0.88);8.022(1.14);7.94(1.98);7.92(2.35);7.783(2.55);7.646(1.19);4.332(3.05);4.314(4.5);4.296(3.16);3.44 9(22.02);3.31(102.47);3.175(0.65);3.162(0.66);2.747(16);2.674(1.3);2.669(1.84);2.665(1.3);2.551(0.5);2.54(0.62);2.535(0.75);2. 523(5.28);2.518(8);2.51(105.64);2.505(228.67);2.5(317.05);2.496(221.05);2.491(98.27);2.463(0.66);2.45(0.99);2.332(1.29);2.32 7(1.81);2.322(1.34);1.919(1.69);1.901(3.04);1.882(3.12);1.864(1.77);0.913(6.63);0.895(14.54);0.876(6.17);0.008(1.76);0(61.61); -0.009(1.8) |
| Beispiel 4-1: ¹H-NMR(400.0 MHz, CDC13): |
| δ= 7.677(6.48);7.521(1.05);7.382(2.45);7.261(15.43);7.244(1.19);7.1(1.13);3.881(13.55);2.72(12.81);2.272(16);0(6.76) |
| Beispiel 4-2: ¹H-NMR(400.0 MHz, CDCl3): |
| δ=7.857(0.66);7.837(1.38);7.803(1.37);7.783(0.66);7.644(2.96);7.268(0.7);7.26(67.37);4.063(1.58);3.915(0.9);3.908(14.64);3.9 05(3.49);3.055(1.21);2.967(0.66);2.958(16);2.62(6.52);0.008(0.82);0(30.16);-0.008(0.93) |
| Beispiel 4-3: ¹H-NMR(400.0 MHz, CDCl3): |
| δ= 7.945(0.87);7.854(3.09);7.807(1.93);7.668(0.94);7.603(0.77);7.519(0.67);7.26(125.38);6.996(0.66);3.971(0.5);3.898(0.6);3.8 74(8.69);3.195(16);2.844(11.22);0.008(1.27);0(48.95);-0.008(1.37) |
| Beispiel 4-4: ¹H-NMR(400.0 MHz, CDCl3): |
| δ= 7.678(5.46);7.536(1.25);7.397(2.9);7.288(1.73);7.26(81.53);3.889(16);2.754(1.62);2.735(5.25);2.726(15.87);2.716(4.96);2.69 8(1.55);1.589(0.52);1.223(5.31);1.204(10.84);1.186(4.93);0.008(1.22);0(28.79);-0.008(0.79) |
| Beispiel 4-5: ¹H-NMR(400.0 MHz, CDCl3): |
| δ= 7.86(4.53);7.842(1.29);7.796(1.85);7.776(1.05);7.262(24.93);5.299(0.8);3.965(16);3.281(0.56);3.262(0.52);3.016(0.93);2.997 (0.98);2.983(0.79);2.964(0.77);2.608(3.34);1.425(3.08);1.406(6.35);1.388(2.93);1.285(0.77);1.255(0.72);0(9.06) |
| Beispiel 4-6: ¹H-NMR(400.0 MHz, CDCl3): |
| δ= 7.957(1.24);7.899(0.89);7.879(2.38);7.862(1.52);7.841(0.91);7.818(2.72);7.68(1.54);7.665(1.82);7.518(2.1);7.355(0.51);7.25 9(384.1);6.995(1.99);5.298(3.43);3.966(1.1);3.869(12.06);3.296(1.61);3.277(5.09);3.258(5.26);3.24(1.71);2.85(16);1.539(2.37);1 .461(1.05);1.43(5.1);1.412(10.1);1.393(4.94);1.37(1.31);1.332(7.88);1.284(10.97);1.256(4.17);0.88(0.71);0.008(5.04);0(144.57); -0.008(5.71) |
| Beispiel 4-25: ¹H-NMR(400.0 MHz, d₆-DMSO): |
| δ= 11.0342 (1.2); 7.8979 (1.1); 7.6361 (2.6); 7.5898 (0.6); 7.4521 (1.3); 7.3146 (0.6); 3.8047 (16.0); 3.4855 (0.5); 3.3085 (38.0); 2.5227 (1.8); 2.5180 (2.6); 2.5093 (28.2); 2.5047 (58.3); 2.5001 (80.0); 2.4956 (54.8); 2.4910 (24.8); 2.4229 (4.6); 2.4014 (0.8); 2.3269 (0.5); 1.2364 (0.9); 1.1167 (1.4); 1.0965 (1.4); 0.6673 (1.5); 0.6553 (1.4); 0.0080 (1.2); 0.0064 (0.5); -0.0002 (34.3); - 0.0085 (1.0) |
| Beispiel 4-28: ¹H-NMR(400.0 MHz, d₆-DMSO): |
| δ= 11.0592 (1.8); 7.9013 (1.8); 7.6478 (3.8); 7.6011 (0.8); 7.4647 (1.5); 7.3261 (0.7); 3.8054 (16.0); 3.3130 (161.1); 2.9099 (1.7); 2.8917 (1.7); 2.6696 (0.9); 2.5230 (2.8); 2.5095 (54.6); 2.5050 (116.8); 2.5005 (162.6); 2.4959 (115.8); 2.4914 (53.3); 2.3274 (0.8); 2.1905 (0.8); 1.9878 (1.6); 1.2375 (0.6); 1.1688 (2.1); 1.1506 (3.6); 1.1319 (2.2); 1.1113 (2.3); 1.0889 (2.2); 0.6566 (2.3); -0.0002 (27.6) |
| Beispiel 4-29: ¹H-NMR(400.0 MHz, d₆-DMSO): |
| δ= 11.1164 (3.0); 8.4288 (0.6); 8.2920 (1.2); 8.1534 (0.7); 7.9208 (2.8); 7.9027 (1.4); 7.8295 (1.3); 7.8110 (0.8); 3.8214 (16.0); 3.3277 (114.0); 3.2046 (1.0); 3.1861 (1.4); 3.1662 (1.1); 2.6853 (0.6); 2.5254 (40.1); 2.5208 (83.0); 2.5163 (114.3); 2.5117 (79.0); 2.5072 (35.9); 2.3431 (0.7); 2.0703 (0.7); 2.0037 (1.6); 1.3551 (2.0); 1.3370 (3.8); 1.3182 (2.1); 1.2747 (0.6); 1.2522 (0.8); 1.2080 (0.8); 1.1903 (1.4); 1.1726 (1.3); 1.0397 (0.8); 0.7382 (1.4) |
| Beispiel 4-30: ¹H-NMR(400.0 MHz, d₆-DMSO): |
| δ= 7.9772 (0.5); 7.9574 (1.2); 7.9376 (1.9); 7.7136 (0.8); 4.5780 (1.8); 3.8021 (16.0); 3.6474 (1.3); 3.6289 (1.4); 3.6107 (0.5); 3.3705 (11.5); 3.1686 (4.0); 2.5234 (1.9); 2.5100 (26.7); 2.5056 (54.0); 2.5011 (72.8); 2.4966 (51.3); 2.4921 (23.8); 1.3612 (1.3); 1.3428 (2.8); 1.3247 (1.5); 1.2356 (0.6); 1.0850 (1.2); 1.0644 (1.1); 0.7614 (1.2); 0.7487 (1.2); -0.0002 (13.2) |
| Beispiel 4-37: ¹H-NMR(400.0 MHz, CDCl3): |
| δ= 8.1451 (0.6); 8.1257 (1.1); 8.1066 (0.7); 7.7963 (3.7); 7.6484 (1.3); 7.6277 (1.2); 7.3332 (1.0); 7.2602 (77.4); 7.1961 (2.1); 7.0590 (1.0); 3.8658 (16.0); 2.4727 (10.8); 1.5622 (0.7); 0.0080 (0.9); -0.0002 (27.2); -0.0085 (0.7) |
| Beispiel 4-38: ¹H-NMR(400.0 MHz, d₆-DMSO): |
| δ= 8.0690 (1.1); 7.9259 (1.1); 7.8977 (0.9); 7.7766 (1.7); 7.7613 (2.4); 7.6255 (1.0); 3.7408 (16.0); 3.3106 (71.9); 3.1751 (1.7); 3.1619 (1.7); 3.0900 (8.1); 3.0503 (0.6); 2.6693 (0.8); 2.5092 (52.8); 2.5049 (102.7); 2.5004 (136.0); 2.4959 (96.5); 2.4916 (46.0); 2.3268 (0.8); 1.2372 (0.8); -0.0002 (12.6) |
| Beispiel 4-39: ¹H-NMR(400.0 MHz, d₆-DMSO): |
| δ= 8.2608 (0.7); 7.9077 (1.3); 7.8869 (1.1); 7.8573 (0.7); 7.7217 (1.7); 7.5859 (0.8); 3.7525 (16.0); 3.7397 (0.8); 3.5804 (1.2); 3.5620 (1.2); 3.3099 (117.1); 2.6740 (0.8); 2.6694 (1.0); 2.6649 (0.7); 2.5228 (4.2); 2.5181 (5.9); 2.5094 (59.8); 2.5049 (123.2); 2.5003 (168.7); 2.4958 (116.6); 2.4912 (53.3); 2.3317 (0.7); 2.3271 (1.0); 2.3225 (0.7); 1.2552 (2.2); 1.2367 (5.2); 1.2184 (2.2); 0.0080 (1.0); -0.0002 (30.6); -0.0085 (1.0) |
| Beispiel 4-40: ¹H-NMR(400.0 MHz, CDCl3): |
| δ= 8.1662 (0.5); 8.1466 (0.8); 8.1276 (0.6); 7.8130 (3.5); 7.6678 (1.1); 7.6472 (1.0); 7.3586 (0.7); 7.2602 (83.1); 7.2215 (1.5); 7.0843 (0.8); 3.9896 (1.5); 3.8706 (16.0); 3.8331 (1.8); 3.6488 (1.5); 2.9588 (0.8); 2.9403 (2.4); 2.9219 (2.5); 2.9034 (0.8); 1.2712 (2.2); 1.2532 (4.4); 1.2348 (2.1); 0.0079 (0.9); -0.0002 (29.5); -0.0085 (0.8) |
| Beispiel 4-41: ¹H-NMR(400.0 MHz, d₆-DMSO): |
| δ= 8.0781 (0.8); 7.9416 (1.1); 7.9227 (0.6); 7.8055 (3.0); 7.7846 (1.1); 7.6695 (1.0); 4.0382 (0.7); 4.0204 (0.7); 3.7485 (1.7); 3.7376 (16.0); 3.6589 (1.0); 3.3441 (0.8); 3.3097 (128.8); 3.2694 (0.8); 3.2490 (0.6); 2.6740 (0.8); 2.6694 (1.0); 2.6648 (0.8); 2.5229 (4.3); 2.5181 (6.0); 2.5094 (59.3); 2.5049 (121.8); 2.5003 (166.1); 2.4958 (114.4); 2.4912 (52.1); 2.3317 (0.7); 2.3271 (1.0); 2.3225 (0.7); 1.9876 (3.0); 1.2482 (2.1); 1.2297 (4.1); 1.2114 (2.3); 1.1923 (1.2); 1.1745 (1.8); 1.1567 (0.9); 0.0080 (0.9); - 0.0002 (29.1); -0.0085 (0.9) |
| Beispiel 4-42: ¹H-NMR(400.0 MHz, d₆-DMSO): |
| δ= 8.2633 (0.9); 7.8929 (1.3); 7.8750 (1.0); 7.8552 (0.8); 7.7189 (2.1); 7.5823 (0.9); 7.4594 (0.5); 3.7397 (8.9); 3.6685 (1.8); 3.6387 (0.9); 3.5752 (1.8); 3.5641 (1.6); 3.5559 (1.8); 3.5101 (0.6); 3.4853 (0.8); 3.3590 (2.0); 3.3475 (1.6); 3.3093 (409.3); 2.6740 (2.2); 2.6694 (3.0); 2.6647 (2.2); 2.5497 (2.2); 2.5228 (13.1); 2.5181 (18.6); 2.5094 (180.5); 2.5049 (368.1); 2.5003 (502.5); 2.4957 (348.7); 2.4912 (159.5); 2.4706 (1.3); 2.4655 (1.4); 2.3317 (2.1); 2.3270 (2.9); 2.3224 (2.1); 1.3196 (1.5); 1.2982 (0.8); 1.2538 (3.7); 1.2357 (16.0); 1.2178 (4.1); 1.2013 (1.0); 0.8538 (1.4); 0.8364 (0.6); 0.0080 (3.4); -0.0002 (158.0); -0.0085 (7.0); -0.1501 (0.6) |
| Beispiel 4-49: ¹H-NMR(400.0 MHz, d₆-DMSO): |
| δ= 11.333(0.88);7.918(0.83);7.859(0.7);7.84(0.92);7.776(1.1);7.755(0.72);7.605(0.64);7.468(1.32);7.332(0.65);3.791(16);3.474(2.91);2.674(0.65);2.669(0.91);2.665(0.62);2.523(2.01);2.518(3.06);2.51(48.55);2.505(107.49);2.5(151.19);2.496(105.96);2.491( 47.61);2.455(1.15);2.45(1.42);2.446(1.18);2.425(5.59);2.332(0.73);2.327(1.04);2.322(0.75);0.008(1.02);0(43.65);-0.008(1.4) |
| Beispiel 4-50: ¹H-NMR(400.0 MHz, d₆-DMSO): |
| δ= 8.063(0.72);7.985(0.92);7.926(0.75);3.78(16);3.092(3.3);2.523(1.85);2.518(2.82);2.51 (30.57);2.505(63.43);2.5(86.36);2.496( 61.52);2.491(28.65);2.327(0.55) |
| Beispiel 4-51: ¹H-NMR(400.0 MHz, d₆-DMSO): |
| δ= 8.176(1.48);8.076(1.77);8.055(1.58);7.96(2.24);7.823(1.95);3.825(16);3.595(10.57);3.402(63.21);2.694(1.86);2.548(4.42);2.5 43(6.94);2.534(108.49);2.53(231.87);2.525(318.71);2.521(225.86);2.516(103.46);2.352(1.86) |
| Beispiel 4-52: ¹H-NMR(400.0 MHz, d₆-DMSO): |
| δ= 11.3456 (1.1); 7.9188 (1.0); 7.8657 (0.7); 7.8474 (1.0); 7.7935 (1.2); 7.7743 (0.8); 7.6052 (0.6); 7.4685 (1.2); 7.3325 (0.6); 3.7908 (16.0); 3.4179 (1.9); 3.1684 (5.6); 2.9482 (1.6); 2.9298 (1.6); 2.6695 (0.6); 2.5230 (1.3); 2.5183 (2.0); 2.5096 (33.3); 2.5050 (73.7); 2.5004 (103.6); 2.4958 (72.8); 2.4912 (33.0); 2.4549 (0.7); 2.4505 (0.9); 2.4459 (0.6); 2.3272 (0.7); 1.1703 (1.7); 1.1522 (3.2); 1.1342 (1.9); 0.0080 (0.7); -0.0002 (27.9); -0.0085 (0.9) |
| Beispiel 4-53: ¹H-NMR(400.0 MHz, d₆-DMSO): |
| δ= 8.2356 (0.6); 8.0988 (1.2); 8.0019 (1.7); 7.9620 (0.9); 7.9317 (0.9); 5.7549 (2.5); 5.7532 (2.6); 3.7799 (16.0); 3.5748 (3.5); 3.2873 (1.5); 3.2691 (1.5); 2.5052 (49.2); 2.5013 (61.2); 2.4979 (46.0); 1.3319 (1.7); 1.3137 (3.2); 1.2961 (1.8); 0.0014 (8.0); - 0.0002 (8.3) |
| Beispiel 4-54: ¹H-NMR(400.0 MHz, d₆-DMSO): |
| δ= 8.1904 (0.7); 8.1708 (0.9); 8.0763 (1.0); 8.0562 (0.7); 7.9493 (1.4); 7.7989 (1.0); 4.7745 (1.3); 3.7990 (16.0); 3.7420 (0.7); 3.7242 (0.8); 3.6949 (1.3); 3.6772 (1.3); 3.6598 (0.6); 3.6191 (0.6); 3.3703 (14.0); 2.6701 (0.6); 2.5233 (3.0); 2.5100 (40.0); 2.5055 (79.0); 2.5010 (105.4); 2.4965 (74.2); 2.4920 (35.0); 2.3277 (0.6); 1.2679 (1.5); 1.2498 (2.8); 1.2322 (1.6); 1.1195 (0.8); 1.1018 (1.6); 1.0840 (0.8); 0.0080 (1.4); -0.0002 (36.1); -0.0085 (1.3) |
| Beispiel 4-61: ¹H-NMR(400.0 MHz, d₆-DMSO): |
| δ= 11.317(1.92);7.912(1.73);7.8(4.35);7.617(0.87);7.48(1.79);7.344(0.9);3.815(16);3.311(53.37);3.261(0.82);2.674(0.73);2.669( 0.99);2.665(0.76);2.552(0.59);2.523(2.3);2.518(3.39);2.51(52.75);2.505(116.08);2.5(162.65);2.496(114.42);2.491(53.15);2.451( 3.86);2.421(9.65);2.332(0.83);2.327(1.16);2.322(0.84);0.008(1.51);0(61.65);-0.008(2.21) |
| Beispiel 4-63: ¹H-NMR(400.0 MHz, d₆-DMSO): |
| δ= 11.473(3.87);8.107(7.38);7.969(1.31);7.946(3.93);7.832(2.21);7.696(1.12);3.855(16);3.603(14.46);3.338(145.94);2.697(1.06) ;2.528(176.33);2.355(1.03);2.015(1.04);1.202(0.57) |
| Beispiel 4-64: ¹H-NMR(400.0 MHz, d₆-DMSO): |
| δ= 11.328(2.17);7.912(2.21);7.816(2.84);7.619(0.75);7.483(1.49);7.346(0.7);3.815(16);3.315(46.07);3.263(0.82);2.968(0.84);2.9 49(2.25);2.931(2.34);2.913(1.01);2.674(0.67);2.67(0.94);2.665(0.68);2.523(2.27);2.518(3.58);2.51(54.71);2.505(116.11);2.5(158 .97);2.496(112.64);2.491(52.4);2.447(3.71);2.332(0.8);2.327(1.05);2.323(0.77);1.182(2.46);1.164(4.91);1.146(2.73);0.008(1.35); 0(52.7);-0.008(2.08) |
| Beispiel 4-65: ¹H-NMR(400.0 MHz, d₆-DMSO): |
| δ= 8.174(1.02);8.052(1.27);7.986(1.22);7.955(1.36);3.828(12.61);3.548(16);3.297(1.3);2.698(0.93);2.533(111.33);2.528(151.51) ;2.524(117.84);2.354(0.88);1.388(1.61);1.368(2.99) |
| Beispiel 4-66: ¹H-NMR(400.0 MHz, d₆-DMSO): |
| δ= 8.098(2.51);7.949(1.23);7.805(0.67);5.754(2.65);4.135(16);3.824(6.27);3.716(1.04);3.698(1.05);2.507(35.28);2.503(46.53);2. 499(35.74);1.282(1.14);1.263(2.14);1.245(1.19);0(5.49) |
| Beispiel 4-73: ¹H-NMR(400.0 MHz, d₆-DMSO): |
| δ= 11.258(2.68);7.908(2.46);7.808(1.75);7.789(2.35);7.684(2.11);7.665(1.62);7.61(1.19);7.473(2.51);7.336(1.22);3.852(16);3.30 9(29.72);2.669(0.58);2.523(1.41);2.518(2.12);2.509(31.57);2.505(68.77);2.5(96.02);2.496(68.07);2.491(30.91);2.387(14.1);2.33 2(0.52);2.327(0.68);2.322(0.51);0.008(1.48);0(50.83);-0.009(1.48) |
| Beispiel 4-74: ¹H-NMR(400.0 MHz, d₆-DMSO): |
| δ= 8.201(1.16);8.066(1.67);7.951(1.79);7.849(1.28);7.828(1.05);3.865(16);3.086(7.03);2.698(0.95);2.529(162.8);2.354(0.98) |
| Beispiel 4-75: ¹H-NMR(400.0 MHz, d₆-DMSO): |
| δ= 8.0572 (2.0); 8.0369 (2.9); 7.9281 (4.6); 7.9137 (2.1); 7.7875 (2.1); 7.6509 (1.0); 3.8625 (16.0); 3.8380 (4.4); 3.7837 (15.1); 3.5457 (15.0); 3.0584 (0.7); 2.6744 (1.3); 2.6697 (1.8); 2.6653 (1.3); 2.5516 (1.8); 2.5449 (4.6); 2.5309 (9.4); 2.5097 (122.3); 2.5052 (234.2); 2.5007 (305.7); 2.4961 (214.7); 2.4916 (98.4); 2.4519 (1.3); 2.3321 (1.3); 2.3275 (1.8); 2.3229 (1.3); 0.0079 (1.3); -0.0002 (26.9); -0.0084 (0.9) |
| Beispiel 4-76: ¹H-NMR(400.0 MHz, d₆-DMSO): |
| δ= 11.263(2.98);7.908(3.37);7.823(1.96);7.802(2.43);7.689(2.25);7.67(1.89);7.615(1.23);7.478(2.49);7.34(1.24);3.851(16);3.308 (51.97);2.933(1.48);2.915(3.76);2.897(3.97);2.878(1.5);2.669(0.84);2.509(53.27);2.505(113.96);2.5(157.5);2.496(110.8);2.491(5 0.03);2.327(0.81);1.2(4.11);1.182(8.11);1.164(4.14);0.008(1.49);0(46.07);-0.008(1.32) |
| Beispiel 4-77: ¹H-NMR(400.0 MHz, d₆-DMSO): |
| δ= 11.325(4.98);8.316(0.92);8.18(1.86);8.044(2.62);8.025(2.17);7.914(3.12);7.83(2.03);7.812(1.72);3.841(16);3.314(108.52);3.2 74(2.19);3.259(1.75);3.235(1.04);3.214(1.46);3.198(1.43);3.18(1.13);3.164(0.82);2.674(1.48);2.67(1.94);2.665(1.38);2.523(7.61) ;2.509(130.64);2.505(254.8);2.5(331.08);2.496(233.82);2.492(114.74);2.332(1.64);2.327(2.06);2.072(0.76);1.393(3.77);1.375(7. 41);1.356(3.97);0.008(2.62);0.001(36.17);0(52.19);-0.008(2.17) |
| Beispiel 4-78: ¹H-NMR(400.0 MHz, d₆-DMSO): |
| δ= 8.086(1.73);8.066(2.41);7.954(2.16);7.928(3.89);7.788(1.83);7.652(0.87);3.861(13.04);3.774(16);3.698(3.82);3.679(3.57);3.6 61(1.52);2.67(1.78);2.523(4.5);2.518(6.77);2.51(98.48);2.505(213.47);2.501(296.57);2.496(207.91);2.492(92.34);2.328(1.65);2. 072(1.37);1.306(3.22);1.288(6.95);1.27(3.01);0.008(1.64);0(58.19);-0.008(1.91) |
| Beispiel 5-1: ¹H-NMR(400.0 MHz, CDCl3): |
| δ= 7.783(0.59);7.685(0.8);7.665(1.75);7.633(1.65);7.613(0.77);7.503(0.93);7.365(2.03);7.26(83.93);7.227(0.97);7.21(0.55);4.14 5(1.68);2.76(10.22);2.522(1.57);2.513(12.53);2.495(1.81);2.468(1.37);2.297(16);2.262(1.93);1.55(1.06);1.269(0.93);1.038(0.9);0 .008(0.97);0(35.94);-0.008(1.14) |
| Beispiel 5-2: ¹H-NMR(400.0 MHz, CDCl3): |
| δ= 7.842(0.83);7.822(1.2);7.741(1.21);7.721(0.83);7.519(0.81);7.26(149.61);7.21(1.2);6.996(0.81);2.95(16);2.56(6.37);2.502(15. 6);1.548(1.99);0.008(1.71);0(63.1);-0.008(1.72) |
| Beispiel 6-4: ¹H-NMR(400.0 MHz, CDCl3): |
| δ= 7.7(1.58);7.68(2.83);7.667(1.09);7.623(2.63);7.603(1.55);7.524(1.52);7.386(3.25);7.266(0.59);7.266(0.68);7.265(0.87);7.264( 1.12);7.26(67.17);7.248(1.69);2.777(2.16);2.759(6.92);2.751(16);2.74(6.59);2.722(2.12);1.581(1.4);1.243(7.3);1.225(15.01);1.20 6(6.88);0.008(0.78);0(26.31);-0.008(0.67) |
| Beispiel 6-5: ¹H-NMR(400.0 MHz, CDCl3): |
| δ= 9.616(0.89);7.825(3.54);7.805(5.32);7.738(7.15);7.718(4.84);7.519(2.95);7.295(0.71);7.287(0.59);7.283(0.66);7.281(0.62);7. 28(0.82);7.279(0.94);7.278(0.98);7.277(1.06);7.276(1.11);7.276(1.23);7.275(1.28);7.274(1.54);7.273(1.58);7.272(1.75);7.272(2. 04);7.271(2.41);7.27(2.79);7.269(3.23);7.269(3.51);7.268(3.91);7.267(4.83);7.266(6.04);7.265(7.71);7.264(9.93);7.26(522.59);7. 256(6);7.255(4.35);7.254(3.49);7.253(2.95);7.252(2.34);7.252(1.78);7.251(1.71);7.25(1.38);7.249(1.32);7.248(1.32);7.248(1.23); 7.247(1.01);7.246(0.92);7.245(1);7.244(0.79);7.244(0.78);7.243(0.69);7.242(0.73);7.241(0.67);7.24(0.59);7.24(0.71);7.239(0.64) ;7.236(0.59);7.232(0.52);7.228(0.62);7.21(0.65);6.996(2.77);5.298(0.53);3.265(0.62);3.247(1.93);3.229(2.25);3.214(2.55);3.195( 2.39);3.177(0.8);2.982(1.09);2.963(4.08);2.948(1.27);2.944(4.25);2.93(3.43);2.925(1.54);2.91(3.31);2.892(1.06);2.506(16);1.576 (67.91);1.49(1.05);1.421(13.84);1.402(28.46);1.383(12.97);1.37(1);1.33(0.75);1.285(1.54);1.256(1.71);0.146(0.57);0.008(6.02);0 (206.24);-0.008(5.61);-0.149(0.6) |
| Beispiel 6-6: ¹H-NMR(400.0 MHz, CDCl3): |
| δ= 8.161(0.52);7.924(1.71);7.904(2.51);7.821(1.51);7.81(1.18);7.801(1.07);7.673(1.84);7.535(0.94);7.26(73.64);3.286(1.79);3.2 67(5.88);3.248(5.99);3.23(1.85);2.806(16);1.547(3.33);1.426(5.36);1.407(11.09);1.389(5.05);0.008(1.08);0(28.46);-0.008(0.85) |
| Beispiel 7-1: ¹H-NMR(400.0 MHz, CDC13): |
| δ= 7.688(0.76);7.668(1.42);7.624(1.56);7.604(0.83);7.496(1.04);7.357(2.44);7.266(0.56);7.266(0.68);7.265(0.85);7.26(52.32);7. 219(1.26);4.126(0.93);2.748(10.89);2.582(0.99);2.531(16);2.46(0.81);2.283(15.03);0.008(0.75);0(22.28);-0.008(0.61) |
| Beispiel 7-2: ¹H-NMR(400.0 MHz, d₆-DMSO): |
| δ= 7,979(1,0); 7,842(0,5); 7,790(5,8); 5,753(1,6); 3,309(25,9); 2,989(16,0); 2,537(9,3); 2,523 (1,4); 2,518(1,8); 2,509(19,5); 2,505(41,8); 2,500(58,2); 2,496(41,3); 2,491(19,5); 2,485(13,1); 0,000(13,3) |
| Beispiel 7-3: ¹H-NMR(400.0 MHz, CDCl3): |
| δ= 7.938(1.26);7.842(1.97);7.822(0.54);7.8(2.82);7.661(1.44);7.52(1.23);7.311(0.5);7.261(208.56);6.996(1.21);3.206(16);2.831( 14.52);2.534(13.34);1.545(2.44);0.008(2.67);0(92.86);-0.008(2.76) |
| Beispiel 7-4: ¹H-NMR(400.0 MHz, CDCl3): |
| δ= 7.689(1.07);7.668(2.22);7.632(1.94);7.612(0.94);7.517(0.93);7.379(2.09);7.261(28.01);7.24(1.03);5.299(1.21);2.764(1.27);2. 745(4.06);2.734(11.3);2.726(4.32);2.708(1.29);2.526(16);1.229(4.49);1.21(9.27);1.192(4.23);0(10.2) |
| Beispiel 7-11: ¹H-NMR(400.0 MHz, CDCl3): |
| δ= 7.66(1);7.64(2.9);7.62(2.1);7.599(0.76);7.513(1.11);7.374(2.53);7.261(31.84);7.236(1.28);5.299(2.55);3.217(0.86);3.199(2.88 );3.18(2.94)3.162(0.9);2.532(16);2.313(15.92);1.25(1.49);1.232(2.93);1.213(1.43);0(13.53) |
| Beispiel 7-12: ¹H-NMR(400.0 MHz, d₆-DMSO): |
| δ= 12.191(0.74);8.105(0.88);7.854(0.51);7.833(2.41);7.823(1.92);3.311(26.21);3.175(0.51);3.162(0.51);3.03(16);2.846(0.55);2.5 23(0.76);2.518(1.24);2.51(16.09);2.505(34.38);2.5(47.68);2.496(33.94);2.491(18.19);1.209(2.47);1.191(5.96);1.172(2.44);0(12.0 7) |
| Beispiel 7-13: ¹H-NMR(400.0 MHz, d₆-DMSO): |
| δ= 12.284(1.91);7.972(2.8);7.952(5.13);7.914(6.07);7.895(4.46);7.76(5.53);7.624(2.74);3.425(40.65);3.375(0.71);3.31(156.79);3 .26(1.2);3.19(4.01);3.174(4.57);3.162(2.89);3.019(0.57);2.67(1.54);2.509(103.43);2.505(199.64);2.5(263.74);2.496(199.47);2.45 (2.82);2.327(1.79);1.232(7.42);1.214(16);1.196(7.18);1.097(0.77);1.022(0.74);1.006(0.65);0(45.05) |
| Beispiel 7-14: ¹H-NMR(400.0 MHz, d₆-DMSO): |
| δ= 12.098(1.07);7.667(3.86);7.663(4.92);7.638(1.19);7.5(3.23);7.363(1.46);5.754(3.08);3.31(89.88);3.26(0.78);3.087(0.6);3.07(1 .7);3.051(1.72);3.033(0.63);2.795(1.75);2.776(5.81);2.758(5.86);2.739(1.86);2.674(0.64);2.669(0.87);2.665(0.63);2.523(1.96);2. 518(2.92);2.51(47.85);2.505(105.77);2.5(149.11);2.496(104.09);2.491(46.99);2.486(16.77);2.467(0.77);2.455(1.29);2.451(1.37); 2.446(0.86);2.332(0.69);2.327(0.94);2.322(0.68);2.072(0.57);1.174(7.09);1.166(3.91);1.155(16);1.147(9.93);1.137(7.11);1.128(3 .84);0.008(2.34);0.006(0.52);0.006(0.55);0.005(0.65);0.004(0.88);0.003(1.49);0.002(2.69);0.002(4.22);0(83.52);-0.003(4.54);-0.003(2.92);-0.004(1.79);-0.005(1.32);-0.006(1.09);-0.007(0.97);-0.008(0.99);-0.008(2.5);-0.011(0.55);-0.05(0.54) |
| Beispiel 7-15: ¹H-NMR(400.0 MHz, d₆-DMSO): |
| δ= 12.2(1.5);8.087(0.69);7.857(1.06);7.836(4.77);7.826(4.02);7.806(0.88);3.31(124.99);3.29(0.62);3.271(1.38);3.26(1.29);3.253( 1.64);3.238(2.02);3.22(1.95);3.201(0.56);3.118(0.5);3.099(1.68);3.08(1.8);3.066(1.33);3.047(1.22);2.964(0.58);2.87(0.72);2.853( 0.89);2.836(0.66);2.674(0.8);2.669(1.14);2.665(0.82);2.523(2.79);2.518(4.14);2.51(63.33);2.505(140.43);2.5(198.05);2.496(137. 85);2.491(63.3);2.486(24.91);2.46(0.94);2.455(1.39);2.45(1.88);2.446(1.55);2.441(0.97);2.332(0.98);2.327(1.27);2.322(0.96);1.3 23(7.23);1.305(16);1.286(6.94);1.203(5.31);1.185(13.1);1.166(5.3);0.008(1.44);0(59.62);-0.009(1.78) |
| Beispiel 7-16: ¹H-NMR(400.0 MHz, d₆-DMSO): |
| δ= 12.285(1.74);7.987(1.13);7.967(2.5);7.939(3.39);7.918(1.49);7.891(1.53);7.754(3.59);7.618(1.8);3.495(1.78);3.476(6.1);3.45 8(6.27);3.44(1.93);3.31(113.65);3.26(0.7);3.166(1.64);3.148(1.68);2.674(0.9);2.669(1.26);2.665(0.92);2.523(3.54);2.518(5.03);2. 51(67.76);2.505(148.13);2.5(208.18);2.496(146.16);2.491(71.36);2.47(0.55);2.455(1.22);2.45(1.56);2.446(1.18);2.332(0.94);2.3 27(1.33);2.322(0.95);1.265(6.82);1.247(16);1.228(7.02);1.22(4.53);1.202(10.39);1.184(4.33);0.008(2.23);0(85.2);-0.004(1.13);-0.005(0.79);-0.006(0.68);-0.007(0.63);-0.008(2.41) |
| Beispiel 7-47: ¹H-NMR(400.0 MHz, d₆-DMSO): |
| δ= 12.3070 (2.4); 11.4223 (0.7); 7.7989 (6.2); 7.7790 (14.0); 7.7563 (16.0); 7.7361 (5.9); 7.5962 (5.3); 7.4592 (11.9); 7.3226 (5.8); 4.0859 (0.7); 4.0077 (0.6); 3.8043 (0.6); 3.3105 (152.1); 3.1746 (3.3); 3.1626 (3.0); 2.6742 (2.9); 2.6696 (3.6); 2.6648 (2.8); 2.5868 (5.2); 2.5094 (218.0); 2.5050 (397.9); 2.5004 (513.0); 2.4960 (361.2); 2.4915 (177.5); 2.4520 (5.4); 2.4305 (19.4); 2.4139 (76.2); 2.3930 (3.2); 2.3629 (1.2); 2.3479 (1.8); 2.3321 (2.7); 2.3272 (3.4); 2.2618 (1.7); 2.1052 (5.1); 1.9153 (0.6); 1.2989 (0.5); 1.2353 (2.3); -0.0002 (43.6) |
| Beispiel 7-48: ¹H-NMR(400.0 MHz, d₆-DMSO): |
| δ= 12.3984 (0.7); 8.2036 (0.9); 8.0666 (1.5); 7.9723 (2.9); 7.9652 (3.2); 7.9448 (0.5); 7.9295 (1.0); 3.3267 (60.2); 3.0981 (16.0); 2.5385 (2.0); 2.5338 (2.8); 2.5251 (27.8); 2.5205 (57.2); 2.5159 (78.4); 2.5114 (53.6); 2.5068 (25.0); 2.4994 (6.9) |
| Beispiel 7-49: ¹H-NMR(400.0 MHz, d₆-DMSO): |
| δ= 12.4960 (1.2); 8.1011 (1.2); 8.0495 (2.0); 8.0291 (1.2); 7.9414 (1.0); 7.8049 (2.4); 7.6679 (1.2); 4.0541 (1.1); 4.0363 (1.0); 3.5704 (16.0); 3.3765 (0.6); 3.3257 (79.3); 3.3134 (1.8); 3.3089 (0.9); 3.3031 (0.5); 3.2759 (1.2); 3.0732 (0.6); 2.6895 (0.7); 2.6850 (1.0); 2.5892 (0.6); 2.5759 (0.8); 2.5711 (0.9); 2.5665 (0.8); 2.5501 (0.7); 2.5387 (3.2); 2.5340 (4.9); 2.5253 (52.3); 2.5208 (109.5); 2.5162 (152.1); 2.5116 (105.5); 2.5071 (49.3); 2.5002 (5.5); 2.4954 (2.7); 2.4715 (1.2); 2.4670 (1.8); 2.4623 (1.6); 2.4574 (1.1); 2.4540 (0.7); 2.4451 (0.6); 2.4404 (0.5); 2.4173 (0.7); 2.3430 (0.9); 2.2580 (0.6); 2.0036 (4.6); 1.2081 (1.4); 1.1904 (2.7); 1.1726 (1.4) |
| Beispiel 7-50: ¹H-NMR(400.0 MHz, d₆-DMSO): |
| δ= 7.7852 (0.8); 7.7749 (1.0); 7.4596 (0.7); 3.3497 (16.0); 2.9360 (1.0); 2.9176 (1.0); 2.5123 (3.5); 2.5077 (7.3); 2.5031 (10.1); 2.4986 (7.0); 2.4940 (3.4); 2.4845 (1.4); 1.9175 (0.6); 1.1652 (1.2); 1.1468 (2.5); 1.1364 (0.6); 1.1284 (1.2) |
| Beispiel 7-51: ¹H-NMR(400.0 MHz, d₆-DMSO): |
| δ= 7.9641 (0.7); 7.9515 (0.8); 3.3105 (16.0); 3.2738 (0.7); 3.2553 (0.7); 2.5229 (0.6); 2.5094 (10.1); 2.5049 (21.0); 2.5004 (28.8); 2.4958 (20.0); 2.4913 (9.1); 2.4774 (2.2); 1.3241 (0.8); 1.3055 (1.6); 1.2868 (0.8) |
| Beispiel 7-52: ¹H-NMR(400.0 MHz, d₆-DMSO): |
| δ= 8.1218 (0.9); 7.9739 (9.1); 7.9528 (1.2); 7.9095 (1.7); 7.8961 (1.2); 7.8764 (1.1); 7.7728 (3.7); 7.6365 (1.8); 4.0383 (1.7); 4.0206 (1.7); 4.0028 (1.0); 3.6786 (3.5); 3.6600 (7.9); 3.6416 (7.8); 3.6227 (3.2); 3.3074 (136.1); 3.2562 (1.0); 2.6692 (6.8); 2.5089 (156.6); 2.5046 (276.6); 2.5001 (358.5); 2.4956 (270.3); 2.4461 (1.1); 2.4045 (17.7); 2.3269 (2.1); 2.1780 (0.7); 2.1232 (1.0); 1.9875 (5.0); 1.9077 (0.5); 1.2461 (8.2); 1.2276 (16.0); 1.2090 (7.3); 1.1923 (1.7); 1.1746 (2.9); 1.1567 (1.6); 0.8735 (0.8); 0.8545 (0.5); -0.0002 (25.6) |
| Beispiel 7-59: ¹H-NMR(400.0 MHz, d₆-DMSO): |
| δ= 12.285(11.15);7.797(7.79);7.777(16);7.741(11.02);7.722(6.04);7.607(7.23);7.588(1.37);7.543(1.25);7.47(15.69);7.333(7.54);3. 806(2.57);3.309(308.67);3.259(2.39);2.674(3.82);2.669(5.18);2.665(3.73);2.556(1.87);2.522(21.21);2.509(336.55);2.505(677.17); 2.5(910.54);2.496(647.97);2.491(314.67);2.451(8.82);2.409(92.72);2.39(5.64);2.332(4.77);2.327(6.16);2.323(4.7);2.072(8.25);0.1 46(1.44);0.008(16.38);0(391.24);-0.008(14.27);-0.05(2.19);-0.15(1.69) |
| Beispiel 7-60: ¹H-NMR(400.0 MHz, d₆-DMSO): |
| δ= 3.3107 (16.0); 3.0709 (3.6); 2.5228 (0.7); 2.5095 (13.7); 2.5050 (28.8); 2.5005 (39.6); 2.4959 (27.7); 2.4915 (12.6); 2.4611 (2.8) |
| Beispiel 7-62: ¹H-NMR(400.0 MHz, d₆-DMSO): |
| δ= 12.297(3.89);7.813(2.72);7.793(4.79);7.745(2.81);7.725(1.74);7.608(2.26);7.471(5.25);7.335(2.53);3.312(116.32);3.279(1.3); 3.262(1.7);3.243(0.79);2.954(2.05);2.935(6.37);2.917(6.6);2.898(2.49);2.674(1.25);2.67(1.78);2.665(1.3);2.551(0.94);2.523(4.29) ;2.518(6.37);2.51(95.36);2.505(211.57);2.5(298.93);2.496(212.49);2.491(102.95);2.451(6.21);2.332(1.45);2.327(1.99);2.323(1.49 );2.073(0.82);1.174(7.7);1.156(16);1.137(7.73);0.008(2.67);0.006(0.55);0.006(0.59);0.005(0.7);0(94.47);-0.005(2.23);-0.006(1.94);-0.007(1.75);-0.008(3.38);-0.051(0.56) |
| Beispiel 7-71: ¹H-NMR(400.6 MHz, d₆-DMSO): |
| δ= 7.808(1.5);7.789(1.75);7.615(1.07);7.595(0.94);7.583(1.11);7.446(2.14);7.309(1.05);4.152(16);3.187(1.47);3.183(2.73);3.179( 3.8);3.175(2.65);3.171(1.34);2.58(1.33);2.551(6.96);2.547(13.3);2.542(17.67);2.538(12.57);2.533(6.12);2.502(3.34);2.393(11.85) ;2.375(0.65);2.047(0.78);0(1.87) |
| Beispiel 7-72: ¹H-NMR(400.0 MHz, d₆-DMSO): |
| δ= 8.3017 (0.6); 8.1636 (1.3); 8.0261 (0.7); 7.9259 (1.1); 7.9062 (1.2); 7.6232 (1.2); 7.6031 (1.1); 3.3090 (37.8); 3.0268 (16.0); 2.6739 (0.7); 2.6693 (1.0); 2.6646 (0.7); 2.5556 (0.5); 2.5507 (0.6); 2.5227 (3.6); 2.5180 (5.1); 2.5093 (54.6); 2.5048 (112.2); 2.5002 (152.6); 2.4956 (105.2); 2.4911 (47.9); 2.4561 (0.6); 2.4518 (0.6); 2.3878 (10.1); 2.3316 (0.7); 2.3270 (0.9); 2.3224 (0.6); 1.9075 (1.3); 0.0080 (1.2); -0.0002 (32.9); -0.0085 (1.0) |
| Beispiel 7-74: ¹H-NMR(400.0 MHz, d₆-DMSO): |
| δ= 12.225(3.02);7.802(3.59);7.782(4.31);7.625(2.36);7.605(4);7.468(4.83);7.331(2.29);3.308(31.48);2.922(2.06);2.904(6.48);2.8 85(6.79);2.867(2.46);2.67(0.72);2.523(2.24);2.51(44.57);2.505(95.06);2.5(132.34);2.496(96.1);2.491(47.54);2.327(0.76);1.193(7. 72);1.174(16);1.156(7.79);0.008(1.47);0(50.69);-0.009(1.57) |
| Beispiel 7-75: ¹H-NMR(400.0 MHz, d₆-DMSO): |
| δ= 8.3100 (0.6); 8.1716 (1.3); 8.0333 (0.7); 7.8209 (1.7); 7.8009 (1.9); 7.4686 (2.2); 7.4487 (2.0); 4.0881 (0.7); 4.0747 (0.8); 3.3109 (62.3); 3.2707 (1.2); 3.2518 (0.8); 3.2372 (1.2); 3.2183 (1.1); 3.1743 (3.5); 3.1613 (3.8); 3.1444 (1.0); 3.1262 (1.1); 3.1106 (0.7); 3.0927 (0.6); 2.6693 (0.6); 2.5498 (0.7); 2.5450 (0.7); 2.5046 (66.7); 2.5001 (94.3); 2.4957 (73.7); 2.3270 (0.6); 2.2695 (16.0); 1.3734 (3.2); 1.3549 (6.7); 1.3363 (3.2); 0.0080 (0.9); -0.0002 (27.5); -0.0084 (1.6) |
| Beispiel 9-25: ¹H-NMR(400.0 MHz, CDCl3): |
| δ= 7.963(1.47);7.942(1.75);7.712(1.65);7.692(1.43);7.518(0.58);7.449(0.9);7.312(1.97);7.271(0.56);7.271(0.56);7.268(0.79);7.25 9(100.61);7.174(0.95);6.995(0.57);2.423(16);0.008(1.4);0(43.08);-0.008(1.14) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (^{®}Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

Die hier verwendeten Abkürzungen bedeuten:

| | | | |
|---|---|---|---|
| ABUTH | Abutilon theophrasti | ALOMY | Alopecurus myosuroides |
| AMARE | Amaranthus retroflexus | AVEFA | Avena fatua |
| CYPES | Cyperus serotinus | MATIN | Matricaria inodora |
| PHBPU | Pharbitis purpureum | POLCO | Polygonum convolvulus |
| SETVI | Setaria viridis | STEME | Stellaria media |
| VERPE | Veronica persica | VIOTR | Viola tricolor |

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigten zahlreiche erfindungsgemäße Verbindungen bei einer Aufwandmenge von 320 g oder weniger pro Hektar eine mindestens 80%-ige Wirkung gegen eine Vielzahl bedeutender Schadpflanzen.
Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen. Die Daten nachfolgender Tabellen A und B zeigen beispielhaft die herbizide Wirkung der erfindungsgemäßen Verbindungen im Vorauflauf, wobei die herbizide Wirkung in Prozent angegeben ist.

**Tabelle A: Vorauflauf**

| **Beispiel Nr.** | **Dosierung [g/ha]** | **ALOMY** | **AVEFA** | **CYPES** | **SETVI** | **ABUTH** |
|---|---|---|---|---|---|---|
| 1-1 | 320 | 100 | 100 | 80 | 100 | 100 |
| 1-2 | 320 | 100 | 100 | 90 | 100 | 100 |
| 1-3 | 320 | 100 | 100 | 90 | 100 | 100 |
| 1-4 | 320 | 100 | 100 | 80 | 100 | 100 |
| 1-5 | 320 | 100 | 100 | 100 | 100 | 100 |
| 1-6 | 320 | 100 | 100 | 100 | 100 | 100 |
| 1-13 | 320 | 100 | 100 | 100 | 100 | 100 |
| 1-14 | 320 | 100 | 100 | 100 | 100 | 100 |
| 1-15 | 320 | 100 | 100 | 100 | 100 | 100 |
| 1-16 | 320 | 100 | 100 | 100 | 100 | 100 |
| 1-17 | 320 | 100 | 100 | 100 | 100 | 100 |
| 1-18 | 320 | 100 | 100 | 100 | 100 | 100 |
| 1-25 | 320 | 100 | 100 | 100 | 100 | 100 |
| 1-26 | 320 | 100 | 100 | 100 | 100 | 100 |
| 1-27 | 320 | 100 | 100 | 100 | 100 | 100 |
| 1-28 | 320 | 100 | 100 | 100 | 100 | 100 |
| 1-29 | 320 | 100 | 100 | 100 | 100 | 100 |
| 1-30 | 320 | 100 | 100 | 90 | 100 | 100 |
| 1-37 | 320 | 100 | 100 | 100 | 100 | 100 |
| 1-38 | 320 | 100 | 100 | 90 | 100 | 100 |
| 1-39 | 320 | 90 | 100 | 90 | 100 | 100 |
| 1-40 | 320 | 100 | 90 | 100 | 100 | 100 |
| 1-41 | 320 | 100 | 100 | 100 | 100 | 100 |
| 1-42 | 320 | 100 | 100 | 100 | 100 | 100 |
| 1-49 | 320 | 100 | 100 | 90 | 100 | 100 |
| 1-50 | 320 | 100 | 100 | 100 | 100 | 100 |
| 1-51 | 320 | 100 | 100 | 100 | 100 | 100 |
| 1-52 | 320 | 100 | 100 | 90 | 100 | 100 |
| 1-53 | 320 | 100 | 100 | 100 | 100 | 100 |
| 1-54 | 320 | 100 | 100 | 100 | 100 | 100 |
| 1-61 | 320 | 100 | 100 | 100 | 100 | 100 |
| 1-62 | 320 | 100 | 100 | 100 | 100 | 100 |
| 1-63 | 320 | 100 | 100 | 100 | 100 | 100 |
| 1-64 | 320 | 100 | 100 | 100 | 100 | 100 |
| 1-65 | 320 | 100 | 100 | 90 | 100 | 100 |
| 1-74 | 320 | 100 | 100 | 100 | 100 | 100 |
| 1-75 | 320 | | | | 100 | 90 |
| 1-78 | 320 | 100 | 100 | 100 | 100 | 100 |
| 2-1 | 320 | 90 | 100 | | 100 | 100 |
| 2-2 | 320 | 100 | 100 | 90 | 100 | 100 |
| 2-3 | 320 | 90 | 100 | 100 | 100 | 100 |
| 2-4 | 320 | 100 | 100 | 100 | 100 | 100 |
| 2-5 | 320 | 100 | 90 | 80 | 100 | 100 |
| 2-6 | 320 | 100 | 100 | 100 | 100 | 100 |
| 2-13 | 320 | 100 | 100 | 100 | 100 | 100 |
| 2-14 | 320 | 100 | 100 | 100 | 100 | 100 |
| 2-15 | 320 | 100 | 100 | 100 | 100 | 100 |
| 2-16 | 320 | 100 | 100 | 100 | 100 | 100 |
| 2-17 | 320 | 100 | 100 | 100 | 100 | 100 |
| 2-18 | 320 | 100 | 100 | 90 | 100 | 100 |
| 2-25 | 320 | 100 | 100 | 100 | 100 | 100 |
| 2-26 | 320 | 100 | 100 | 90 | 100 | 100 |
| 2-27 | 320 | 100 | 100 | 100 | 100 | 100 |
| 2-28 | 320 | 100 | 100 | 100 | 100 | 100 |
| 2-29 | 320 | 100 | 100 | 90 | 100 | 100 |
| 2-30 | 320 | 100 | 100 | 90 | 100 | 100 |
| 2-37 | 320 | 90 | 100 | 80 | 100 | 100 |
| 2-38 | 320 | 100 | 100 | 100 | 100 | 100 |
| 2-39 | 320 | | 100 | 100 | 100 | 100 |
| 2-40 | 320 | | | | 100 | 100 |
| 2-41 | 320 | 100 | 100 | 90 | 100 | 100 |
| 2-42 | 320 | 90 | 90 | 90 | 100 | 100 |
| 2-49 | 320 | 100 | 100 | 90 | 100 | 100 |
| 2-50 | 320 | 100 | 100 | 100 | 100 | 100 |
| 2-51 | 320 | 90 | 100 | 90 | 100 | 100 |
| 2-62 | 320 | 100 | 100 | 100 | 100 | 100 |
| 2-63 | 320 | 100 | 100 | 90 | 100 | 100 |
| 2-64 | 320 | 100 | 100 | 100 | 100 | 100 |
| 2-61 | 320 | 100 | 100 | 100 | 100 | 100 |
| 2-78 | 320 | 100 | 100 | 100 | 100 | 100 |
| 3-1 | 320 | 100 | 100 | | 100 | 100 |
| 3-2 | 320 | 90 | 90 | 80 | 100 | 100 |
| 3-3 | 320 | 90 | 100 | 90 | 100 | 100 |
| 4-1 | 320 | | 80 | | 100 | 100 |
| 4-2 | 320 | | 80 | 80 | 100 | 100 |
| 4-3 | 320 | 90 | 80 | 100 | 100 | 100 |
| 4-6 | 320 | | | 100 | 90 | 100 |
| 4-25 | 320 | | | | | 90 |
| 4-28 | 320 | | | | | 100 |
| 4-29 | 320 | | | | | 100 |
| 4-30 | 320 | | | 80 | | 100 |
| 4-37 | 320 | | | | | 100 |
| 4-38 | 320 | | | | | 90 |
| 4-39 | 320 | | | | 100 | 100 |
| 4-40 | 320 | | | 100 | 80 | 100 |
| 4-41 | 320 | | | | 80 | 100 |
| 4-52 | 320 | 100 | 100 | 80 | 100 | 100 |
| 4-53 | 320 | 100 | 100 | 100 | 100 | 100 |
| 4-54 | 320 | 90 | 80 | 80 | 90 | 100 |
| 4-61 | 320 | 100 | 90 | 90 | 100 | 100 |
| 4-64 | 320 | 90 | 100 | | 100 | 100 |
| 4-75 | 320 | | | 80 | 90 | 100 |
| 5-1 | 320 | | | 80 | 100 | 100 |
| 6-4 | 320 | | 90 | | 100 | 90 |
| 6-5 | 320 | 90 | 90 | | 100 | 100 |
| 6-6 | 320 | 90 | 90 | 80 | 100 | 100 |
| 7-2 | 320 | 90 | 90 | 90 | 100 | 100 |
| 7-3 | 320 | 100 | 100 | 100 | 100 | 100 |
| 7-4 | 320 | 90 | | | 100 | 100 |
| 7-11 | 320 | 80 | 90 | 90 | 100 | 100 |
| 7-12 | 320 | 100 | 100 | 100 | 100 | 100 |
| 7-13 | 320 | 100 | 100 | 100 | 100 | 100 |
| 7-14 | 320 | 100 | 100 | 100 | 100 | 100 |
| 7-15 | 320 | 100 | 100 | 100 | 100 | 100 |
| 7-16 | 320 | 100 | 100 | 100 | 100 | 100 |
| 7-47 | 320 | 90 | 90 | 80 | 100 | 100 |
| 7-50 | 320 | 100 | 100 | 90 | 100 | 100 |
| 7-64 | 320 | 100 | 100 | 100 | 100 | 100 |
| 7-62 | 320 | 100 | 90 | 80 | 100 | 100 |
| 7-75 | 320 | 100 | 100 | 100 | 100 | 100 |

**Tabelle B: Vorauflauf**

| **Beispiel Nr.** | **Dosierung [g/ha]** | **AMARE** | **MATIN** | **STEME** | **VIOTR** |
|---|---|---|---|---|---|
| 1-1 | 320 | 100 | 100 | 100 | 100 |
| 1-2 | 320 | 100 | 100 | 90 | 100 |
| 1-3 | 320 | 100 | 100 | 100 | 100 |
| 1-4 | 320 | 100 | 100 | 100 | 100 |
| 1-5 | 320 | 100 | 100 | 90 | 100 |
| 1-6 | 320 | 100 | 100 | 100 | 100 |
| 1-13 | 320 | 100 | 100 | 100 | 100 |
| 1-14 | 320 | 100 | 100 | 100 | 100 |
| 1-15 | 320 | 100 | 100 | 100 | 100 |
| 1-16 | 320 | 100 | 100 | 100 | 100 |
| 1-17 | 320 | 100 | 100 | 100 | 100 |
| 1-18 | 320 | 100 | 100 | 100 | 100 |
| 1-25 | 320 | 100 | 100 | 100 | 100 |
| 1-26 | 320 | 100 | 100 | 100 | 100 |
| 1-27 | 320 | 100 | 100 | 100 | 100 |
| 1-28 | 320 | 100 | 100 | 100 | 100 |
| 1-29 | 320 | 100 | 100 | 100 | 100 |
| 1-30 | 320 | 100 | 100 | 100 | 100 |
| 1-37 | 320 | 100 | 100 | 100 | 100 |
| 1-38 | 320 | 100 | 100 | 100 | 100 |
| 1-39 | 320 | 100 | 100 | 100 | 100 |
| 1-40 | 320 | 100 | 100 | 100 | 100 |
| 1-41 | 320 | 100 | 100 | 100 | 100 |
| 1-42 | 320 | 100 | 100 | 100 | 100 |
| 1-49 | 320 | 100 | 100 | 90 | 100 |
| 1-50 | 320 | 100 | 100 | 90 | 100 |
| 1-51 | 320 | 100 | 100 | 100 | 100 |
| 1-52 | 320 | 100 | 100 | 100 | 100 |
| 1-53 | 320 | 100 | 100 | 100 | 100 |
| 1-54 | 320 | 100 | 100 | 100 | 100 |
| 1-61 | 320 | 100 | 100 | 100 | 100 |
| 1-62 | 320 | 100 | 100 | 100 | 100 |
| 1-63 | 320 | 100 | 100 | 100 | 100 |
| 1-64 | 320 | 100 | 100 | 100 | 100 |
| 1-65 | 320 | 100 | 100 | 100 | 100 |
| 1-74 | 320 | 100 | 100 | 100 | 100 |
| 1-75 | 320 | 90 | 90 | 100 | 90 |
| 1-78 | 320 | 100 | 100 | | 100 |
| 2-2 | 320 | 100 | 100 | | 100 |
| 2-1 | 320 | 100 | 100 | | 100 |
| 2-3 | 320 | 100 | 100 | 90 | 100 |
| 2-4 | 320 | 100 | 100 | 90 | 100 |
| 2-5 | 320 | 100 | 100 | 90 | 100 |
| 2-6 | 320 | 100 | 100 | 100 | 100 |
| 2-13 | 320 | 100 | 100 | 100 | 100 |
| 2-14 | 320 | 100 | 100 | 100 | 100 |
| 2-15 | 320 | 100 | 100 | 100 | 100 |
| 2-16 | 320 | 100 | 100 | 100 | 100 |
| 2-17 | 320 | 100 | 100 | 100 | 100 |
| 2-18 | 320 | 100 | 100 | 100 | 100 |
| 2-25 | 320 | 100 | 100 | 100 | 100 |
| 2-26 | 320 | 100 | 100 | 100 | 100 |
| 2-27 | 320 | 100 | 100 | 100 | 100 |
| 2-28 | 320 | 100 | 100 | 100 | 100 |
| 2-29 | 320 | 100 | 100 | 100 | 100 |
| 2-30 | 320 | 100 | 100 | 100 | 100 |
| 2-37 | 320 | 100 | 100 | 100 | 100 |
| 2-38 | 320 | 100 | 100 | 100 | 100 |
| 2-39 | 320 | 100 | 100 | 100 | 100 |
| 2-40 | 320 | 100 | 100 | 100 | 100 |
| 2-41 | 320 | 100 | 100 | 100 | 100 |
| 2-42 | 320 | 100 | 100 | 90 | 100 |
| 2-49 | 320 | 100 | 100 | 90 | 100 |
| 2-50 | 320 | 100 | 100 | 100 | 100 |
| 2-51 | 320 | 100 | 100 | 90 | 100 |
| 2-62 | 320 | 100 | 100 | 100 | 100 |
| 2-63 | 320 | 100 | 100 | 100 | 100 |
| 2-64 | 320 | 100 | 100 | 100 | 100 |
| 2-61 | 320 | 100 | 100 | 100 | 100 |
| 2-78 | 320 | 100 | 100 | 100 | 100 |
| 3-1 | 320 | 100 | 100 | 100 | 100 |
| 3-2 | 320 | 100 | 100 | 90 | 100 |
| 3-3 | 320 | 100 | 100 | | 100 |
| 4-1 | 320 | 100 | 90 | 90 | 100 |
| 4-2 | 320 | 100 | 100 | 100 | 90 |
| 4-3 | 320 | 100 | 100 | 100 | 100 |
| 4-4 | 320 | 100 | 100 | 100 | 100 |
| 4-5 | 320 | 100 | 90 | | 100 |
| 4-6 | 320 | 100 | 100 | 90 | 100 |
| 4-25 | 320 | 90 | 90 | 90 | 100 |
| 4-28 | 320 | 90 | 90 | 90 | 100 |
| 4-29 | 320 | 90 | 90 | 90 | 100 |
| 4-30 | 320 | 90 | 100 | 90 | 90 |
| 4-37 | 320 | 100 | 100 | 100 | 90 |
| 4-39 | 320 | 100 | 90 | 100 | 100 |
| 4-40 | 320 | 100 | 100 | 100 | 100 |
| 4-41 | 320 | 100 | | 80 | 90 |
| 4-52 | 320 | 100 | 100 | 100 | 100 |
| 4-53 | 320 | 100 | 100 | 100 | 100 |
| 4-54 | 320 | 100 | 100 | 100 | 100 |
| 4-61 | 320 | 100 | 100 | 90 | 100 |
| 4-64 | 320 | 100 | 100 | 100 | 100 |
| 4-75 | 320 | 100 | 100 | 100 | 90 |
| 5-1 | 320 | 100 | 100 | 90 | 100 |
| 6-4 | 320 | 100 | 90 | 90 | 100 |
| 6-5 | 320 | 100 | 90 | 90 | 100 |
| 6-6 | 320 | 90 | 100 | 90 | 100 |
| 7-1 | 320 | 100 | 80 | 80 | 100 |
| 7-2 | 320 | 100 | 90 | 80 | 100 |
| 7-3 | 320 | 100 | 100 | 100 | 100 |
| 7-4 | 320 | 100 | 80 | 90 | 100 |
| 7-11 | 320 | 100 | | 80 | 100 |
| 7-12 | 320 | 100 | 100 | 100 | 100 |
| 7-13 | 320 | 100 | 100 | 100 | 100 |
| 7-14 | 320 | 100 | 90 | 100 | 100 |
| 7-15 | 320 | 100 | 100 | 100 | 100 |
| 7-16 | 320 | 100 | 100 | 100 | 100 |
| 7-47 | 320 | 100 | 100 | 90 | 100 |
| 7-50 | 320 | 90 | 90 | 100 | 100 |
| 7-62 | 320 | 100 | 90 | 100 | 100 |
| 7-64 | 320 | | 100 | 100 | 100 |
| 7-75 | 320 | 100 | 100 | 100 | 100 |

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigten zahlreiche erfindungsgemäße Verbindungen bei einer Aufwandmenge von 80 g oder weniger pro Hektar eine mindestens 80%-ige Wirkung gegen eine Vielzahl bedeutender Schadpflanzen. Gleichzeitig lassen erfindungsgemäße Verbindungen Gramineenkulturen wie Gerste, Weizen, Roggen, Hirse, Mais oder Reis im Nachauflaufverfahren selbst bei hohen Wirkstoffdosierungen praktisch ungeschädigt. Einige Substanzen schonen darüber hinaus auch zweikeimblättrige Kulturen wie Soja, Baumwolle, Raps, Zuckerrüben oder Kartoffeln. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen. Die Daten nachfolgender Tabellen C und D zeigen beispielhaft die herbizide Wirkung der erfindungsgemäßen Verbindungen im Vorauflauf, wobei die herbizide Wirkung in Prozent angegeben ist.

**Tabelle C: Nachauflauf**

| **Beispiel Nr.** | **Dosierung [g/ha]** | **ALOMY** | **AVEFA** | **ECHCG** | **SETVI** |
|---|---|---|---|---|---|
| 1-1 | 80 | 90 | 100 | 100 | 100 |
| 1-2 | 80 | 90 | 100 | 90 | 90 |
| 1-3 | 80 | 90 | 100 | 90 | 90 |
| 1-4 | 80 | 90 | 100 | 100 | 100 |
| 1-5 | 80 | 100 | 100 | 100 | 100 |
| 1-6 | 80 | 100 | 100 | 100 | 100 |
| 1-13 | 80 | 90 | 100 | 90 | 90 |
| 1-14 | 80 | 90 | 90 | 100 | 100 |
| 1-15 | 80 | 90 | 100 | 90 | 80 |
| 1-16 | 80 | 90 | 90 | 90 | 90 |
| 1-17 | 80 | 90 | 90 | 90 | 90 |
| 1-18 | 80 | 90 | 100 | 90 | 100 |
| 1-25 | 80 | 100 | 100 | 100 | 100 |
| 1-26 | 80 | 90 | 100 | 100 | 100 |
| 1-27 | 80 | 100 | 100 | 100 | 100 |
| 1-28 | 80 | 90 | 100 | 100 | 100 |
| 1-29 | 80 | 100 | 90 | 100 | 100 |
| 1-30 | 80 | 100 | 100 | 100 | 100 |
| 1-37 | 80 | 80 | 100 | 100 | 100 |
| 1-38 | 80 | 90 | 90 | 100 | 100 |
| 1-39 | 80 | 90 | 80 | 90 | 100 |
| 1-40 | 80 | 90 | 80 | 100 | 100 |
| 1-41 | 80 | 90 | 100 | 90 | 90 |
| 1-42 | 80 | | | 80 | 90 |
| 1-49 | 80 | 90 | 100 | 80 | 90 |
| 1-50 | 80 | 100 | 100 | 100 | 100 |
| 1-51 | 80 | 100 | 100 | 100 | 100 |
| 1-52 | 80 | 100 | 100 | 90 | 90 |
| 1-53 | 80 | 100 | 100 | 90 | 100 |
| 1-54 | 80 | 90 | 100 | 90 | 100 |
| 1-61 | 80 | 90 | 100 | 90 | 90 |
| 1-62 | 80 | 100 | 100 | 100 | 100 |
| 1-63 | 80 | 90 | 100 | 90 | 100 |
| 1-64 | 80 | 90 | 100 | 90 | 90 |
| 1-65 | 80 | 100 | 100 | 100 | 100 |
| 1-74 | 80 | 90 | 90 | 90 | 100 |
| 1-78 | 80 | 90 | 80 | 100 | 100 |
| 2-1 | 80 | 90 | 100 | 90 | 90 |
| 2-2 | 80 | 90 | 90 | 90 | 100 |
| 2-3 | 80 | 80 | 90 | 90 | 100 |
| 2-4 | 80 | 90 | 100 | 90 | 90 |
| 2-5 | 80 | 90 | 100 | 100 | 100 |
| 2-6 | 80 | 80 | 100 | 90 | 90 |
| 2-13 | 80 | 80 | 90 | 90 | 100 |
| 2-14 | 80 | | 90 | 100 | 100 |
| 2-15 | 80 | 80 | 100 | 90 | 100 |
| 2-16 | 80 | 90 | 90 | 90 | 90 |
| 2-17 | 80 | 90 | 100 | 90 | 90 |
| 2-18 | 80 | 80 | 100 | 90 | 90 |
| 2-25 | 80 | 100 | 100 | 100 | 100 |
| 2-26 | 80 | 90 | 100 | 100 | 100 |
| 2-27 | 80 | 90 | 100 | 100 | 100 |
| 2-28 | 80 | 90 | 100 | 100 | 100 |
| 2-29 | 80 | 100 | 90 | 100 | 100 |
| 2-30 | 80 | 90 | 90 | 100 | 100 |
| 2-37 | 80 | 80 | 90 | 100 | 100 |
| 2-38 | 80 | 90 | 90 | 100 | 100 |
| 2-39 | 80 | | 80 | 90 | 100 |
| 2-40 | 80 | 80 | | 100 | 100 |
| 2-41 | 80 | 80 | 90 | 100 | 100 |
| 2-42 | 80 | | | 100 | 100 |
| 2-49 | 80 | 100 | 100 | 100 | 100 |
| 2-50 | 80 | | 100 | 90 | 100 |
| 2-51 | 80 | | 90 | 90 | 90 |
| 2-61 | 80 | 90 | 90 | 90 | 90 |
| 2-62 | 80 | 90 | 100 | 100 | 100 |
| 2-63 | 80 | 100 | 100 | 100 | 100 |
| 2-64 | 80 | 100 | 100 | 100 | 100 |
| 2-78 | 80 | 80 | 90 | 90 | 90 |
| 3-1 | 80 | 90 | 100 | 100 | 100 |
| 3-2 | 80 | 90 | 90 | 90 | 100 |
| 3-3 | 80 | 90 | 90 | 90 | 90 |
| 4-1 | 80 | 80 | | 90 | 100 |
| 4-2 | 80 | | 80 | 100 | 80 |
| 4-3 | 80 | | 90 | 100 | 100 |
| 4-5 | 80 | | | 90 | 90 |
| 4-4 | 80 | | | 100 | 90 |
| 4-6 | 80 | | | 100 | 100 |
| 4-37 | 80 | | | 80 | 90 |
| 4-38 | 80 | | | 80 | |
| 4-40 | 80 | | | 90 | 90 |
| 4-41 | 80 | | | 80 | |
| 4-52 | 80 | 90 | 100 | 100 | 100 |
| 4-53 | 80 | 90 | 100 | 90 | 100 |
| 4-54 | 80 | 90 | 100 | 100 | 100 |
| 4-61 | 80 | 100 | 100 | 100 | 100 |
| 4-64 | 80 | 100 | 100 | 100 | 100 |
| 6-4 | 80 | | | 100 | 100 |
| 6-5 | 80 | | | 100 | 90 |
| 6-6 | 80 | 80 | 80 | 90 | 90 |
| 7-1 | 80 | 90 | | 100 | 100 |
| 7-2 | 80 | | 90 | 90 | 100 |
| 7-3 | 80 | 100 | 100 | 90 | 100 |
| 7-4 | 80 | 90 | 90 | 100 | 100 |
| 7-11 | 80 | 90 | | 90 | 90 |
| 7-12 | 80 | 90 | 90 | 100 | 90 |
| 7-13 | 80 | 90 | 100 | 100 | 100 |
| 7-14 | 80 | 90 | 100 | 90 | 90 |
| 7-15 | 80 | 90 | 100 | 90 | 100 |
| 7-16 | 80 | 90 | 90 | 90 | 100 |
| 7-47 | 80 | 80 | 100 | 100 | 100 |
| 7-50 | 80 | 90 | 90 | 100 | 100 |
| 7-62 | 80 | 90 | 100 | 100 | 100 |
| 7-64 | 80 | 90 | 100 | 100 | 100 |
| 7-75 | 80 | 80 | 100 | 100 | 90 |

**Tabelle D: Nachauflauf**

| **Beispiel Nr.** | **Dosierung [g/ha]** | **ABUTH** | **AMARE** | **PHBPU** | **STEME** | **VIOTR** |
|---|---|---|---|---|---|---|
| 1-1 | 80 | 100 | 100 | 90 | 100 | 100 |
| 1-2 | 80 | 80 | 80 | 90 | 100 | 100 |
| 1-3 | 80 | 80 | | 100 | 100 | 100 |
| 1-4 | 80 | 100 | 100 | 100 | 100 | 100 |
| 1-5 | 80 | 90 | 100 | 100 | 100 | 100 |
| 1-6 | 80 | 90 | 100 | 100 | 100 | 100 |
| 1-13 | 80 | 100 | 100 | 100 | 100 | 100 |
| 1-14 | 80 | 100 | 90 | 90 | 100 | 90 |
| 1-15 | 80 | 90 | 100 | 90 | 100 | 100 |
| 1-16 | 80 | 100 | 100 | | 100 | 100 |
| 1-17 | 80 | 100 | 100 | 90 | 100 | 100 |
| 1-18 | 80 | 100 | 100 | 90 | 100 | 100 |
| 1-25 | 80 | 100 | 100 | 100 | 100 | 100 |
| 1-26 | 80 | 100 | 100 | 90 | 100 | 100 |
| 1-27 | 80 | 100 | 100 | 90 | 100 | 100 |
| 1-28 | 80 | 100 | 100 | 100 | 100 | 100 |
| 1-29 | 80 | 100 | 100 | 100 | 100 | 100 |
| 1-30 | 80 | 100 | 90 | 90 | 100 | 100 |
| 1-37 | 80 | 90 | 100 | 90 | 100 | 100 |
| 1-38 | 80 | 100 | 90 | 90 | 100 | 100 |
| 1-39 | 80 | 90 | 100 | 100 | 100 | 100 |
| 1-40 | 80 | 100 | 100 | 100 | 90 | 90 |
| 1-41 | 80 | 100 | 100 | 90 | 100 | 100 |
| 1-42 | 80 | 90 | 100 | 100 | 100 | 100 |
| 1-49 | 80 | 90 | 80 | 90 | 100 | 100 |
| 1-50 | 80 | 100 | 100 | 100 | 100 | 100 |
| 1-51 | 80 | 100 | 100 | 100 | 100 | 100 |
| 1-52 | 80 | 100 | 100 | 100 | 100 | 100 |
| 1-53 | 80 | 100 | 100 | 100 | 100 | 100 |
| 1-54 | 80 | 100 | 100 | 100 | 100 | 100 |
| 1-61 | 80 | 100 | 90 | 100 | 100 | 100 |
| 1-62 | 80 | 100 | 100 | 100 | 100 | 100 |
| 1-63 | 80 | 100 | 100 | 100 | 100 | 100 |
| 1-64 | 80 | 100 | 100 | 100 | 100 | 100 |
| 1-65 | 80 | 100 | 100 | 100 | 100 | 100 |
| 1-74 | 80 | 100 | 100 | 100 | 100 | 100 |
| 1-75 | 80 | | 90 | 80 | 100 | |
| 1-78 | 80 | 80 | 100 | 100 | 100 | 90 |
| 2-1 | 80 | 90 | 100 | 100 | 100 | 100 |
| 2-2 | 80 | 90 | 80 | 90 | 100 | 100 |
| 2-3 | 80 | 80 | 80 | 100 | 100 | 100 |
| 2-4 | 80 | | | 100 | 100 | 100 |
| 2-5 | 80 | 100 | 100 | 90 | 100 | 100 |
| 2-6 | 80 | 100 | 90 | 100 | 100 | 100 |
| 2-13 | 80 | 100 | 100 | 100 | 100 | 100 |
| 2-14 | 80 | 100 | 100 | 90 | 100 | 100 |
| 2-15 | 80 | 100 | 100 | 80 | 100 | 100 |
| 2-16 | 80 | 100 | 100 | 100 | 100 | 100 |
| 2-17 | 80 | 100 | 100 | 100 | 100 | 100 |
| 2-18 | 80 | 100 | 100 | 100 | 100 | 100 |
| 2-25 | 80 | 100 | 100 | 100 | 100 | 100 |
| 2-26 | 80 | 90 | 100 | 90 | 100 | 100 |
| 2-27 | 80 | 100 | 100 | 90 | 100 | 100 |
| 2-28 | 80 | 100 | 100 | 100 | 100 | 100 |
| 2-29 | 80 | 100 | 100 | 90 | 100 | 100 |
| 2-30 | 80 | 100 | 100 | 90 | 100 | 100 |
| 2-37 | 80 | 100 | 100 | 100 | 100 | 90 |
| 2-38 | 80 | 90 | 100 | 90 | 100 | 100 |
| 2-39 | 80 | 100 | 90 | 90 | 100 | 100 |
| 2-40 | 80 | 100 | 100 | 90 | 100 | 100 |
| 2-41 | 80 | 100 | 90 | 90 | 100 | 100 |
| 2-42 | 80 | 100 | 90 | 90 | 100 | 100 |
| 2-49 | 80 | 100 | 100 | 100 | 100 | 100 |
| 2-50 | 80 | 100 | 100 | 100 | 100 | 100 |
| 2-51 | 80 | 100 | 100 | 90 | 100 | 100 |
| 2-61 | 80 | 100 | 90 | 100 | 100 | 100 |
| 2-62 | 80 | 100 | 100 | 100 | 100 | 100 |
| 2-63 | 80 | 100 | 100 | 100 | 100 | 100 |
| 2-64 | 80 | 100 | 100 | 100 | 100 | 100 |
| 2-78 | 80 | 80 | 100 | 90 | 100 | 90 |
| 3-1 | 80 | 100 | 100 | 90 | 100 | 100 |
| 3-2 | 80 | 80 | | 90 | 100 | 100 |
| 3-3 | 80 | 90 | 80 | 90 | 90 | 100 |
| 4-1 | 80 | 100 | 100 | 100 | 100 | 100 |
| 4-2 | 80 | 90 | 100 | 90 | 90 | 100 |
| 4-3 | 80 | 100 | 100 | 100 | 90 | 100 |
| 4-5 | 80 | 80 | 90 | 90 | 90 | 100 |
| 4-4 | 80 | 80 | 100 | 80 | 90 | 100 |
| 4-6 | 80 | 100 | 100 | 80 | 100 | 100 |
| 4-25 | 80 | | 90 | 80 | 100 | 90 |
| 4-28 | 80 | | 80 | 90 | 100 | 80 |
| 4-29 | 80 | 80 | 80 | 90 | 90 | 90 |
| 4-30 | 80 | 80 | | 90 | 90 | 80 |
| 4-37 | 80 | 80 | 100 | | 80 | 80 |
| 4-38 | 80 | 90 | 90 | 90 | 90 | 100 |
| 4-39 | 80 | 90 | 90 | 90 | 100 | 90 |
| 4-40 | 80 | 80 | 100 | | 90 | 80 |
| 4-41 | 80 | 100 | 90 | 90 | 90 | 90 |
| 4-52 | 80 | 90 | 100 | 100 | 90 | 90 |
| 4-53 | 80 | 100 | 100 | 90 | 90 | 100 |
| 4-54 | 80 | 90 | 90 | 100 | 100 | 100 |
| 4-61 | 80 | 100 | 100 | 100 | 100 | 100 |
| 4-64 | 80 | 100 | 100 | 100 | 100 | 100 |
| 4-75 | 80 | | 100 | 80 | 90 | 80 |
| 5-1 | 80 | 100 | 80 | 90 | 100 | 100 |
| 6-4 | 80 | 90 | | 80 | 90 | 100 |
| 6-5 | 80 | 80 | | | 90 | 100 |
| 6-6 | 80 | 80 | 80 | 100 | 100 | 100 |
| 7-1 | 80 | 100 | 100 | 90 | 90 | 100 |
| 7-2 | 80 | 100 | 100 | 90 | 100 | 100 |
| 7-3 | 80 | 100 | 90 | 90 | 100 | 100 |
| 7-4 | 80 | | 100 | | 90 | 100 |
| 7-11 | 80 | 90 | 90 | 90 | 90 | 100 |
| 7-12 | 80 | 100 | 100 | 90 | 100 | 100 |
| 7-13 | 80 | 100 | 100 | 100 | 100 | 100 |
| 7-14 | 80 | 100 | 100 | 90 | 100 | 100 |
| 7-15 | 80 | 100 | 100 | 90 | 100 | 100 |
| 7-16 | 80 | 100 | 100 | 90 | 100 | 100 |
| 7-47 | 80 | 90 | 90 | 90 | 100 | 100 |
| 7-50 | 80 | 100 | 90 | | 90 | 100 |
| 7-62 | 80 | 100 | 100 | 90 | 100 | 100 |
| 7-64 | 80 | 100 | 100 | 100 | 90 | 90 |
| 7-75 | 80 | 90 | 100 | 80 | 80 | 100 |

### 3. Vergleichsversuche

Zu Vergleichszwecken wurde die herbizide Wirkung zahlreicher erfindungsgemäßer Verbindungen mit den strukturell ähnlichsten aus den Dokumenten WO 2011/035874, WO 2012/028579 und WO 2012/126932 bekannten Verbindungen im Vor- und Nachauflauf geprüft. Diese Daten sind in nachfolgenden Tabellen E bis M aufgeführt, wobei in jedem Vergleichspärchen die jeweils obere Verbindung die erfindungsgemäße und die jeweils darunter stehende die aus dem Stand der Technik bekannte Verbindung ist.

**Tabelle E: Vergleich mit aus WO 2011035874 bekannten Verbindungen im Nachauflauf**

| Beispiel- Nr. | Dosierung [g / ha] | Herbizide Wirkung gegen | | | | | | |
|---|---|---|---|---|---|---|---|---|
| | | ABUTH | AMARE | MATIN | PHBPU | STEME | VIOTR | VERPE |
| 5-1 | 80 | 100 | 80 | 40 | 90 | 100 | 100 | 100 |
| 1-81 | 80 | 30 | 60 | 10 | 10 | 0 | 0 | 30 |

**Tabelle F: Vergleich mit aus WO2011035874 bekannten Verbindungen im Nachauflauf**

| Beispiel-Nr. | Dosierung [g / ha] | Herbizide Wirkung gegen | | | | |
|---|---|---|---|---|---|---|
| | | ABUTH | MATIN | PHBPU | STEME | VIOTR |
| 5-1 | 80 | 100 | | | 100 | |
| 1-51 | 80 | 80 | | | 40 | |
| | | | | | | |
| 5-1 | 80 | | 40 | 90 | 100 | 100 |
| 1-77 | 80 | | 0 | 40 | 80 | 60 |

**Tabelle G: Vergleich mit aus WO 2011035874 bekannten Verbindungen im Vorauflauf**

| Beispiel- Nr. | Dosierung [g / ha] | Herbizide Wirkung gegen | | | | | |
|---|---|---|---|---|---|---|---|
| | | ALOMY | AVEFA | CYPES | ECHCG | SETVI | ABUTH |
| 5-1 | 80 | 30 | 30 | 50 | 80 | 80 | 100 |
| 1-51 | 80 | 10 | 0 | 0 | 30 | 60 | 100 |
| | | | | | | | |
| 5-1 | 80 | 30 | 30 | 50 | 80 | 80 | 100 |
| 1-81 | 80 | 0 | 40 | 0 | 10 | 10 | 20 |
| | | | | | | | |
| 5-1 | 320 | 40 | 60 | 80 | 100 | 100 | 100 |
| 1-77 | 320 | 10 | 0 | 30 | 70 | 20 | 100 |

**Tabelle H: Vergleich mit aus WO 2011035874 bekannten Verbindungen im Vorauflauf**

| Beispiel-Nr. | Dosierung [g / ha] | Herbizide Wirkung gegen | | | | |
|---|---|---|---|---|---|---|
| | | AMARE | POLCO | STEME | VIOTR | VERPE |
| 5-1 | 80 | 100 | 80 | 80 | 100 | 100 |
| 1-51 | 80 | 100 | 0 | 90 | 60 | 100 |
| | | | | | | |
| 5-1 | 80 | 100 | 80 | 80 | 100 | 100 |
| 1-81 | 80 | 60 | 10 | 50 | 90 | 70 |
| | | | | | | |
| 5-1 | 320 | 100 | 100 | 90 | 100 | 100 |
| 1-77 | 320 | 100 | 30 | 80 | 60 | 90 |

**Tabelle I: Vergleich mit aus WO 2012/028579 bekannten Verbindungen im Nachauflauf**

| Beispiel- Nr. | Dosierung [g / ha] | Herbizide Wirkung gegen | | | | | |
|---|---|---|---|---|---|---|---|
| | | ALOMY | AVEFA | CYPES | SETVI | STEME | VIOTR |
| 1-1 | 80 | 90 | 100 | 80 | 100 | 100 | 100 |
| 4-803 | 80 | 20 | 0 | 60 | 100 | 100 | 100 |
| | | | | | | | |
| 1-2 | 5 | 60 | 70 | 50 | 90 | 90 | 100 |
| 4-804 | 5 | 0 | 0 | 0 | 10 | 10 | 20 |
| | | | | | | | |
| 1-3 | 80 | 90 | 100 | 80 | 90 | 100 | 100 |
| 4-805 | 80 | 0 | 0 | 0 | 0 | 0 | 0 |
| | | | | | | | |
| 1-1 | 20 | 90 | 90 | 40 | 100 | 100 | 100 |
| 4-812 | 20 | 30 | 50 | 40 | 100 | 100 | 90 |
| | | | | | | | |
| 1-2 | 5 | 60 | 70 | | | | 100 |
| 4-813 | 5 | 30 | 40 | | | | 100 |
| | | | | | | | |
| 1-3 | 5 | 60 | 70 | 60 | 90 | 90 | 100 |
| 4-814 | 5 | 0 | 30 | 20 | 80 | 90 | 100 |
| | | | | | | | |
| 1-49 | 5 | 60 | 50 | 50 | 80 | | 100 |
| 4-803 | 5 | 0 | 0 | 20 | 0 | | 90 |
| | | | | | | | |
| 1-51 | 20 | 90 | 90 | 90 | 100 | 100 | 100 |
| 4-805 | 20 | 0 | 0 | 0 | 0 | 0 | 0 |
| | | | | | | | |
| 1-49 | 5 | 60 | 50 | 50 | | | 100 |
| 4-812 | 5 | 10 | 20 | 20 | | | 70 |
| | | | | | | | |
| 1-51 | 5 | 40 | 60 | 80 | | 100 | 100 |
| 4-814 | 5 | 0 | 30 | 20 | | 90 | 100 |
| | | | | | | | |
| 1-2 | 5 | 60 | 70 | 50 | 90 | 90 | 100 |
| 4-136 | 5 | 40 | 80 | 80 | 90 | 100 | 80 |
| | | | | | | | |
| 1-2 | 5 | 60 | 70 | 50 | 90 | 90 | 100 |
| 4-175 | 5 | 0 | 0 | 30 | 40 | 80 | 70 |
| | | | | | | | |
| 1-3 | 5 | 60 | 70 | | | | 100 |
| 4-176 | 5 | 30 | 10 | | | | 100 |
| | | | | | | | |
| 1-1 | 20 | 90 | 90 | 40 | 100 | 100 | 100 |
| 4-78 | 20 | 20 | 80 | 40 | 80 | 80 | 60 |
| | | | | | | | |
| 1-2 | 20 | 80 | 90 | 70 | 90 | 90 | 100 |
| 4-79 | 20 | 20 | 40 | 10 | 90 | 40 | 40 |
| | | | | | | | |
| 1-3 | 5 | 60 | | 60 | 90 | 90 | 100 |
| 4-80 | 5 | 10 | | 20 | 20 | 90 | 60 |
| | | | | | | | |
| 1-1 | 5 | 60 | | | 100 | 100 | 100 |
| 4-108 | 5 | 60 | | | 90 | 70 | 90 |
| | | | | | | | |
| 1-2 | 5 | 60 | 70 | 50 | 90 | 90 | |
| 4-109 | 5 | 20 | 40 | 40 | 90 | 90 | |
| | | | | | | | |
| 1-3 | 5 | 60 | 70 | | 90 | 90 | 100 |
| 4-110 | 5 | 20 | 20 | | 90 | 90 | 100 |
| | | | | | | | |
| 1-1 | 5 | 60 | 40 | | 100 | 100 | 100 |
| 4-128 | 5 | 20 | 0 | | 80 | 60 | 100 |
| | | | | | | | |
| 1-2 | 5 | 60 | 70 | 50 | 90 | 90 | 100 |
| 4-129 | 5 | 20 | 0 | 50 | 80 | 70 | 80 |
| | | | | | | | |
| 1-3 | 5 | 60 | 70 | 60 | 90 | 90 | 100 |
| 4-130 | 5 | 20 | 0 | 10 | 60 | 100 | 90 |
| | | | | | | | |
| 1-1 | 5 | 60 | 40 | 20 | 100 | 100 | 100 |
| 4-122 | 5 | 80 | 40 | 20 | 40 | 90 | 100 |
| | | | | | | | |
| 1-2 | 5 | 60 | 70 | 50 | | 90 | 100 |
| 4-123 | 5 | 30 | 10 | 20 | | 90 | 100 |
| | | | | | | | |
| 1-3 | 5 | 60 | 70 | 60 | 90 | 90 | 100 |
| 4-124 | 5 | 20 | 60 | 60 | 90 | 70 | 100 |
| | | | | | | | |
| 1-1 | 5 | 60 | 40 | | 100 | 100 | 100 |
| 4-406 | 5 | 60 | 0 | | 80 | 100 | 100 |
| | | | | | | | |
| 1-49 | 5 | 60 | | 50 | 80 | 70 | 100 |
| 4-229 | 5 | 10 | | 10 | 90 | 40 | 40 |
| | | | | | | | |
| 1-51 | 5 | 40 | | 80 | 90 | 100 | 100 |
| 4-231 | 5 | 0 | | 50 | 90 | 90 | 60 |
| | | | | | | | |
| 1-49 | 5 | 60 | 50 | | 80 | | 100 |
| 4-292 | 5 | 10 | 0 | | 40 | | 70 |
| | | | | | | | |
| 1-51 | 5 | 40 | 60 | 80 | | 100 | 100 |
| 4-756 | 5 | 40 | 0 | 10 | | 100 | 90 |
| | | | | | | | |
| 1-49 | 5 | 60 | 50 | 50 | | | 100 |
| 4-245 | 5 | 30 | 20 | 30 | | | 90 |
| | | | | | | | |
| 1-51 | 80 | 100 | 100 | 100 | 100 | 100 | 100 |
| 4-634 | 80 | 80 | 80 | 100 | 100 | 100 | 100 |
| | | | | | | | |
| 1-51 | 20 | 90 | 90 | 90 | 100 | 100 | 100 |
| 4-640 | 20 | 50 | 70 | 60 | 100 | 100 | 100 |
| | | | | | | | |
| 4-1 | 20 | 70 | 20 | 0 | 60 | 80 | 100 |
| 1-573 | 20 | 0 | 0 | 10 | 10 | 10 | 70 |
| | | | | | | | |
| 4-3 | 20 | 30 | 50 | 70 | 40 | 60 | 100 |
| 1-574 | 20 | 0 | 0 | 0 | 20 | 50 | 80 |
| | | | | | | | |
| 4-3 | 20 | | 50 | 70 | 40 | 60 | 100 |
| 1-90 | 20 | | 0 | 0 | 0 | 10 | 0 |
| | | | | | | | |
| 4-1 | 20 | 70 | 20 | 0 | 60 | 80 | 100 |
| 1-119 | 20 | 0 | 0 | 0 | 10 | 40 | 60 |
| | | | | | | | |
| 4-3 | 20 | | 50 | 70 | 40 | | 100 |
| 1-121 | 20 | | 0 | 40 | 40 | | 40 |
| | | | | | | | |
| 4-1 | 20 | 70 | 20 | 0 | 60 | 80 | 100 |
| 1-387 | 20 | 10 | 0 | 0 | 0 | 60 | 60 |
| | | | | | | | |
| 4-1 | 20 | 70 | 20 | 0 | 60 | 80 | 100 |
| 1-139 | 20 | 0 | 0 | 0 | 0 | 40 | 80 |
| | | | | | | | |

**Tabelle J: Vergleich mit aus WO 2012/028579 bekannten Verbindungen im Vorauflauf**

| Beispiel-Nr. | Dosierung [g / ha] | Herbizide Wirkung gegen | | | | |
|---|---|---|---|---|---|---|
| | | ALOMY | AVEFA | CYPES | ECHCG | SETVI |
| 1-1 | 20 | 90 | | 30 | 80 | 70 |
| 4-803 | 20 | 0 | | 0 | 0 | 0 |
| | | | | | | |
| 1-2 | 320 | 100 | 100 | 90 | 100 | 100 |
| 4-804 | 320 | 0 | 10 | 10 | 0 | 0 |
| | | | | | | |
| 1-2 | 80 | 90 | 70 | 80 | 100 | 90 |
| 4-804 | 80 | 0 | 0 | 10 | 0 | 0 |
| | | | | | | |
| 1-3 | 320 | 100 | 100 | 90 | 100 | 100 |
| 4-805 | 320 | 0 | 0 | 0 | 0 | 0 |
| | | | | | | |
| 1-3 | 80 | 90 | 100 | 90 | 100 | 100 |
| 4-805 | 80 | 0 | 0 | 0 | 0 | 0 |
| | | | | | | |
| 1-1 | 20 | 90 | | 30 | | 70 |
| 4-812 | 20 | 50 | | 0 | | 40 |
| | | | | | | |
| 1-3 | 80 | 90 | 100 | 90 | | 100 |
| 4-814 | 80 | 40 | 20 | 20 | | 20 |
| | | | | | | |
| 1-3 | 20 | 50 | 80 | 30 | | 70 |
| 4-814 | 20 | 20 | 10 | 10 | | 10 |
| | | | | | | |
| 1-49 | 20 | 40 | 50 | 50 | 50 | 90 |
| 4-803 | 20 | 0 | 0 | 0 | 0 | 0 |
| | | | | | | |
| 1-50 | 320 | 100 | 100 | 100 | 100 | 100 |
| 4-804 | 320 | 0 | 10 | 10 | 0 | 0 |
| | | | | | | |
| 1-50 | 80 | 100 | 90 | 100 | 100 | 100 |
| 4-804 | 80 | 0 | 0 | 10 | 0 | 0 |
| | | | | | | |
| 1-51 | 320 | 100 | 100 | 100 | 100 | 100 |
| 4-805 | 320 | 0 | 0 | 0 | 0 | 0 |
| | | | | | | |
| 1-51 | 80 | 100 | 90 | 90 | 100 | 100 |
| 4-805 | 80 | 0 | 0 | 0 | 0 | 0 |
| | | | | | | |
| 1-50 | 20 | 90 | | | | 100 |
| 4-813 | 20 | 0 | | | | 10 |
| | | | | | | |
| 1-51 | 80 | 100 | 90 | 90 | | 100 |
| 4-814 | 80 | 40 | 20 | 20 | | 20 |
| | | | | | | |
| 1-51 | 20 | 80 | | 90 | | 100 |
| 4-814 | 20 | 20 | | 10 | | 10 |
| | | | | | | |
| 1-1 | 20 | 90 | | 30 | | |
| 4-135 | 20 | 60 | | 0 | | |
| | | | | | | |
| 1-3 | 20 | | 80 | | 90 | |
| 4-137 | 20 | | 0 | | 20 | |
| | | | | | | |
| 1-1 | 20 | 90 | | 30 | 80 | 70 |
| 4-174 | 20 | 0 | | 10 | 0 | 0 |
| | | | | | | |
| 1-2 | 20 | 70 | 20 | 60 | 40 | |
| 4-175 | 20 | 20 | 0 | 0 | 0 | |
| | | | | | | |
| 1-3 | 20 | 50 | 80 | | 90 | 70 |
| 4-176 | 20 | 20 | 20 | | 20 | 40 |
| | | | | | | |
| 1-1 | 80 | 90 | 90 | 70 | 100 | 100 |
| 4-78 | 80 | 10 | 30 | 10 | 0 | 70 |
| | | | | | | |
| 1-2 | 80 | 90 | 70 | 80 | 100 | 90 |
| 4-79 | 80 | 20 | 20 | 0 | 0 | 20 |
| | | | | | | |
| 1-3 | 20 | 50 | 80 | 30 | 90 | 70 |
| 4-80 | 20 | 0 | 10 | 0 | 0 | 0 |
| | | | | | | |
| 1-1 | 20 | 90 | | 30 | | |
| 4-108 | 20 | 50 | | 0 | | |
| | | | | | | |
| 1-2 | 20 | | | 60 | | |
| 4-109 | 20 | | | 0 | | |
| | | | | | | |
| 1-3 | 20 | | 80 | | | 70 |
| 4-110 | 20 | | 10 | | | 20 |
| | | | | | | |
| 1-1 | 20 | 90 | | 30 | 80 | 70 |
| 4-128 | 20 | 10 | | 0 | 0 | 10 |
| | | | | | | |
| 1-2 | 80 | 90 | 70 | | | |
| 4-129 | 80 | 30 | 50 | | | |
| | | | | | | |
| 1-3 | 20 | 50 | 80 | 30 | 90 | 70 |
| 4-130 | 20 | 20 | 0 | 0 | 70 | 20 |
| | | | | | | |
| 1-1 | 20 | | | 30 | 80 | |
| 4-122 | 20 | | | 0 | 50 | |
| | | | | | | |
| 1-2 | 20 | 70 | 20 | 60 | | |
| 4-123 | 20 | 20 | 0 | 0 | | |
| | | | | | | |
| 1-3 | 20 | 50 | 80 | 30 | | |
| 4-124 | 20 | 10 | 0 | 10 | | |
| | | | | | | |
| 1-1 | 20 | 90 | | | 80 | 70 |
| 4-406 | 20 | 0 | | | 10 | 0 |
| | | | | | | |
| 1-49 | 20 | 40 | 50 | 50 | 50 | 90 |
| 4-229 | 20 | 0 | 0 | 0 | 10 | 50 |
| | | | | | | |
| 1-50 | 80 | 100 | | 100 | | |
| 4-230 | 80 | 60 | | 40 | | |
| | | | | | | |
| 1-50 | 20 | 90 | | 70 | 90 | 100 |
| 4-230 | 20 | 40 | | 0 | 0 | 60 |
| | | | | | | |
| 1-51 | 20 | 80 | | 90 | 100 | |
| 4-231 | 20 | 20 | | 50 | 70 | |
| | | | | | | |
| 1-49 | 20 | 40 | 50 | | | 90 |
| 4-292 | 20 | 20 | 0 | | | 20 |
| | | | | | | |
| 1-50 | 80 | 100 | 90 | 100 | 100 | 100 |
| 4-293 | 80 | 20 | 0 | 40 | 0 | 10 |
| | | | | | | |
| 1-51 | 20 | 80 | | 90 | 100 | 100 |
| 4-756 | 20 | 40 | | 0 | 0 | 40 |
| | | | | | | |
| 1-49 | 80 | | 80 | 70 | | |
| 4-245 | 80 | | 60 | 30 | | |
| | | | | | | |
| 1-49 | 20 | | | 50 | | |
| 4-245 | 20 | | | 0 | | |
| | | | | | | |
| 1-50 | 20 | 90 | | 70 | 90 | 100 |
| 4-246 | 20 | 70 | | 50 | 60 | 70 |
| | | | | | | |
| 1-51 | 20 | 80 | | 90 | | |
| 4-247 | 20 | 60 | | 70 | | |
| | | | | | | |
| 1-49 | 80 | 90 | 80 | 70 | 100 | |
| 4-632 | 80 | 30 | 0 | 20 | 70 | |
| | | | | | | |
| 1-50 | 20 | 90 | | 70 | | 100 |
| 4-633 | 20 | 0 | | 20 | | 10 |
| | | | | | | |
| 1-51 | 20 | 80 | | 90 | 100 | 100 |
| 4-634 | 20 | 60 | | 60 | 80 | 30 |
| | | | | | | |
| 1-49 | 20 | | 50 | 50 | | |
| 4-638 | 20 | | 0 | 0 | | |
| | | | | | | |
| 1-50 | 20 | 90 | | | | 100 |
| 4-639 | 20 | 30 | | | | 70 |
| | | | | | | |
| 1-51 | 20 | | | 90 | | |
| 4-640 | 20 | | | 0 | | |
| | | | | | | |
| 4-1 | 320 | 40 | 80 | 50 | 100 | 100 |
| 1-573 | 320 | 0 | 0 | 0 | 60 | 0 |
| | | | | | | |
| 4-3 | 320 | 90 | 80 | 100 | 100 | 100 |
| 1-574 | 320 | 0 | 0 | 50 | 30 | 0 |
| | | | | | | |
| 4-1 | 80 | | 30 | 30 | 70 | 80 |
| 1-146 | 80 | | 0 | 0 | 30 | 0 |
| | | | | | | |
| 4-3 | 80 | 60 | 30 | 100 | 100 | 90 |
| 1-148 | 80 | 0 | 0 | 0 | 0 | 0 |
| | | | | | | |
| 4-1 | 80 | | 30 | 30 | 70 | 80 |
| 1-186 | 80 | | 0 | 0 | 0 | 0 |
| | | | | | | |
| 4-3 | 80 | 60 | 30 | 100 | 100 | 90 |
| 1-188 | 80 | 10 | 0 | 0 | 0 | 0 |
| | | | | | | |
| 4-3 | 320 | 90 | 80 | | 100 | 100 |
| 1-90 | 320 | 0 | 10 | | 30 | 20 |
| | | | | | | |
| 4-1 | 320 | 40 | 80 | 50 | 100 | 100 |
| 1-119 | 320 | 0 | 40 | 0 | 20 | 40 |
| | | | | | | |
| 4-3 | 80 | 60 | 30 | 100 | 100 | 90 |
| 1-121 | 80 | 0 | 0 | 20 | 0 | 0 |
| | | | | | | |
| 4-1 | 80 | | 30 | 30 | 70 | 80 |
| 1-387 | 80 | | 0 | 0 | 0 | 0 |
| | | | | | | |
| 4-3 | 80 | 60 | 30 | 100 | 100 | 90 |
| 1-389 | 80 | 30 | 0 | 0 | 0 | 0 |
| | | | | | | |
| 4-1 | 320 | 40 | 80 | | 100 | 100 |
| 1-139 | 320 | 0 | 30 | | 20 | 0 |
| | | | | | | |

**Tabelle K: Vergleich mit aus WO 2012/028579 bekannten Verbindungen im Vorauflauf**

| Beispiel-Nr. | Dosierung [g / ha] | Herbizide Wirkung gegen | | | |
|---|---|---|---|---|---|
| | | ABUTH | MATIN | POLCO | VIOTR |
| 1-1 | 20 | 90 | 70 | 60 | 100 |
| 4-803 | 20 | 0 | 10 | 0 | 0 |
| | | | | | |
| 1-2 | 320 | 100 | 100 | 80 | 100 |
| 4-804 | 320 | 80 | 0 | 0 | 0 |
| | | | | | |
| 1-2 | 80 | 100 | 100 | 70 | 100 |
| 4-804 | 80 | 0 | 0 | 0 | 0 |
| | | | | | |
| 1-3 | 320 | 100 | 100 | 90 | 100 |
| 4-805 | 320 | 20 | 30 | 0 | 0 |
| | | | | | |
| 1-3 | 80 | 100 | 100 | 90 | 100 |
| 4-805 | 80 | 0 | 30 | 0 | 0 |
| | | | | | |
| 1-3 | 80 | | 100 | 90 | |
| 4-814 | 80 | | 80 | 20 | |
| | | | | | |
| 1-3 | 20 | 100 | 80 | 30 | 100 |
| 4-814 | 20 | 40 | 20 | 10 | 80 |
| | | | | | |
| 1-49 | 20 | 70 | 80 | | 100 |
| 4-803 | 20 | 0 | 10 | | 0 |
| | | | | | |
| 1-50 | 320 | 100 | 100 | 90 | 100 |
| 4-804 | 320 | 80 | 0 | 0 | 0 |
| | | | | | |
| 1-50 | 80 | 100 | 100 | 80 | 100 |
| 4-804 | 80 | 0 | 0 | 0 | 0 |
| | | | | | |
| 1-51 | 320 | 100 | 100 | 90 | 100 |
| 4-805 | 320 | 20 | 30 | 0 | 0 |
| | | | | | |
| 1-51 | 80 | 100 | 100 | 80 | 100 |
| 4-805 | 80 | 0 | 30 | 0 | 0 |
| | | | | | |
| 1-50 | 20 | | 90 | | |
| 4-813 | 20 | | 70 | | |
| | | | | | |
| 1-51 | 80 | | 100 | 80 | |
| 4-814 | 80 | | 80 | 20 | |
| | | | | | |
| 1-51 | 20 | 100 | 90 | 30 | 100 |
| 4-814 | 20 | 40 | 20 | 10 | 80 |
| | | | | | |
| 1-1 | 20 | | 70 | 60 | 100 |
| 4-135 | 20 | | 0 | 0 | 50 |
| | | | | | |
| 1-3 | 20 | | | 30 | |
| 4-137 | 20 | | | 0 | |
| | | | | | |
| 1-1 | 20 | 90 | 70 | 60 | 100 |
| 4-174 | 20 | 70 | 30 | 0 | 70 |
| | | | | | |
| 1-2 | 20 | | 70 | | 80 |
| 4-175 | 20 | | 40 | | 0 |
| | | | | | |
| 1-3 | 20 | | | 30 | 100 |
| 4-176 | 20 | | | 0 | 80 |
| | | | | | |
| 1-1 | 80 | 100 | 90 | 70 | 100 |
| 4-78 | 80 | 50 | 0 | 0 | 10 |
| | | | | | |
| 1-2 | 80 | 100 | 100 | 70 | 100 |
| 4-79 | 80 | 0 | 0 | 0 | 0 |
| | | | | | |
| 1-3 | 20 | 100 | 80 | 30 | 100 |
| 4-80 | 20 | 0 | 0 | 0 | 0 |
| | | | | | |
| 1-1 | 20 | 90 | 70 | | 100 |
| 4-108 | 20 | 60 | 0 | | 20 |
| | | | | | |
| 1-2 | 20 | | | | 80 |
| 4-109 | 20 | | | | 10 |
| | | | | | |
| 1-3 | 20 | | | | 100 |
| 4-110 | 20 | | | | 80 |
| | | | | | |
| 1-1 | 20 | 90 | 70 | 60 | 100 |
| 4-128 | 20 | 70 | 50 | 0 | 50 |
| | | | | | |
| 1-2 | 80 | | | 70 | |
| 4-129 | 80 | | | 10 | |
| | | | | | |
| 1-3 | 20 | | 80 | 30 | |
| 4-130 | 20 | | 20 | 0 | |
| | | | | | |
| 1-1 | 20 | | | 60 | 100 |
| 4-122 | 20 | | | 0 | 70 |
| | | | | | |
| 1-2 | 20 | | 70 | | 80 |
| 4-123 | 20 | | 0 | | 20 |
| | | | | | |
| 1-3 | 20 | | | 30 | |
| 4-124 | 20 | | | 0 | |
| | | | | | |
| 1-1 | 20 | 90 | | 60 | 100 |
| 4-406 | 20 | 40 | | 10 | 0 |
| | | | | | |
| 1-49 | 20 | 70 | 80 | | 100 |
| 4-229 | 20 | 0 | 0 | | 0 |
| | | | | | |
| 1-50 | 80 | 100 | 100 | 80 | 100 |
| 4-230 | 80 | 80 | 0 | 0 | 30 |
| | | | | | |
| 1-50 | 20 | 100 | | | 100 |
| 4-230 | 20 | 30 | | | 0 |
| | | | | | |
| 1-51 | 20 | 100 | 90 | 30 | 100 |
| 4-231 | 20 | 60 | 70 | 0 | 0 |
| | | | | | |
| 1-50 | 80 | 100 | | 80 | 100 |
| 4-293 | 80 | 40 | | 0 | 70 |
| | | | | | |
| 1-51 | 20 | 100 | | 30 | 100 |
| 4-756 | 20 | 70 | | 0 | 20 |
| | | | | | |
| 1-49 | 20 | | | | 100 |
| 4-245 | 20 | | | | 50 |
| | | | | | |
| 1-50 | 20 | | | | 100 |
| 4-246 | 20 | | | | 20 |
| | | | | | |
| 1-49 | 80 | 100 | | 50 | 100 |
| 4-632 | 80 | 60 | | 0 | 70 |
| | | | | | |
| 1-50 | 20 | 100 | 90 | | 100 |
| 4-633 | 20 | 60 | 60 | | 0 |
| | | | | | |
| 1-51 | 20 | 100 | 90 | 30 | |
| 4-634 | 20 | 70 | 70 | 0 | |
| | | | | | |
| 1-49 | 20 | | 80 | | |
| 4-638 | 20 | | 40 | | |
| | | | | | |
| 1-50 | 20 | | 90 | | 100 |
| 4-639 | 20 | | 60 | | 80 |
| | | | | | |
| 1-51 | 20 | | | 30 | |
| 4-640 | 20 | | | 0 | |
| | | | | | |
| 4-1 | 320 | 100 | | 30 | 100 |
| 1-573 | 320 | 60 | | 0 | 80 |
| | | | | | |
| 4-3 | 320 | 100 | 100 | 70 | 100 |
| 1-574 | 320 | 40 | 70 | 0 | 10 |
| | | | | | |
| 4-1 | 80 | 90 | 80 | 30 | 100 |
| 1-146 | 80 | 70 | 60 | 0 | 30 |
| | | | | | |
| 4-3 | 80 | 100 | | 40 | |
| 1-148 | 80 | 0 | | 0 | |
| | | | | | |
| 4-1 | 80 | 90 | 80 | 30 | 100 |
| 1-186 | 80 | 0 | 0 | 0 | 0 |
| | | | | | |
| 4-3 | 80 | 100 | 100 | 40 | 100 |
| 1-188 | 80 | 0 | 40 | 0 | 40 |
| | | | | | |
| 4-3 | 320 | 100 | 100 | 70 | 100 |
| 1-90 | 320 | 0 | 0 | 10 | 0 |
| | | | | | |
| 4-1 | 320 | 100 | | | 100 |
| 1-119 | 320 | 40 | | | 0 |
| | | | | | |
| 4-3 | 80 | 100 | | 40 | 100 |
| 1-121 | 80 | 60 | | 0 | 50 |
| | | | | | |
| 4-1 | 80 | 90 | 80 | 30 | 100 |
| 1-387 | 80 | 30 | 30 | 0 | 0 |
| | | | | | |
| 4-3 | 80 | 100 | 100 | | 100 |
| 1-389 | 80 | 30 | 50 | | 80 |
| | | | | | |
| 4-1 | 320 | 100 | 90 | 30 | |
| 1-139 | 320 | 70 | 70 | 0 | |
| | | | | | |

**Tabelle L: Vergleich mit aus WO2012126932 bekannten Verbindungen im Vorauflauf**

| Beispiel- Nr. | Dosierung [g / ha] | Herbizide Wirkung gegen | | | | | | |
|---|---|---|---|---|---|---|---|---|
| | | ALOMY | AVEFA | CYPES | LOLMU | SETVI | POLCO | VIOTR |
| 7-1 | 80 | 60 | 20 | 60 | 0 | | 30 | |
| 2-143 | 80 | 30 | 0 | 0 | 0 | | 0 | |
| | | | | | | | | |
| 7-3 | 80 | 90 | 90 | 100 | 80 | 100 | 40 | 100 |
| 2-145 | 80 | 90 | 80 | 90 | 60 | 100 | 10 | 100 |
| | | | | | | | | |
| 7-1 | 80 | 60 | 20 | | 0 | 70 | | 90 |
| 2-183 | 80 | 0 | 0 | | 0 | 0 | | 0 |
| | | | | | | | | |
| 7-3 | 80 | 90 | 90 | 100 | 80 | 100 | 40 | 100 |
| 2-185 | 80 | 80 | 20 | 70 | 40 | 100 | 40 | 100 |
| | | | | | | | | |
| 7-1 | 320 | 70 | 70 | 70 | 30 | 100 | | 100 |
| 2-130 | 320 | 70 | 30 | 0 | 30 | 100 | | 90 |
| | | | | | | | | |
| 7-1 | 80 | 60 | 20 | 60 | 0 | 70 | 30 | 90 |
| 2-136 | 80 | 0 | 0 | 0 | 0 | 20 | 0 | 0 |
| | | | | | | | | |
| 7-1 | 320 | | 70 | 70 | 30 | 100 | | 100 |
| 2-116 | 320 | | 40 | 50 | 10 | 90 | | 80 |
| | | | | | | | | |
| 7-2 | 80 | 80 | 70 | 80 | 10 | 90 | 30 | 100 |
| 2-117 | 80 | 20 | 0 | 50 | 0 | 20 | 0 | 0 |
| | | | | | | | | |
| 7-3 | 80 | 90 | 90 | 100 | 80 | 100 | | 100 |
| 2-118 | 80 | 30 | 50 | 70 | 40 | 60 | | 90 |
| | | | | | | | | |
| 7-1 | 320 | 70 | 70 | 70 | 30 | 100 | 40 | 100 |
| 2-85 | 320 | 0 | 0 | 40 | 0 | 0 | 0 | 0 |
| | | | | | | | | |
| 7-3 | 320 | 100 | 100 | 100 | 100 | 100 | 70 | 100 |
| 2-87 | 320 | 70 | 30 | 20 | 0 | 80 | 20 | 60 |

**Tabelle M: Vergleich mit aus WO2012126932 bekannten Verbindungen im Nachauflauf**

| Beispiel- Nr. | Dosierung [g / ha] | Herbizide Wirkung gegen | | | | | |
|---|---|---|---|---|---|---|---|
| | | ALOMY | AVEFA | SETVI | MATIN | VIOTR | VERPE |
| 7-1 | 20 | 70 | 40 | 100 | | 100 | 90 |
| 2-143 | 20 | 50 | 20 | 100 | | 60 | 80 |
| | | | | | | | |
| 7-2 | 20 | | 70 | 100 | 70 | 100 | 100 |
| 2-144 | 20 | | 70 | 90 | 90 | 60 | 100 |
| | | | | | | | |
| 7-1 | 20 | 70 | 40 | 100 | | 100 | |
| 2-183 | 20 | 20 | 10 | 70 | | 70 | |
| | | | | | | | |
| 7-1 | 20 | 70 | 40 | 100 | 40 | 100 | 90 |
| 2-130 | 20 | 40 | 20 | 80 | 10 | 30 | 80 |
| | | | | | | | |
| 7-1 | 20 | 70 | 40 | 100 | 40 | 100 | 90 |
| 2-136 | 20 | 30 | 0 | 90 | 20 | 30 | 60 |
| | | | | | | | |
| 7-1 | 20 | 70 | 40 | 100 | 40 | 100 | 90 |
| 2-116 | 20 | 70 | 40 | 70 | 40 | 60 | 50 |
| | | | | | | | |
| 7-2 | 20 | | 70 | 100 | 70 | 100 | 100 |
| 2-117 | 20 | | 60 | 70 | 30 | 90 | 70 |
| | | | | | | | |
| 7-3 | 80 | 100 | 100 | 100 | 100 | 100 | 100 |
| 2-118 | 80 | 70 | 100 | 90 | 70 | 100 | 90 |
| | | | | | | | |
| 7-3 | 20 | 60 | 90 | 90 | 90 | 100 | 100 |
| 2-118 | 20 | 60 | 90 | 90 | 50 | 90 | 80 |
| | | | | | | | |
| 7-1 | 20 | 70 | 40 | 100 | 40 | 100 | 90 |
| 2-85 | 20 | 0 | 0 | 40 | 20 | 0 | 50 |
| | | | | | | | |
| 7-3 | 80 | 100 | 100 | 100 | 100 | 100 | 100 |
| 2-87 | 80 | 60 | 70 | 80 | 0 | 70 | 80 |

## Patentansprüche

1. Benzoylamide der Formel (I) und deren Salze 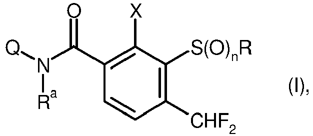 worin die Symbole und Indizes folgende Bedeutungen haben:
Q bedeutet einen Rest Q1, Q2, Q3 oder Q4, 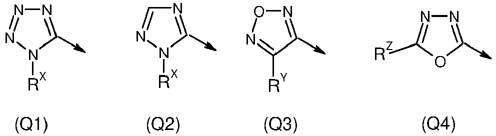
X bedeutet (C₁-C₆)-Alkyl, (C₃-C₆)-Cycloalkyl oder Halogen,
R bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
R^{a} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C, R¹O(O)C, (R¹)₂N(O)C, R¹O, (R¹)₂N, R²O(O)C(R¹)N, (R¹)₂N(O)C(R¹)N, R²(O)₂S,
oder jeweils durch s Reste aus der Gruppe bestehend aus Methyl, Ethyl, Methoxy, Nitro, Trifluormethyl und Halogen substituiertes Benzyl,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus Nitro, Cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl-S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl, und wobei Heterocyclyl n Oxogruppen trägt,
R^{Z} bedeutet Wasserstoff, (C₁-C₆)-Alkyl, R¹O-(C₁-C₆)-Alkyl, R*'*CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(Ci-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl,
R*'* bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
n bedeutet 0, 1 oder 2,
s bedeutet 0, 1, 2 oder 3,
mit der Maßgabe, dass die Verbindungen 4-Difluormethlyl-3-ethylsulfinyl-2-methyl-N-(5-methyl-1,3,4-oxadiazol-2-yl)benzamid und 4-Difluormethlyl-3-ethylsulfonyl-2-methyl-N-(5-methyl-1,3,4-oxadiazol-2-yl)benzamid sowie deren Natriumsalze ausgenommen sind.

2. Benzoylamide gemäß Anspruch 1, worin
Q bedeutet einen Rest Q1, Q2, Q3 oder Q4, 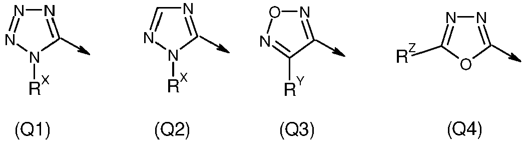
X bedeutet (C₁-C₆)-Alkyl oder (C₃-C₆)-Cycloalkyl,
R bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
R^{a} bedeutet Wasserstoff,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste durch s Reste aus der Gruppe bestehend aus (C₁-C₆)-Alkyl, Halogen-(C₁-C₆)-alkyl, (C₁-C₆)-Alkoxy und Halogen substituiert sind, und wobei Heterocyclyl n Oxogruppen trägt,
oder R^{X} bedeutet (C₃-C₇)-Cycloalkyl, wobei dieser Rest durch s Reste aus der Gruppe bestehend aus Halogen, (C₁-C₆)-Alkyl und Halogen-(C₁-C₆)-alkyl substituiert ist,
R^{Y} bedeutet Wasserstoff, (C₁-C₆)-Alkyl, Halogen-(C₁-C₆)-alkyl, (C₃-C₇)-Cycloalkyl, (C₁-C₆)-Alkoxy, Methoxycarbonyl, Methoxycarbonylmethyl, Halogen, Amino, Aminocarbonyl oder Methoxymethyl,
R^{Z} bedeutet Wasserstoff, (C₁-C₆)-Alkyl, R¹O-(C₁-C₆)-Alkyl, R*'*CH₂, (C₃-C₇)-Cycloalkyl, Halogen-(C₁-C₆)-alkyl, R¹O, R¹(H)N, Methoxycarbonyl, Acetylamino oder Methylsulfonyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(Ci-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R*'* bedeutet Acetoxy, Acetamido, Methoxycarbonyl oder (C₃-C₆)-Cycloalkyl,
n bedeutet 0, 1 oder 2,
s bedeutet 0, 1, 2 oder 3.

3. Benzoylamide gemäß Anspruch 1, worin
Q bedeutet einen Rest Q1, Q2, Q3 oder Q4, 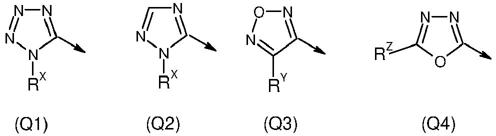
X bedeutet Halogen,
R bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
R^{a} bedeutet Wasserstoff,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste durch s Reste aus der Gruppe bestehend aus (C₁-C₆)-Alkyl, Halogen-(C₁-C₆)-alkyl, (C₁-C₆)-Alkoxy und Halogen substituiert sind, und wobei Heterocyclyl n Oxogruppen trägt,
oder R^{X} bedeutet (C₃-C₇)-Cycloalkyl, wobei dieser Rest durch s Reste aus der Gruppe bestehend aus Halogen, (C₁-C₆)-Alkyl und Halogen-(C₁-C₆)-alkyl substituiert ist,
R^{Y} bedeutet Wasserstoff, (C₁-C₆)-Alkyl, Halogen-(C₁-C₆)-alkyl, (C₃-C₇)-Cycloalkyl, (C₁-C₆)-Alkoxy, Methoxycarbonyl, Methoxycarbonylmethyl, Halogen, Amino, Aminocarbonyl oder Methoxymethyl,
R^{Z} bedeutet Wasserstoff, (C₁-C₆)-Alkyl, R¹O-(C₁-C₆)-Alkyl, R*'*CH₂, (C₃-C₇)-Cycloalkyl, Halogen-(C₁-C₆)-alkyl, R¹O, R¹(H)N, Methoxycarbonyl, Acetylamino oder Methylsulfonyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(Ci-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R*'* bedeutet Acetoxy, Acetamido, Methoxycarbonyl oder (C₃-C₆)-Cycloalkyl,
n bedeutet 0, 1 oder 2,
s bedeutet 0, 1, 2 oder 3.

4. Benzoylamide gemäß Anspruch 1 oder 2, worin
Q bedeutet einen Rest Q1, Q2, Q3 oder Q4, 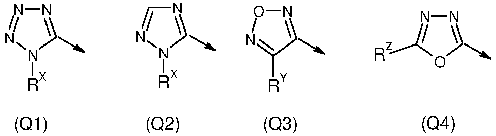
X bedeutet Methyl, Ethyl oder Cyclopropyl,
R bedeutet Methyl, Ethyl, Cyclopropylmethyl oder Methoxyethyl,
R^{a} bedeutet Wasserstoff,
R^{X} bedeutet Methyl, Ethyl oder n-Propyl,
R^{Y} bedeutet Methyl oder Chlor,
R^{Z} bedeutet Methyl,
n bedeutet 0, 1 oder 2.

5. Benzoylamide gemäß Anspruch 1 oder 3, worin
Q bedeutet einen Rest Q1, Q2, Q3 oder Q4, 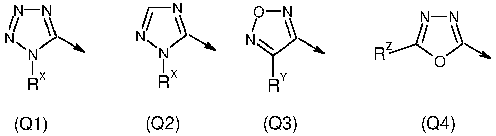
X bedeutet Fluor, Chlor, Brom oder Iod,
R bedeutet Methyl, Ethyl, Cyclopropylmethyl oder Methoxyethyl,
R^{a} bedeutet Wasserstoff,
R^{X} bedeutet Methyl, Ethyl oder n-Propyl,
R^{Y} bedeutet Methyl oder Chlor,
R^{Z} bedeutet Methyl,
n bedeutet 0, 1 oder 2.

6. Herbizide Mittel enthaltend mindestens eine Verbindung gemäß einem der Ansprüche 1 bis 5 in Mischung mit Formulierungshilfsmitteln.

7. Herbizide Mittel gemäß einem der Ansprüche 1 bis 5 enthaltend mindestens einen weiteren pestizid wirksamen Stoff aus der Gruppe Insektizide, Akarizide, Herbizide, Fungizide, Safener und Wachstumsregulatoren.

8. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 5 oder von herbiziden Mitteln nach Anspruch 6 oder 7 auf die Pflanzen oder auf den Ort des unerwünschten Pflanzenwachstums appliziert.

9. Verwendung von Verbindungen der Formel (I) gemäß einem der Ansprüche 1 bis 5 oder von herbiziden Mitteln nach Anspruch 6 oder 7 zur Bekämpfung unerwünschter Pflanzen.

10. Verwendung nach Anspruch 9, **dadurch gekennzeichnet, daß** die Verbindungen der Formel (I) zur Bekämpfung unerwünschter Pflanzen in Kulturen von Nutzpflanzen eingesetzt werden.

11. Verwendung nach Anspruch 10, **dadurch gekennzeichnet, daß** die Nutzpflanzen transgene Nutzpflanzen sind.

## Claims

1. Benzoylamides of the formula (I) and salts thereof 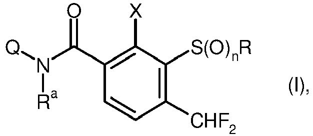 in which the symbols and indices are defined as follows:
Q represents a radical Q1, Q2, Q3 or Q4, 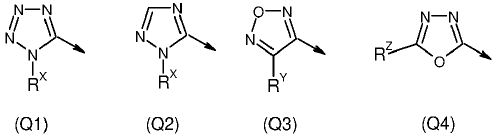
X represents (C₁-C₆) -alkyl, (C₃-C₆) -cycloalkyl or halogen,
R represents (C₁-C₆) -alkyl, (C₃-C₆) -cycloalkyl, (C₃-C₆)-cycloalkyl- (C₁-C₆) -alkyl, (C₁-C₆) -alkyl-O- (C₁-C₆) -alkyl,
R^{a} represents hydrogen, (C₁-C₆) -alkyl, halo- (C₁-C₆) - alkyl, (C₂-C₆)-alkenyl, halo- (C₂-C₆)-alkenyl, (C₂-C₆)-alkynyl, halo- (C₃-C₆) -alkynyl, (C₃-C₆) -cycloalkyl, halo-(C₃-C₆) -cycloalkyl, (C₃-C₆) -cycloalkyl- (C₁-C₆) -alkyl, halo- (C₃-C₆) -cycloalkyl- (C₁-C₆) -alkyl, R¹(O)C-(C₁-C₆) - alkyl, R¹O(O)C-(C₁-C₆)-alkyl, (R¹)₂N(O)C-(C₁-C₆)-alkyl, NC-(C₁-C₆) -alkyl, R¹O-(C₁-C₆)-alkyl, R¹(O)CO-(C₁-C₆)-alkyl, R²(O)₂SO-(C₁-C₆)-alkyl, (R¹)₂N-(C₁-C₆)-alkyl, R¹(O)C (R¹)N-(C₁-C₆)-alkyl, R²(O)₂S(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C, R¹O(O)C, (R¹)₂N(O)C, R¹O, (R¹)₂N, R²O(O)C(R¹)N, (R¹)₂N(O)C(R¹)N, R²(O)₂S,
or benzyl substituted in each case by s radicals from the group consisting of methyl, ethyl, methoxy, nitro, trifluoromethyl and halogen,
R^{X} represents (C₁-C₆) -alkyl, halo- (C₁-C₆) -alkyl, (C₂-C₆)-alkenyl, halo- (C₂-C₆)-alkenyl, (C₂-C₆)-alkynyl, halo-(C₃-C₆)-alkynyl, where the six radicals mentioned above are in each case substituted by s radicals from the group consisting of nitro, cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyl, heteroaryl, heterocyclyl and phenyl, where the four last-mentioned radicals are substituted by s radicals from the group consisting of (C₁-C₆)-alkyl, halo- (C₁-C₆) -alkyl, (C₁-C₆)-alkoxy, halo- (C₁-C₆) -alkoxy and halogen, and where heterocyclyl carries n oxo groups,
or R^{X} represents (C₃-C₇) -cycloalkyl, heteroaryl, heterocyclyl or phenyl, where the four radicals mentioned above are in each case substituted by s radicals from the group consisting of halogen, nitro, cyano, (C₁-C₆)-alkyl, halo- (C₁-C₆) -alkyl, (C₃-C₆)-cycloalkyl, (C₁-C₆) -alkyl-S(O)n, (C₁-C₆) -alkoxy, halo- (C₁-C₆) -alkoxy and (C₁-C₆)-alkoxy- (C₁-C₄)-alkyl,
R^{Y} represents hydrogen, (C₁-C₆) -alkyl, halo- (C₁-C₆)-alkyl, (C₂-C₆)-alkenyl, halo-(C₂-C₆)-alkenyl, (C₂-C₆)-alkynyl, halo- (C₃-C₆) -alkynyl, (C₃-C₇)-cycloalkyl, (C₁-C₆)-alkoxy, halo- (C₁-C₆) -alkoxy, (C₂-C₆)-alkenyloxy, (C₂-C₆)-alkynyloxy, cyano, nitro, methylsulfenyl, methylsulfinyl, methylsulfonyl, acetylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or represents heteroaryl, heterocyclyl or phenyl, each of which is substituted by s radicals from the group consisting of (C₁-C₆) -alkyl, halo- (C₁-C₆) -alkyl, (C₁-C₆) -alkoxy, halo- (C₁-C₆) -alkoxy and halogen, and where heterocyclyl carries n oxo groups,
R^{Z} represents hydrogen, (C₁-C₆)-alkyl, R¹O-(C₁-C₆)-alkyl, R*'*CH₂, (C₃-C₇) -cycloalkyl, halo- (C₁-C₆) -alkyl, (C₂-C₆)-alkenyl, halo- (C₂-C₆)-alkenyl, (C₂-C₆)-alkynyl, halo- (C₃-C₆)-alkynyl, R¹O, R¹(H)N, methoxycarbonyl, ethoxycarbonyl, methylcarbonyl, dimethylamino, trifluoromethylcarbonyl, acetylamino, methylsulfenyl, methylsulfinyl, methylsulfonyl, or represents heteroaryl, heterocyclyl, benzyl or phenyl, each of which is substituted by s radicals from the group consisting of halogen, nitro, cyano, (C₁-C₆) -alkyl, halo- (C₁-C₆)-alkyl, (C₃-C₆) -cycloalkyl, (C₁-C₆) -alkyl-S(O)n, (C₁-C₆)-alkoxy, halo- (C₁-C₆) -alkoxy and (C₁-C₆) -alkoxy- (C₁-C₄)-alkyl, where heterocyclyl carries n oxo groups,
R¹ represents hydrogen, (C₁-C₆)-alkyl, halo-(C₁-C₆)-alkyl, (C₂-C₆)-alkenyl, halo-(C₂-C₆)-alkenyl, (C₂-C₆)-alkynyl, halo- (C₃-C₆) -alkynyl, (C₃-C₆) -cycloalkyl, (C₃-C₆)-cycloalkenyl, halo- (C₃-C₆) -cycloalkyl, (C₃-C₆)-cycloalkyl- (C₁-C₆) -alkyl, (C₁-C₆)-alkyl-O-(C₁-C₆) -alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, phenyl-N(R³)-(C₁-C₆) -alkyl, heteroaryl-N(R³)-(C₁-C₆)-alkyl, heterocyclyl-N(R³)-(C₁-C₆)-alkyl,
phenyl-S(O)ₙ-(C₁-C₆)-alkyl, heteroaryl-S(O)ₙ-(C₁-C₆)-alkyl or heterocyclyl-S(O)ₙ-(C₁-C₆)-alkyl, where the fifteen last-mentioned radicals are in each case substituted by s radicals from the group consisting of nitro, halogen, cyano, thiocyanato, (C₁-C₆)-alkyl, halo-(C₁-C₆)-alkyl, (C₃-C₆)-cycloalkyl, R³O(O)C, (R³)₂N(O)C, R³O, (R³)₂N, R⁴(O)ₙS, R³O(O)₂S, (R³)₂N(O)₂S and R³O-(C₁-C₆)-alkyl, and where heterocyclyl carries n oxo groups,
R² represents (C₁-C₆) -alkyl, halo- (C₁-C₆) -alkyl, (C₂-C₆)-alkenyl, halo-(C₂-C₆)-alkenyl, (C₂-C₆)-alkynyl, halo-(C₃-C₆)-alkynyl, (C₃-C₆) -cycloalkyl, (C₃-C₆) -cycloalkenyl, halo- (C₃-C₆) -cycloalkyl, (C₃-C₆) -cycloalkyl- (C₁-C₆)-alkyl, (C₁-C₆) -alkyl-O- (C₁-C₆) -alkyl, cycloalkyl- (C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl- (C₁-C₆) -alkyl, phenyl-O-(C₁-C₆) -alkyl, heteroaryl-O-(C₁-C₆)-alkyl, heterocyclyl-O-(C₁-C₆)-alkyl, phenyl-N(R³)-(C₁-C₆)-alkyl, heteroaryl-N(R³)-(C₁-C₆)-alkyl, heterocyclyl-N(R³)-(C₁-C₆) -alkyl, phenyl-S(O)ₙ-(C₁-C₆)-alkyl, heteroaryl-S(O)ₙ-(C₁-C₆)-alkyl or heterocyclyl-S(O)ₙ-(C₁-C₆)-alkyl, where the fifteen last-mentioned radicals are in each case substituted by s radicals from the group consisting of nitro, halogen, cyano, thiocyanato, (C₁-C₆) -alkyl, halo-(C₁-C₆)-alkyl, (C₃-C₆) -cycloalkyl, R³O(O)C, (R³)₂N(O)C, R³O, (R³)₂N, R⁴(O)ₙS, R³O(O)₂S, (R³)₂N(O)₂S and R³O-(C₁-C₆) -alkyl, and where heterocyclyl carries n oxo groups,
R³ represents hydrogen, (C₁-C₆) -alkyl, halo- (C₁-C₆)-alkyl, (C₂-C₆)-alkenyl, (C₂-C₆)-alkynyl, (C₃-C₆)-cycloalkyl, (C₃-C₆) -cycloalkyl- (C₁-C₆) -alkyl or phenyl,
R⁴ is (C₁-C₆) -alkyl, halo- (C₁-C₆) -alkyl, (C₂-C₆)-alkenyl, (C₂-C₆)-alkynyl, (C₃-C₆) -cycloalkyl, (C₃-C₆)-cycloalkyl-(C₁-C₆)-alkyl or phenyl,
R⁵ represents hydrogen or (C₁-C₄)-alkyl,
R⁶ represents (C₁-C₄)-alkyl,
R*'* represents acetoxy, acetamido, N-methylacetamido, benzoyloxy, benzamido, N-methylbenzamido, methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, (C₃-C₆)-cycloalkyl, or represents heteroaryl or heterocyclyl, in each case substituted by s radicals from the group consisting of methyl, ethyl, methoxy, trifluoromethyl and halogen,
n represents 0, 1 or 2,
s represents 0, 1, 2 or 3,
with the proviso that the compounds 4-difluoromethyl-3-ethylsulfinyl-2-methyl-N-(5-methyl-1,3,4-oxadiazol-2-yl)benzamide and 4-difluoromethyl-3-ethylsulfonyl-2-methyl-N-(5-methyl-1,3,4-oxadiazol-2-yl)benzamide and their sodium salts are excluded.

2. Benzoylamides according to Claim 1, in which
Q represents a radical Q1, Q2, Q3 or Q4, 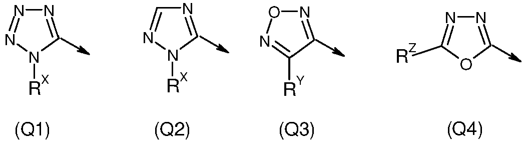
X represents (C₁-C₆) -alkyl or (C₃-C₆)-cycloalkyl,
R represents (C₁-C₆) -alkyl, (C₃-C₆)-cycloalkyl, (C₃-C₆)-cycloalkyl- (C₁-C₆) -alkyl, (C₁-C₆) -alkyl-O- (C₁-C₆) -alkyl,
R^{a} represents hydrogen,
R^{X} represents (C₁-C₆) -alkyl, halo- (C₁-C₆) -alkyl, (C₂-C₆)-alkenyl, halo- (C₂-C₆)-alkenyl, (C₂-C₆)-alkynyl, halo-(C₃-C₆)-alkynyl, where the six radicals mentioned above are in each case substituted by s radicals from the group consisting of R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyl, heteroaryl, heterocyclyl and phenyl, where the four last-mentioned radicals are substituted by s radicals from the group consisting of (C₁-C₆) -alkyl, halo-(C₁-C₆)-alkyl, (C₁-C₆) -alkoxy and halogen, and where heterocyclyl carries n oxo groups,
or R^{X} represents (C₃-C₇)-cycloalkyl, where this radical is substituted by s radicals from the group consisting of halogen, (C₁-C₆) -alkyl and halo- (C₁-C₆) -alkyl,
R^{Y} represents hydrogen, (C₁-C₆) -alkyl, halo- (C₁-C₆)-alkyl, (C₃-C₇)-cycloalkyl, (C₁-C₆) -alkoxy, methoxycarbonyl, methoxycarbonylmethyl, halogen, amino, aminocarbonyl or methoxymethyl,
R^{Z} represents hydrogen, (C₁-C₆) -alkyl, R¹O-(C₁-C₆)-alkyl, R*'*CH₂, (C₃-C₇) -cycloalkyl, halo- (C₁-C₆) -alkyl, R¹O, R¹(H)N, methoxycarbonyl, acetylamino or methylsulfonyl,
R¹ represents hydrogen, (C₁-C₆) -alkyl, halo- (C₁-C₆) - alkyl, (C₃-C₆) -cycloalkyl, halo- (C₃-C₆) -cycloalkyl, (C₃-C₆)-cycloalkyl- (C₁-C₆) -alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl- (C₁-C₆)-alkyl, heterocyclyl, heterocyclyl-(C₁-C₆)-alkyl, phenyl-O-(C₁-C₆) -alkyl, heteroaryl-O-(C₁-C₆) -alkyl, heterocyclyl-O-(C₁-C₆)-alkyl, where the nine last-mentioned radicals are in each case substituted by s radicals from the group consisting of nitro, halogen, (C₁-C₆)-alkyl, halo-(C₁-C₆)-alkyl, R³O(O)C, (R³)₂N(O)C, R³O, (R³)₂N, R⁴(O)ₙS and R³O-(C₁-C₆)-alkyl, and where heterocyclyl carries n oxo groups,
R² represents (C₁-C₆)-alkyl, halo- (C₁-C₆) -alkyl, (C₃-C₆)-cycloalkyl, halo-(C₃-C₆)-cycloalkyl, (C₃-C₆) -cycloalkyl-(C₁-C₆)-alkyl, (C₁-C₆) -alkyl-O- (C₁-C₆) -alkyl, cycloalkyl-(C₁-C₆)-alkyl-O-(C₁-C₆)-alkyl, phenyl, phenyl- (C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, where the nine last-mentioned radicals are in each case substituted by s radicals from the group consisting of nitro, halogen, (C₁-C₆) -alkyl, halo-(C₁-C₆)-alkyl, R³O(O)C, (R³)₂N(O)C, R³O, (R³)₂N, R⁴(O)ₙS and R³O-(C₁-C₆)-alkyl, and where heterocyclyl carries n oxo groups,
R³ represents hydrogen or (C₁-C₆)-alkyl,
R⁴ represents (C₁-C₆)-alkyl,
R*'* represents acetoxy, acetamido, methoxycarbonyl or (C₃-C₆) -cycloalkyl,
n represents 0, 1 or 2,
s represents 0, 1, 2 or 3.

3. Benzoylamides according to Claim 1, in which
Q represents a radical Q1, Q2, Q3 or Q4, 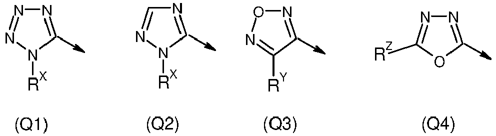
X represents halogen,
R represents (C₁-C₆) -alkyl, (C₃-C₆)-cycloalkyl, (C₃-C₆)-cycloalkyl- (C₁-C₆) -alkyl, (C₁-C₆) -alkyl-O- (C₁-C₆) -alkyl,
R^{a} represents hydrogen,
R^{X} represents (C₁-C₆) -alkyl, halo- (C₁-C₆) -alkyl, (C₂-C₆)-alkenyl, halo- (C₂-C₆)-alkenyl, (C₂-C₆)-alkynyl, halo-(C₃-C₆)-alkynyl, where the six radicals mentioned above are in each case substituted by s radicals from the group consisting of R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyl, heteroaryl, heterocyclyl and phenyl, where the four last-mentioned radicals are substituted by s radicals from the group consisting of (C₁-C₆) -alkyl, halo-(C₁-C₆)-alkyl, (C₁-C₆)-alkoxy and halogen, and where heterocyclyl carries n oxo groups,
or R^{X} represents (C₃-C₇)-cycloalkyl, where this radical is substituted by s radicals from the group consisting of halogen, (C₁-C₆) -alkyl and halo- (C₁-C₆) -alkyl,
R^{Y} represents hydrogen, (C₁-C₆) -alkyl, halo- (C₁-C₆) - alkyl, (C₃-C₇)-cycloalkyl, (C₁-C₆)-alkoxy, methoxycarbonyl, methoxycarbonylmethyl, halogen, amino, aminocarbonyl or methoxymethyl,
R^{Z} represents hydrogen, (C₁-C₆)-alkyl, R¹O-(C₁-C₆)-alkyl, R*'*CH₂, (C₃-C₇)-cycloalkyl, halo- (C₁-C₆)-alkyl, R¹O, R¹(H)N, methoxycarbonyl, acetylamino or methylsulfonyl,
R¹ represents hydrogen, (C₁-C₆)-alkyl, halo- (C₁-C₆)-alkyl, (C₃-C₆) -cycloalkyl, halo- (C₃-C₆) -cycloalkyl, (C₃-C₆)-cycloalkyl- (C₁-C₆)-alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl- (C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, where the nine last-mentioned radicals are in each case substituted by s radicals from the group consisting of nitro, halogen, (C₁-C₆)-alkyl, halo- (C₁-C₆)-alkyl, R³O(O)C, (R³)₂N(O)C, R³O, (R³)₂N, R⁴(O)ₙS and R³O-(C₁-C₆)-alkyl, and where heterocyclyl carries n oxo groups,
R² represents (C₁-C₆) -alkyl, halo- (C₁-C₆) -alkyl, (C₃-C₆)-cycloalkyl, halo-(C₃-C₆)-cycloalkyl, (C₃-C₆)-cycloalkyl-(C₁-C₆)-alkyl, (C₁-C₆)-alkyl-O- (C₁-C₆)-alkyl, cycloalkyl-(C₁-C₆)-alkyl-O-(C₁-C₆)-alkyl, phenyl, phenyl- (C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, where the nine last-mentioned radicals are in each case substituted by s radicals from the group consisting of nitro, halogen, (C₁-C₆)-alkyl, halo-(C₁-C₆)-alkyl, R³O(O)C, (R³)₂N(O)C, R³O, (R³)₂N, R⁴(O)ₙS and R³O-(C₁-C₆)-alkyl, and where heterocyclyl carries n oxo groups,
R³ represents hydrogen or (C₁-C₆)-alkyl,
R⁴ represents (C₁-C₆)-alkyl,
R*'* represents acetoxy, acetamido, methoxycarbonyl or (C₃-C₆) -cycloalkyl,
n represents 0, 1 or 2,
s represents 0, 1, 2 or 3.

4. Benzoylamides according to Claim 1 or 2, in which
Q represents a radical Q1, Q2, Q3 or Q4, 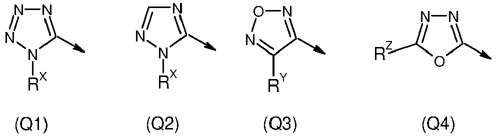
X represents methyl, ethyl or cyclopropyl,
R represents methyl, ethyl, cyclopropylmethyl or methoxyethyl,
R^{a} represents hydrogen,
R^{X} represents methyl, ethyl or n-propyl,
R^{Y} represents methyl or chlorine,
R^{Z} represents methyl,
n represents 0, 1 or 2.

5. Benzoylamides according to Claim 1 or 3, in which
Q represents a radical Q1, Q2, Q3 or Q4, 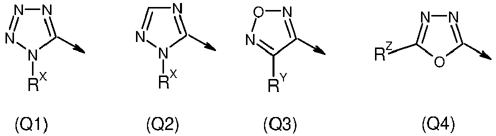
X represents fluorine, chlorine, bromine or iodine,
R represents methyl, ethyl, cyclopropylmethyl or methoxyethyl,
R^{a} represents hydrogen,
R^{X} represents methyl, ethyl or n-propyl,
R^{Y} represents methyl or chlorine,
R^{Z} represents methyl,
n represents 0, 1 or 2.

6. Herbicidal compositions comprising at least one compound according to any of Claims 1 to 5 mixed with formulation auxiliaries.

7. Herbicidal compositions according to any of Claims 1 to 5, comprising at least one further pesticidally active substance from the group consisting of insecticides, acaricides, herbicides, fungicides, safeners, and growth regulators.

8. Method for controlling unwanted plants, **characterized in that** an effective amount of at least one compound of the formula (I) according to any of Claims 1 to 5 or of herbicidal compositions according to Claim 6 or 7 is applied to the plants or the site of the unwanted vegetation.

9. Use of compounds of the formula (I) according to any of Claims 1 to 5 or of herbicidal compositions according to Claim 6 or 7 for controlling unwanted plants.

10. Use according to Claim 9, **characterized in that** the compounds of the formula (I) are used for controlling unwanted plants in crops of useful plants.

11. Use according to Claim 10, **characterized in that** the useful plants are transgenic useful plants.

## Revendications

1. Benzoylamides de la formule (I) et leurs sels 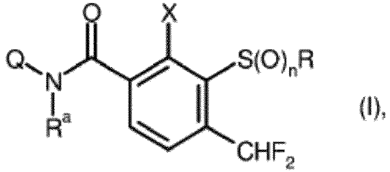 dans laquelle les symboles et indices possèdent les significations suivantes :
Q signifie un radical Q1, Q2, Q3 ou Q4, 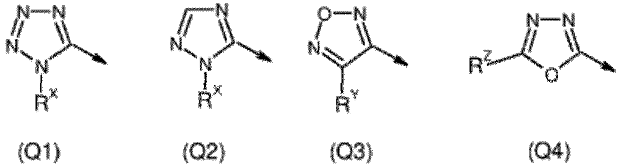
X signifie (C₁-C₆)-alkyle, (C₃-C₆) -cycloalkyle ou halogène,
R signifie (C₁-C₆)-alkyle, (C₃-C₆) -cycloalkyle, (C₃-C₆)-cycloalkyl-(C₁-C₆)-alkyle, (C₁-C₆)-alkyl-O-(C₁-C₆)-alkyle,
R^{a} signifie hydrogène, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆) -alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, R¹(O)C-(C₁-C₆)-alkyle, R¹O(O)C-(C₁-C₆)-alkyle, (R¹)₂N(O)C-(C₁-C₆)-alkyle, NC-(C₁-C₆)-alkyle, R¹O-(C₁-C₆)-alkyle, R¹(O)CO-(C₁-C₆)-alkyle, R²(O)₂SO-(C₁-C₆)-alkyle, (R¹)₂N-(C₁-C₆)-alkyle, R¹(O)C(R¹)N-(C₁-C₆) -alkyle, R²(O)₂S(R¹)N-(C₁-C₆)-alkyle, R²(O)ₙS-(C₁-C₆)-alkyle, R¹O(O)₂S-(C₁-C₆)-alkyle, (R¹)₂N(O)₂S-(C₁-C₆)-alkyle, R¹(O)C, R¹O(O)C, (R¹)₂N(O)C, R¹O, (R¹)₂N, R²O(O)C(R¹)N, (R¹)₂N(O)C(R¹)N, R²(O)₂S,
ou benzyle substitué à chaque fois par s radicaux du groupe constitué par méthyle, éthyle, méthoxy, nitro, trifluorométhyle et halogène,
R^{X} signifie (C₁-C₆)-alkyle, halogéno-(C₁-C₆) -alkyle, (C₂-C₆)-alcényle, halogéno-(C₂-C₆)-alcényle, (C₂-C₆)-alcynyle, halogéno-(C₃-C₆)-alcynyle, les six radicaux mentionnés ci-dessus étant en chaque cas substitués par s radicaux du groupe constitué par nitro, cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyle, hétéroaryle, hétérocyclyle et phényle, les quatre radicaux mentionnés en dernier étant substitués par s radicaux du groupe constitué par (C₁-C₆)-alkyle, halogéno-(C₁-C₆)-alkyle, (C₁-C₆)-alcoxy, halogéno-(C₁-C₆)-alcoxy et halogène, et hétérocyclyle portant n groupes oxo,
ou R^{X} signifie (C₃-C₇)-cycloalkyle, hétéroaryle, hétérocyclyle ou phényle, les quatre radicaux mentionnés ci-dessus étant en chaque cas substitués par s radicaux du groupe constitué par halogène, nitro, cyano, (C₁-C₆)-alkyle, halogéno-(C₁-C₆)-alkyle, (C₃-C₆) -cycloalkyle, (C₁-C₆)-alkyl-S(O)ₙ, (C₁-C₆)-alcoxy, halogéno-(C₁-C₆) -alcoxy et (C₁-C₆)-alcoxy-(C₁-C₄)-alkyle,
R^{Y} signifie hydrogène, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₇)-cycloalkyle, (C₁-C₆)-alcoxy, halogéno-(C₁-C₆)-alcoxy, (C₂-C₆)-alcényloxy, (C₂-C₆)-alcynyloxy, cyano, nitro, méthylsulfényle, méthylsulfinyle, méthylsulfonyle, acétylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, halogène, amino, aminocarbonyle, méthylaminocarbonyle, diméthylaminocarbonyle, méthoxyméthyle, ou signifie hétéroaryle, hétérocyclyle ou phényle, chacun desquels étant substitué par s radicaux du groupe constitué par (C₁-C₆) -alkyle, halogéno- (C₁-C₆) -alkyle, (C₁-C₆) -alcoxy, halogène-(C₁-C₆)-alcoxy et halogène, et hétérocyclyle portant n groupes oxo,
R^{Z} signifie hydrogène, (C₁-C₆) -alkyle, R¹O- (C₁-C₆) - alkyle, R'CH₂, (C₃-C₇) -cycloalkyle, halogéno- (C₁-C₆) - alkyle, (C₂-C₆) -alcényle, halogéno- (C₂-C₆) -alcényle, (C₂-C₆) -alcynyle, halogéno- (C₃-C₆) -alcynyle, R¹O, R¹(H)N, méthoxycarbonyle, éthoxycarbonyle, méthylcarbonyle, diméthylamino, trifluorométhylcarbonyle, acétylamino, méthylsulfényle, méthylsulfinyle, méthylsulfonyle, ou signifie hétéroaryle, hétérocyclyle, benzyle ou phényle, chacun desquels étant substitué par s radicaux du groupe constitué par halogène, nitro, cyano, (C₁-C₆)-alkyle, halogéno- (C₁-C₆) -alkyle, (C₃-C₆) -cycloalkyle, (C₁-C₆)-alkyl-S(O)ₙ, (C₁-C₆)-alcoxy, halogéno- (C₁-C₆) -alcoxy et (C₁-C₆)-alcoxy-(C₁-C₄)-alkyle, hétérocyclyle portant n groupes oxo,
R¹ signifie hydrogène, (C₁-C₆) -alkyle, halogéno-(C₁-C₆) -alkyle, (C₂-C₆) -alcényle, halogéno- (C₂-C₆) -alcényle, (C₂-C₆) -alcynyle, halogéno- (C₃-C₆) -alcynyle, (C₃-C₆)-cycloalkyle, (C₃-C₆)-cycloalcényle, halogéno-(C₃-C₆) - cycloalkyle, (C₃-C₆) -cycloalkyl- (C₁-C₆) -alkyle, (C₁-C₆)-alkyl-O- (C₁-C₆) -alkyle, cycloalkyl- (C₁-C₆) -alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆) -alkyle, phényl-O-(C₁-C₆)-alkyle, hétéroaryl-O-(C₁-C₆) -alkyle, hétérocyclyl-O- (C₁-C₆) -alkyle, phényl-N (R³) - (C₁-C₆) -alkyle, hétéroaryl-N (R³) - (C₁-C₆) -alkyle, hétérocyclyl-N (R³) - (C₁-C₆) -alkyle, phényl-S(O)ₙ- (C₁-C₆) - alkyle, hétéroaryl-S (O)ₙ- (C₁-C₆) -alkyle ou hétérocyclyl-S (O)ₙ- (C₁-C₆) -alkyle, les quinze radicaux mentionnés en dernier étant en chaque cas substitués par s radicaux du groupe constitué par nitro, halogène, cyano, thiocyanato, (C₁-C₆) -alkyle, halogéno- (C₁-C₆) -alkyle, (C₃-C₆)-cycloalkyle, R³O(O)C, (R³)₂N(O)C, R³O, (R³)₂N, R⁴(O)ₙS, R³O(O)₂S, (R³)₂N(O)₂S et R³O-(C₁-C₆)-alkyle, et hétérocyclyle portant n groupes oxo,
R² signifie (C₁-C₆) -alkyle, halogéno-(C₁-C₆) -alkyle, (C₂-C₆) -alcényle, halogéno-(C₂-C₆) -alcényle, (C₂-C₆)-alcynyle, halogéno-(C₃-C₆) -alcynyle, (C₃-C₆) -cycloalkyle, (C₃-C₆) -cycloalcényle, halogéno-(C₃-C₆) -cycloalkyle, (C₃-C₆)-cycloalkyl- (C₁-C₆) -alkyle, (C₁-C₆) -alkyl-O-(C₁-C₆)-alkyle, cycloalkyl- (C₁-C₆) -alkyl-O-(C₁-C₆) -alkyle, phényle, phényl-(C₁-C₆)-alkyle, hétéroaryle, hétéroaryl-(C₁-C₆) -alkyle, hétérocyclyle, hétérocyclyl- (C₁-C₆) - alkyle, phényl-O-(C₁-C₆)-alkyle, hétéroaryl-O-(C₁-C₆) - alkyle, hétérocyclyl-O-(C₁-C₆) -alkyle, phényl-N (R³) -(C₁-C₆)-alkyle, hétéroaryl-N(R³) -(C₁-C₆) -alkyle, hétérocyclyl-N(R³) -(C₁-C₆) -alkyle, phényl-S(O)ₙ-(C₁-C₆) - alkyle, hétéroaryl-S(O)ₙ-(C₁-C₆) -alkyle ou hétérocyclyl-S(O)ₙ-(C₁-C₆) -alkyle, les quinze radicaux mentionnés en dernier étant en chaque cas substitués par s radicaux du groupe constitué par nitro, halogène, cyano, thiocyanato, (C₁-C₆) -alkyle, halogéno-(C₁-C₆) -alkyle, (C₃-C₆)-cycloalkyle, R³O(O)C, (R³)₂N(O)C, R³O, (R³)₂N, R⁴(O)ₙS, R³O(O)₂S, (R³)₂N(O)₂S et R³O-(C₁-C₆)-alkyle, et hétérocyclyle portant n groupes oxo,
R³ signifie hydrogène, (C₁-C₆) -alkyle, halogène-(C₁-C₆) -alkyle, (C₂-C₆) -alcényle, (C₂-C₆) -alcynyle, (C₃-C₆)-cycloalkyle, (C₃-C₆) -cycloalkyl- (C₁-C₆) -alkyle ou phényle,
R⁴ signifie (C₁-C₆) -alkyle, halogéno-(C₁-C₆) -alkyle, (C₂-C₆) -alcényle, (C₂-C₆) -alcynyle, (C₃-C₆) -cycloalkyle, (C₃-C₆)-cycloalkyl-(C₁-C₆)-alkyle ou phényle,
R⁵ signifie hydrogène ou (C₁-C₄)-alkyle,
R⁶ signifie (C₁-C₄)-alkyle,
R' signifie acétoxy, acétamido, N-méthylacétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, (C₃-C₆)-cycloalkyle, ou signifie hétéroaryle ou hétérocyclyle, en chaque cas substitué par s radicaux du groupe constitué par méthyle, éthyle, méthoxy, trifluorométhyle et halogène,
n signifie 0, 1 ou 2,
s signifie 0, 1, 2 ou 3,
à la condition que les composés 4-difluorométhyl-3-éthylsulfinyl-2-méthyl-N-(5-méthyl-1,3,4-oxadiazol-2-yl)benzamide et 4-difluorométhyl-3-éthylsulfonyl-2-méthyl-N-(5-méthyl-1,3,4-oxadiazol-2-yl)benzamide ainsi que leurs sels de sodium soient exclus.

2. Benzoylamides selon la revendication 1, dans lesquels
Q signifie un radical Q1, Q2, Q3 ou Q4, 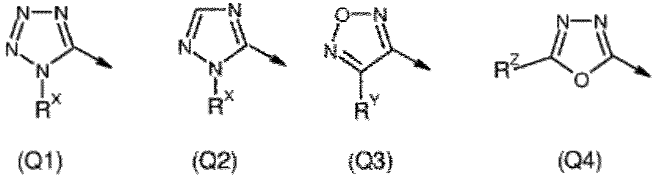
X signifie (C₁-C₆)-alkyle ou (C₃-C₆)-cycloalkyle,
R signifie (C₁-C₆) -alkyle, (C₃-C₆) -cycloalkyle, (C₃-C₆) -cycloalkyl- (C₁-C₆) -alkyle, (C₁-C₆) -alkyl-O-(C₁-C₆)-alkyle,
R^{a} signifie hydrogène,
R^{X} signifie (C₁-C₆) -alkyle, halogéno-(C₁-C₆) -alkyle, (C₂-C₆) -alcényle, halogéno-(C₂-C₆) -alcényle, (C₂-C₆)-alcynyle, halogéno-(C₃-C₆)-alcynyle, les six radicaux mentionnés ci-dessus étant en chaque cas substitués par s radicaux du groupe constitué par R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆) -cycloalkyle, hétéroaryle, hétérocyclyle et phényle, les quatre radicaux mentionnés en dernier étant substitués par s radicaux du groupe constitué par (C₁-C₆)-alkyle, halogéno-(C₁-C₆) -alkyle, (C₁-C₆)-alcoxy et halogène, et hétérocyclyle portant n groupes oxo,
ou R^{X} signifie (C₃-C₇)-cycloalkyle, ce radical étant substitué par s radicaux du groupe constitué par halogène, (C₁-C₆) -alkyle et halogéno-(C₁-C₆) -alkyle,
R^{Y} signifie hydrogène, (C₁-C₆) -alkyle, halogéno-(C₁-C₆)-alkyle, (C₃-C₇)-cycloalkyle, (C₁-C₆) -alcoxy, méthoxycarbonyle, méthoxycarbonylméthyle, halogène, amino, aminocarbonyle ou méthoxyméthyle,
R^{Z} signifie hydrogène, (C₁-C₆)-alkyle, R¹O-(C₁-C₆) - alkyle, R'CH₂, (C₃-C₇)-cycloalkyle, halogéno-(C₁-C₆) - alkyle, R¹O, R¹(H)N, méthoxycarbonyle, acétylamino ou méthylsulfonyle,
R¹ signifie hydrogène, (C₁-C₆) -alkyle, halogéno-(Ci-C₆) -alkyle, (C₃-C₆) -cycloalkyle, halogéno-(C₃-C₆) - cycloalkyle, (C₃-C₆) -cycloalkyl- (C₁-C₆) -alkyle, (C₁-C₆)-alkyl-O-(C₁-C₆) -alkyle, cycloalkyl- (C₁-C₆) -alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆) -alkyle, les neuf radicaux mentionnés en dernier étant en chaque cas substitués par s radicaux du groupe constitué par nitro, halogène, (C₁-C₆) -alkyle, halogéno-(C₁-C₆) -alkyle, R³O(O)C, (R³)₂N(O)C, R³O, (R³)₂N, R⁴(O)ₙS et R³O-(C₁-C₆)-alkyle, et hétérocyclyle portant n groupes oxo,
R² signifie (C₁-C₆) -alkyle, halogéno-(C₁-C₆) -alkyle, (C₃-C₆) -cycloalkyle, halogéno-(C₃-C₆) -cycloalkyle, (C₃-C₆)-cycloalkyl- (C₁-C₆) -alkyle, (C₁-C₆) -alkyl-O-(C₁-C₆)-alkyle, cycloalkyl- (C₁-C₆) -alkyl-O-(C₁-C₆) -alkyle, phényle, phényl-(C₁-C₆)-alkyle, hétéroaryle, hétéroaryl-(C₁-C₆)-alkyle, hétérocyclyle, hétérocyclyl- (C₁-C₆) - alkyle, phényl-O-(C₁-C₆)-alkyle, hétéroaryl-O-(C₁-C₆) - alkyle, hétérocyclyl-O-(C₁-C₆)-alkyle, les neuf radicaux mentionnés en dernier étant en chaque cas substitués par s radicaux du groupe constitué par nitro, halogène, (Ci-C₆)-alkyle, halogéno-(C₁-C₆) -alkyle, R³O(O)C, (R³) ₂N(O)C, R³O, (R³)₂N, R⁴(O)ₙS et R³O-(C₁-C₆) -alkyle, et hétérocyclyle portant n groupes oxo,
R³ signifie hydrogène ou (C₁-C₆)-alkyle,
R⁴ signifie (C₁-C₆)-alkyle,
R' signifie acétoxy, acétamido, méthoxycarbonyle ou (C₃-C₆) -cycloalkyle,
n signifie 0, 1 ou 2,
s signifie 0, 1, 2 ou 3.

3. Benzoylamides selon la revendication 1, dans lesquels
Q signifie un radical Q1, Q2, Q3 ou Q4, 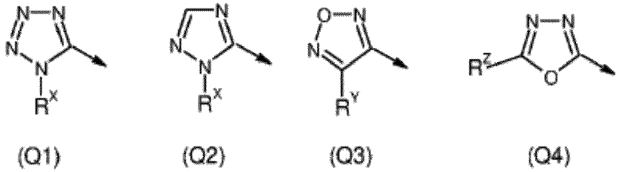
X signifie halogène,
R signifie (C₁-C₆) -alkyle, (C₃-C₆) -cycloalkyle, (C₃-C₆) -cycloalkyl- (C₁-C₆) -alkyle, (C₁-C₆) -alkyl-O-(C₁-C₆)-alkyle,
R^{a} signifie hydrogène,
R^{X} signifie (C₁-C₆) -alkyle, halogéno-(C₁-C₆) -alkyle, (C₂-C₆) -alcényle, halogéno-(C₂-C₆) -alcényle, (C₂-C₆)-alcynyle, halogéno-(C₃-C₆)-alcynyle, les six radicaux mentionnés ci-dessus étant en chaque cas substitués par s radicaux du groupe constitué par R²(O)nS, (R¹)₂N, R¹O, R¹(O)C, R¹O(O)C, R¹(O)CO, R²O(O)CO, R¹(O)C(R¹)N, R² (O)₂S(R¹)N, (C₃-C₆) -cycloalkyle, hétéroaryle, hétérocyclyle et phényle, les quatre radicaux mentionnés en dernier étant substitués par s radicaux du groupe constitué par (C₁-C₆)-alkyle, halogéno-(C₁-C₆) -alkyle, (C₁-C₆)-alcoxy et halogène, et hétérocyclyle portant n groupes oxo,
ou R^{X} signifie (C₃-C₇)-cycloalkyle, ce radical étant substitué par s radicaux du groupe constitué par halogène, (C₁-C₆) -alkyle et halogéno-(C₁-C₆) -alkyle,
R^{Y} signifie hydrogène, (C₁-C₆) -alkyle, halogène-(C₁-C₆)-alkyle, (C₃-C₇)-cycloalkyle, (C₁-C₆)-alcoxy, méthoxycarbonyle, méthoxycarbonylméthyle, halogène, amino, aminocarbonyle ou méthoxyméthyle,
R^{Z} signifie hydrogène, (C₁-C₆)-alkyle, R¹O-(C₁-C₆) - alkyle, R'CH₂, (C₃-C₇)-cycloalkyle, halogéno-(C₁-C₆) - alkyle, R¹O, R¹(H)N, méthoxycarbonyle, acétylamino ou méthylsulfonyle,
R¹ signifie hydrogène, (C₁-C₆)-alkyle, halogéno-(C₁-C₆) -alkyle, (C₃-C₆) -cycloalkyle, halogéno-(C₃-C₆) - cycloalkyle, (C₃-C₆) -cycloalkyl- (C₁-C₆) -alkyle, (C₁-C₆)-alkyl-O-(C₁-C₆) -alkyle, cycloalkyl- (C₁-C₆) -alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆) -alkyle, les neuf radicaux mentionnés en dernier étant en chaque cas substitués par s radicaux du groupe constitué par nitro, halogène, (C₁-C₆) -alkyle, halogéno-(C₁-C₆) -alkyle, R³O(O)C, (R³)₂N(O)C, R³O, (R³)₂N, R⁴(O)ₙS et R³O-(C₁-C₆)-alkyle, et hétérocyclyle portant n groupes oxo,
R² signifie (C₁-C₆) -alkyle, halogéno-(C₁-C₆) -alkyle, (C₃-C₆) -cycloalkyle, halogéno-(C₃-C₆) -cycloalkyle, (C₃-C₆)-cycloalkyl- (C₁-C₆) -alkyle, (C₁-C₆) -alkyl-O-(C₁-C₆)-alkyle, cycloalkyl- (C₁-C₆) -alkyl-O-(C₁-C₆) -alkyle, phényle, phényl-(C₁-C₆)-alkyle, hétéroaryle, hétéroaryl-(C₁-C₆)-alkyle, hétérocyclyle, hétérocyclyl- (C₁-C₆)-alkyle, phényl-O-(C₁-C₆)-alkyle, hétéroaryl-O-(C₁-C₆)-alkyle, hétérocyclyl-O-(C₁-C₆)-alkyle, les neuf radicaux mentionnés en dernier étant en chaque cas substitués par s radicaux du groupe constitué par nitro, halogène, (Ci-C₆) -alkyle, halogéno-(C₁-C₆) -alkyle, R³O(O)C, (R³) ₂N(O)C, R³O, (R³)₂N, R⁴(O)ₙS et R³O-(C₁-C₆) -alkyle, et hétérocyclyle portant n groupes oxo,
R³ signifie hydrogène ou (C₁-C₆)-alkyle,
R⁴ signifie (C₁-C₆)-alkyle,
R' signifie acétoxy, acétamido, méthoxycarbonyle ou (C₃-C₆)-cycloalkyle,
n signifie 0, 1 ou 2,
s signifie 0, 1, 2 ou 3.

4. Benzoylamides selon la revendication 1 ou 2, dans lesquels
Q signifie un radical Q1, Q2, Q3 ou Q4, 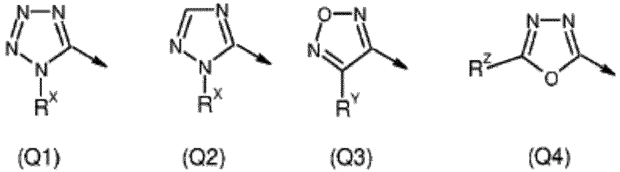
X signifie méthyle, éthyle ou cyclopropyle,
R signifie méthyle, éthyle, cyclopropylméthyle ou méthoxyéthyle,
R^{a} signifie hydrogène,
R^{X} signifie méthyle, éthyle ou n-propyle,
R^{Y} signifie méthyle ou chlore,
R^{Z} signifie méthyle,
n signifie 0, 1 ou 2.

5. Benzoylamides selon la revendication 1 ou 3, dans lesquels
Q signifie un radical Q1, Q2, Q3 ou Q4, 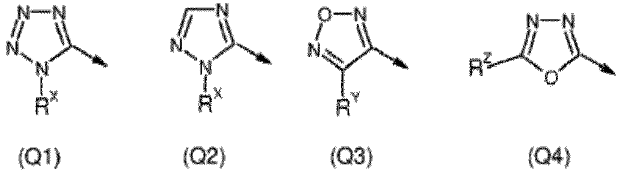
X signifie fluor, chlore, brome ou iode,
R signifie méthyle, éthyle, cyclopropylméthyle ou méthoxyéthyle,
R^{a} signifie hydrogène,
R^{X} signifie méthyle, éthyle ou n-propyle,
R^{Y} signifie méthyle ou chlore,
R^{Z} signifie méthyle,
n signifie 0, 1 ou 2.

6. Agents herbicides contenant au moins un composé selon l'une quelconque des revendications 1 à 5 en mélange avec des auxiliaires de formulation.

7. Agents herbicides selon l'une quelconque des revendications 1 à 5 contenant au moins une substance pesticide supplémentaire du groupe des insecticides, des acaricides, des herbicides, des fongicides, des phytoprotecteurs et des régulateurs de croissance.

8. Procédé pour la lutte contre des végétaux indésirables, **caractérisé en ce qu'**on applique une quantité efficace d'au moins un composé de formule (I) selon l'une quelconque des revendications 1 à 5 ou d'agents herbicides selon la revendication 6 ou 7 sur les végétaux ou sur le lieu de la croissance végétale indésirable.

9. Utilisation de composés de formule (I) selon l'une quelconque des revendications 1 à 5 ou d'agents herbicides selon la revendication 6 ou 7 pour la lutte contre des végétaux indésirables.

10. Utilisation selon la revendication 9, **caractérisée en ce que** les composés de formule (I) sont utilisés pour la lutte contre des végétaux indésirables dans des cultures de végétaux utiles.

11. Utilisation selon la revendication 10, **caractérisée en ce que** les végétaux utiles sont des végétaux utiles transgéniques.
